# EUROPEAN PATENT SPECIFICATION

(11) **EP 4 587 108 B1**
(45) Date of publication and mention of the grant of the patent: **22.07.2026**
(21) Application number: 23776454.3
(22) Date of filing: 14.09.2023
(51) Int. Cl.: A61N 1/05, A61B 5/00, A61N 1/36

(54) **NERVE STIMULATOR FOR SEXUAL DYSFUNCTION**
NERVENSTIMULATOR FÜR SEXUELLE DYSFUNKTION
STIMULATEUR NERVEUX POUR LA DYSFONCTION SEXUELLE

(30) Priority: 15.09.2022 US 202263375835 P
(43) Date of publication of application: 23.07.2025
(73) Proprietor: Amber Therapeutics Holdings Limited, Didcot OX11 0QQ (GB)
(72) Inventor: DENISON, Timothy, London W2 5BY (GB); DE WACHTER, Stefan, London W2 5BY (GB); KNOWLES, Charles, London W2 5BY (GB); CRAWLEY, Aidan, London W2 5BY (GB)
(74) Representative: J A Kemp LLP
(86) International application number: PCT/GB2023/052378
(87) International publication number: WO 2024/057025

(56) References cited:
- WO-A1-2020/033883
- WO-A2-2010/111321
- US-A1- 2013 041 430
- US-A1- 2013 289 664
- US-A1- 2022 111 211
- US-B2- 10 201 702

## Description

### BACKGROUND

Sexual dysfunction refers problems that prevent a person from experiencing satisfaction from a sexual activity. Sexual dysfunction can happen during any phase of the sexual response cycle, which usually includes excitement (i.e., desire and arousal), plateau, orgasm, and resolution. Some degree of sexual dysfunction is common, reported in about 43% of women and 31% of men. Causes of sexual dysfunction can be complex, involving physical and psychological causes, and may be difficult to treat effectively WO2020/033883A1, US2022/111211A1, US10201702B2, US2013/289664A1, US2013/041430A1 and WO2010/111321A2 disclose background art.

### SUMMARY

The invention is defined by the claims. Any aspects, embodiments or examples falling outside of the claimed scope are provided for illustrative purposes. Sexual dysfunction can impact quality of life and may prevent a person from experiencing satisfaction from a sexual activity. Generally, sexual dysfunction may be classified as a desire disorder (lack of sexual desire or interest in sex), an arousal disorder (inability to become physically aroused or excited during sexual activity, an orgasm disorder (delay or absence of orgasm), or a pain disorder (pain during intercourse), or a combination of these disorders. Sexual dysfunction may involve physical and psychological causes. Often, sexual dysfunction may be treated using one or more of a medication, a mechanical aid, psychotherapy, and behavioral treatments. However, especially for sexual dysfunction with a physical cause, the effectiveness of treatment may be limited.

Aspects of the disclosure herein provide methods for method of treating a symptom of sexual dysfunction in an individual, the method comprising: (a) implanting a sensor and a stimulator electrode within the body of the individual; (b) detecting with the implanted sensor a parameter associated with an episode of sexual dysfunction or a parameter with an intent for sexual activity; and (c) providing an adapted electrical stimulation with the implanted electrode to treat the episode of sexual dysfunction, wherein at least one of an intensity, a frequency, or a duration of the adapted electrical stimulation varies according to the parameter detected in step (b). In some embodiments, the sensor, implanted electrode, or a combination thereof, is implanted in a pelvic region and/or area of the individual. In some embodiments, the sexual dysfunction comprises a dysfunction in an erection of an erectile tissue. In some embodiments, the dysfunction of the erection of the erectile tissue comprises a dysfunction of at least one of initiation, induction, or sustentation of the erection of the erectile tissue. In some embodiments, the dysfunction of the erection is measured by tumescence of the erectile tissue. In some embodiments, the dysfunction of the erection is measured by rigidity of the erectile tissue. In some embodiments, the tumescence is measured by intracorporeal pressure of the erectile tissue. In some embodiments, the erectile tissue comprises a penis or a clitoris. In some embodiments, the sexual dysfunction comprises a dysfunction in achieving arousal. In some embodiments, the dysfunction in achieving arousal comprises a dysfunction in dopamine release in striatum. In some embodiments, the adapted electrical stimulation increases dopamine release in striatum. In some embodiments, the dysfunction in achieving arousal comprises a dysfunction in nitric oxide (NO) release in the erectile tissue. In some embodiments, the adapted electrical stimulation increases nitric oxide (NO) release in the erectile tissue. In some embodiments, the sexual dysfunction comprises a dysfunction in achieving ejaculation. In some embodiments, the sexual dysfunction comprises a dysfunction in achieving orgasm. In some embodiments, the sexual dysfunction comprises vaginal dryness. In some embodiments, the adapted electrical stimulation increases vaginal lubrication. In some embodiments, the implanted electrode comprises a first stimulator implanted at or adjacent to a first anatomical site. In some embodiments, the implanted electrode further comprises a second stimulator implanted at or adjacent to a second anatomical site. In some embodiments, the first anatomical site comprises at least one of a post-ganglionic nerve, or a pre-ganglionic nerve, or a combination thereof. In some embodiments, the first anatomical site comprises at least one of a pudendal nerve, a cavernous nerve, a sacral nerve, a pelvic plexus nerve, a splanchnic nerve, or any combination thereof. In some embodiments, the second anatomical site comprises at least one of a pudendal nerve, a cavernous nerve, a sacral nerve, or a splanchnic nerve. In some embodiments, the first anatomical site comprises an afferent nerve, an efferent motor nerve, or any combination thereof. In some embodiments, the second anatomical site comprises an afferent nerve, an efferent motor nerve, or any combination thereof. In some embodiments, the first anatomical site and the second anatomical site are on different nerves. In some embodiments, the adapted electrical stimulation is configured to initiate tumescence of the erectile tissue. In some embodiments, the adapted electrical stimulation is targeted to the pudendo-cavernosal reflex loop. In some embodiments, the adapted electrical stimulation is provided to pudendal afferent nerves. In some embodiments, the adapted electrical stimulation is targeted to the bulbocavernosus reflex loop. In some embodiments, the adapted electrical stimulation is provided to efferent motor fibers from a pudendal nerve. In some embodiments, the adapted electrical stimulation stimulates an ischiocavernosus muscle, a bulbocavernosus muscle, or any combination thereof. In some embodiments, the adapted electrical stimulation is configured to initiate, induce, or sustain an increase in intracorporal pressure of the erectile tissue or a combination thereof. In some embodiments, the adapted electrical stimulation initiates, induces, or sustains an erection of the erectile tissue, or a combination thereof. In some embodiments, the adapted electrical stimulation, alone or together with a response of the individual, promotes an increase in the intracorporal pressure of the erectile tissue. In some embodiments, the adapted electrical stimulation vary over time. In some embodiments, the adapted electrical stimulation comprises a first stimulation pattern and a second stimulation pattern, wherein the first stimulation pattern is provided by the first stimulator and the second stimulation pattern is provided by the second stimulator. In some embodiments, the first and second stimulation patterns vary over time. In some embodiments, the first and second stimulation patterns differ in intensity, frequency, phase, pulse width, or any combination thereof. In some embodiments, the method comprises using software configured to generate the first and second stimulation patterns based on at least the parameter. In some embodiments, the first and second stimulation patterns are configured to promote the increase in intracorporal pressure based on at least the parameter. In some embodiments, the sensor and the stimulator electrode are electrically coupled to a processor. In some embodiments, the sensor is configured to transmit the parameter to the processor. In some embodiments, the parameter comprises a first parameter and a second parameter, wherein the second parameter is later in time than the first parameter, and wherein the adapted electrical stimulation is sustained, modified, or cancelled based on the second parameter. In some embodiments, the second parameter comprises an ending parameter, and wherein the adapted electrical stimulation is reduced or ended after detecting the ending parameter. In some embodiments, the ending parameter comprises an individual-actuated signal, an EMG signal of the pelvic floor associated with ejaculation, an ENG signal associated with ejaculation, a pressure signal associated with ejaculation, or any combination thereof. In some embodiments, the parameter comprises a physiological signal, an EMG signal, an ENG signal, a digital signal, an individual-actuated signal, a pressure signal, a motion signal, an orientation signal, a posture signal, a global positioning system (GPS) signal, a speed signal, a location signal, a time of day signal, a time interval signal, or any combination thereof. In some embodiments, the sensor comprises a sensor electrode, a receiver, a pressure sensor, an accelerometer, a gyroscope, a magnetometer, a global positioning system (GPS), or any combination thereof. In some embodiments, the receiver comprises a controller receiver, digital receiver, radio receiver, or any combination thereof. In some embodiments, the sensor is configured to detect an activity of a muscle of the individual from an EMG signal, an ENG signal, a digital signal, an individual-initiated signal, a pressure signal, a muscle contraction event signal, or any combination thereof. In some embodiments, the muscle comprises a pelvic floor muscle, an ischiocavernosus muscle, a bulbocavernosus muscle, or any combination thereof. In some embodiments, the individual-actuated signal comprises a signal associated with a desire to increase intracorporal pressure. In some embodiments, the individual-actuated signal comprises pressing of an actuator by the individual. In some embodiments, the implanted sensor is located to detect motor nerve fiber, a larger diameter afferent nerve, or any combination thereof. In some embodiments, the implanted sensor is located to detect a pudendal nerve, a sacral nerve, a cavernous nerve, a pelvic plexus nerve, a splanchnic nerve, or any combination thereof. In some embodiments, the episode of sexual dysfunction comprises a response from the individual to promote the increase in the intracorporal pressure of the erectile tissue. In some embodiments, the episode of sexual dysfunction is associated with an inadequate intracorporal pressure of the erectile tissue. In some embodiments, the response is inadequate to induce or sustain an erection of the erectile tissue or a combination thereof. In some embodiments, the response is associated with insufficient rigidity of the erectile tissue. In some embodiments, the parameter is associated with the individual getting aroused, having sexual intercourse, or any combination thereof. In some embodiments, the parameter has diurnal variation. In some embodiments, the diurnal variation comprises a temporal or a circadian variation. In some embodiments, the sensor comprises a tumescence sensor on or near the penis configured to sense the erection. In some embodiments, the tumescence sensor comprises a pressure sensor, a temperature sensor, a blood flow sensor, a stretch sensor, or a combination thereof. In some embodiments, the tumescence sensor comprises a cuff around the penis. In some embodiments, the individual uses or previously used a medicament to treat sexual dysfunction. In some embodiments, the medicament comprises a PDE5-inhibitor. In some embodiments, the medicament is ineffective in treating sexual dysfunction in the individual.

Provided herein are methods of data processing, the method comprising: receiving a measurement of a parameter associated with an episode of sexual dysfunction of an individual measured by an implanted sensor; analyzing the parameter; and generating an electrical stimulation pattern configured to treat the episode sexual dysfunction of the individual when the stimulation pattern is performed by an implanted electrode, wherein at least one of an intensity, a frequency, or a duration of the stimulation pattern varies according to the parameter that is measured. In some embodiments, the sensor, implanted electrode, or a combination thereof, is implanted in a pelvic region and/or area of the individual. In some embodiments, the treating the episode sexual dysfunction comprises promoting an increase in intracorporal pressure of an erectile tissue. In some embodiments, the method comprises using software configured to generate the electrical stimulation pattern based on the parameter. In some embodiments, the software comprises a machine learning model, and wherein the machine learning model is configured to classify the measurement received by the implanted sensor and generate the stimulation pattern. In some embodiments, the machine learning model comprises training a classifier of user-specific activity based on at least one of a GPS reading, time of day, or motion. In some embodiments, the measurement comprises a bio-signal, an extrinsic signal, a learned signal, or any combination thereof. In some embodiments, the sexual dysfunction comprises a dysfunction in an erection of an erectile tissue. In some embodiments, the dysfunction of the erection of the erectile tissue comprises a dysfunction of at least one of initiation, induction, or sustentation of the erection of the erectile tissue. In some embodiments, the dysfunction of the erection is measured by tumescence of the erectile tissue. In some embodiments, the dysfunction of the erection is measured by rigidity of the erectile tissue. In some embodiments, the tumescence is measured by intracorporeal pressure of the erectile tissue. In some embodiments, the erectile tissue comprises a penis or a clitoris. In some embodiments, the sexual dysfunction comprises a dysfunction in achieving arousal. In some embodiments, the dysfunction in achieving arousal comprises a dysfunction in dopamine release in striatum. In some embodiments, the adapted electrical stimulation increases dopamine release in striatum. In some embodiments, the dysfunction in achieving arousal comprises a dysfunction in nitric oxide (NO) release in the erectile tissue. In some embodiments, the adapted electrical stimulation increases nitric oxide (NO) release in the erectile tissue. In some embodiments, the sexual dysfunction comprises a dysfunction in achieving ejaculation. In some embodiments, the sexual dysfunction comprises a dysfunction in achieving orgasm. In some embodiments, the sexual dysfunction comprises vaginal dryness. In some embodiments, the stimulation pattern comprises an adapted electrical stimulation, where the adapted electrical stimulation increases vaginal lubrication. In some embodiments, the implanted electrode comprises a first stimulator implanted at or adjacent to a first anatomical site. In some embodiments, the implanted electrode further comprises a second stimulator implanted at or adjacent to a second anatomical site. In some embodiments, the first anatomical site comprises at least one of a post-ganglionic nerve, or a pre-ganglionic nerve, or a combination thereof. In some embodiments, the first anatomical site comprises at least one of a pudendal nerve, a cavernous nerve, a sacral nerve, a pelvic plexus nerve, a splanchnic nerve, or any combination thereof. In some embodiments, the second anatomical site comprises at least one of a pudendal nerve, a cavernous nerve, a sacral nerve, or a splanchnic nerve. In some embodiments, the first anatomical site comprises an afferent nerve, an efferent motor nerve, or any combination thereof. In some embodiments, the second anatomical site comprises an afferent nerve, an efferent motor nerve, or any combination thereof. In some embodiments, the first anatomical site and the second anatomical site are on different nerves. In some embodiments, the adapted electrical stimulation is configured to initiate tumescence of the erectile tissue. In some embodiments, the adapted electrical stimulation is targeted to the pudendo-cavernosal reflex loop. In some embodiments, the adapted electrical stimulation is provided to pudendal afferent nerves. In some embodiments, the adapted electrical stimulation is targeted to the bulbocavernosus reflex loop. In some embodiments, the adapted electrical stimulation is provided to efferent motor fibers from a pudendal nerve. In some embodiments, the adapted electrical stimulation stimulates an ischiocavernosus muscle, a bulbocavernosus muscle, or any combination thereof. In some embodiments, the adapted electrical stimulation is configured to initiate, induce, or sustain an increase in intracorporal pressure of the erectile tissue or a combination thereof. In some embodiments, the adapted electrical stimulation initiates, induces, or sustains an erection of the erectile tissue, or a combination thereof. In some embodiments, the adapted electrical stimulation, alone or together with a response of the individual, promotes an increase in the intracorporal pressure of the erectile tissue. In some embodiments, the adapted electrical stimulation vary over time. In some embodiments, the adapted electrical stimulation comprises a first stimulation pattern and a second stimulation pattern, wherein the first stimulation pattern is provided by the first stimulator and the second stimulation pattern is provided by the second stimulator. In some embodiments, the first and second stimulation patterns vary over time. In some embodiments, the first and second stimulation patterns differ in intensity, frequency, phase, pulse width, or any combination thereof. In some embodiments, the method comprises using software configured to generate the first and second stimulation patterns based on at least the parameter. In some embodiments, the first and second stimulation patterns are configured to promote the increase in intracorporal pressure based on at least the parameter. In some embodiments, the sensor and the stimulator electrode are electrically coupled to a processor. In some embodiments, the sensor is configured to transmit the parameter to the processor. In some embodiments, the parameter comprises a first parameter and a second parameter, wherein the second parameter is later in time than the first parameter, and wherein the adapted electrical stimulation is sustained, modified, or cancelled based on the second parameter. In some embodiments, the second parameter comprises an ending parameter, and wherein the adapted electrical stimulation is reduced or ended after detecting the ending parameter. In some embodiments, the ending parameter comprises an individual-actuated signal, an EMG signal of the pelvic floor associated with ejaculation, an ENG signal associated with ejaculation, a pressure signal associated with ejaculation, or any combination thereof. In some embodiments, the parameter comprises a physiological signal, an EMG signal, an ENG signal, a digital signal, an individual-actuated signal, a pressure signal, a motion signal, an orientation signal, a posture signal, a global positioning system (GPS) signal, a speed signal, a location signal, a time of day signal, a time interval signal, or any combination thereof. In some embodiments, the sensor comprises a sensor electrode, a receiver, a pressure sensor, an accelerometer, a gyroscope, a magnetometer, a global positioning system (GPS), or any combination thereof. In some embodiments, the receiver comprises a controller receiver, digital receiver, radio receiver, or any combination thereof. In some embodiments, the sensor is configured to detect an activity of a muscle of the individual from an EMG signal, an ENG signal, a digital signal, an individual-initiated signal, a pressure signal, a muscle contraction event signal, or any combination thereof. In some embodiments, the muscle comprises a pelvic floor muscle, an ischiocavernosus muscle, a bulbocavernosus muscle, or any combination thereof. In some embodiments, the individual-actuated signal comprises a signal associated with a desire to increase intracorporal pressure. In some embodiments, the individual-actuated signal comprises pressing of an actuator by the individual. In some embodiments, the implanted sensor is located to detect motor nerve fiber, a larger diameter afferent nerve, or any combination thereof. In some embodiments, the implanted sensor is located to detect a pudendal nerve, a sacral nerve, a cavernous nerve, a pelvic plexus nerve, a splanchnic nerve, or any combination thereof. In some embodiments, the episode of sexual dysfunction comprises a response from the individual to promote the increase in the intracorporal pressure of the erectile tissue. In some embodiments, the episode of sexual dysfunction is associated with an inadequate intracorporal pressure of the erectile tissue. In some embodiments, the response is inadequate to induce or sustain an erection of the erectile tissue or a combination thereof. In some embodiments, the response is associated with insufficient rigidity of the erectile tissue. In some embodiments, the parameter is associated with the individual getting aroused, having sexual intercourse, or any combination thereof. In some embodiments, the parameter has diurnal variation. In some embodiments, the diurnal variation comprises a temporal or a circadian variation. In some embodiments, the sensor comprises a tumescence sensor on or near the penis configured to sense the erection. In some embodiments, the tumescence sensor comprises a pressure sensor, a temperature sensor, a blood flow sensor, a stretch sensor, or a combination thereof. In some embodiments, the tumescence sensor comprises a cuff around the penis. In some embodiments, the individual uses or previously used a medicament to treat sexual dysfunction. In some embodiments, the medicament comprises a PDE5-inhibitor. In some embodiments, the medicament is ineffective in treating sexual dysfunction in the individual.

Described herein are systems for treating an episode of sexual dysfunction of an individual, the system comprising: a sensor configured to sense a parameter associated with the episode of sexual dysfunction of the individual; a stimulator electrode configured to provide an adapted electrical stimulation; a processor operably coupled to the stimulator electrode and the sensor; and a non-transitory computer readable storage medium comprising a software configured to cause the processor to: receive from the sensor a measurement of the parameter associated with the episode of sexual dysfunction; analyze the measurement to determine at least one of an intensity, a frequency, or a duration of the adapted electrical stimulation; and cause the stimulator electrode to provide the adapted electrical stimulation, wherein the adapted electrical stimulation provided is sufficient to treat the episode of sexual dysfunction. In some embodiments, the sensor, implanted electrode, or a combination thereof, is implanted in a pelvic region and/or area of the individual. In some embodiments, the treating the episode of sexual dysfunction comprises promoting an increase in intracorporal pressure of an erectile tissue during the episode of sexual dysfunction. In some embodiments, at least one of the intensity, frequency, or duration of the adapted electrical stimulation varies according to the parameter that is detected by the sensor. In some embodiments, the adapted electrical stimulation, alone or together with a response from the individual, promotes the increase in intracorporal pressure of the erectile tissue. In some embodiments, the sexual dysfunction comprises a dysfunction in an erection of an erectile tissue. In some embodiments, the dysfunction of the erection of the erectile tissue comprises a dysfunction of at least one of initiation, induction, or sustentation of the erection of the erectile tissue. In some embodiments, the dysfunction of the erection is measured by tumescence of the erectile tissue. In some embodiments, the dysfunction of the erection is measured by rigidity of the erectile tissue. In some embodiments, the tumescence is measured by intracorporeal pressure of the erectile tissue. In some embodiments, the erectile tissue comprises a penis or a clitoris. In some embodiments, the sexual dysfunction comprises a dysfunction in achieving arousal. In some embodiments, the dysfunction in achieving arousal comprises a dysfunction in dopamine release in striatum. In some embodiments, the adapted electrical stimulation increases dopamine release in striatum. In some embodiments, the dysfunction in achieving arousal comprises a dysfunction in nitric oxide (NO) release in the erectile tissue. In some embodiments, the adapted electrical stimulation increases nitric oxide (NO) release in the erectile tissue. In some embodiments, the sexual dysfunction comprises a dysfunction in achieving ejaculation. In some embodiments, the sexual dysfunction comprises a dysfunction in achieving orgasm. In some embodiments, the sexual dysfunction comprises vaginal dryness. In some embodiments, the adapted electrical stimulation increases vaginal lubrication. In some embodiments, the implanted electrode comprises a first stimulator implanted at or adjacent to a first anatomical site. In some embodiments, the implanted electrode further comprises a second stimulator implanted at or adjacent to a second anatomical site. In some embodiments, the first anatomical site comprises at least one of a post-ganglionic nerve, or a pre-ganglionic nerve, or a combination thereof. In some embodiments, the first anatomical site comprises at least one of a pudendal nerve, a cavernous nerve, a sacral nerve, a pelvic plexus nerve, a splanchnic nerve, or any combination thereof. In some embodiments, the second anatomical site comprises at least one of a pudendal nerve, a cavernous nerve, a sacral nerve, or a splanchnic nerve. In some embodiments, the first anatomical site comprises an afferent nerve, an efferent motor nerve, or any combination thereof of the erectile tissue. In some embodiments, the second anatomical site comprises an afferent nerve, an efferent motor nerve, or any combination thereof of the erectile tissue. In some embodiments, the first anatomical site and the second anatomical site are on different nerves. In some embodiments, the adapted electrical stimulation is configured to initiate tumescence of the erectile tissue. In some embodiments, the adapted electrical stimulation is targeted to the pudendo-cavernosal reflex loop. In some embodiments, the adapted electrical stimulation is provided to pudendal afferent nerves. In some embodiments, the adapted electrical stimulation is targeted to the bulbocavernosus reflex loop. In some embodiments, the adapted electrical stimulation is provided to efferent motor fibers from a pudendal nerve. In some embodiments, the adapted electrical stimulation stimulates an ischiocavernosus muscle, a bulbocavernosus muscle, or any combination thereof. In some embodiments, the adapted electrical stimulation is configured to initiate, induce, or sustain an increase in intracorporal pressure of the erectile tissue or a combination thereof. In some embodiments, the adapted electrical stimulation initiates, induces, or sustains an erection of the erectile tissue, or a combination thereof. In some embodiments, the adapted electrical stimulation, alone or together with a response of the individual, promotes an increase in the intracorporal pressure of the erectile tissue. In some embodiments, the adapted electrical stimulation vary over time. In some embodiments, the adapted electrical stimulation comprises a first stimulation pattern and a second stimulation pattern, wherein the first stimulation pattern is provided by the first stimulator and the second stimulation pattern is provided by the second stimulator. In some embodiments, the first and second stimulation patterns vary over time. In some embodiments, the first and second stimulation patterns differ in intensity, frequency, phase, pulse width, or any combination thereof. In some embodiments, the method comprises using software configured to generate the first and second stimulation patterns based on at least the parameter. In some embodiments, the first and second stimulation patterns are configured to promote the increase in intracorporal pressure based on at least the parameter. In some embodiments, the sensor and the stimulator electrode are electrically coupled to a processor. In some embodiments, the sensor is configured to transmit the parameter to the processor. In some embodiments, the parameter comprises a first parameter and a second parameter, wherein the second parameter is later in time than the first parameter, and wherein the adapted electrical stimulation is sustained, modified, or cancelled based on the second parameter. In some embodiments, the second parameter comprises an ending parameter, and wherein the adapted electrical stimulation is reduced or ended after detecting the ending parameter. In some embodiments, the ending parameter comprises an individual-actuated signal, an EMG signal of the pelvic floor associated with ejaculation, an ENG signal associated with ejaculation, a pressure signal associated with ejaculation, or any combination thereof. In some embodiments, the parameter comprises a physiological signal, an EMG signal, an ENG signal, a digital signal, an individual-actuated signal, a pressure signal, a motion signal, an orientation signal, a posture signal, a global positioning system (GPS) signal, a speed signal, a location signal, a time of day signal, a time interval signal, or any combination thereof. In some embodiments, the sensor comprises a sensor electrode, a receiver, a pressure sensor, an accelerometer, a gyroscope, a magnetometer, a global positioning system (GPS), or any combination thereof. In some embodiments, the receiver comprises a controller receiver, digital receiver, radio receiver, or any combination thereof. In some embodiments, the sensor is configured to detect an activity of a muscle of the individual from an EMG signal, an ENG signal, a digital signal, an individual-initiated signal, a pressure signal, a muscle contraction event signal, or any combination thereof. In some embodiments, the muscle comprises a pelvic floor muscle, an ischiocavernosus muscle, a bulbocavernosus muscle, or any combination thereof. In some embodiments, the individual-actuated signal comprises a signal associated with a desire to increase intracorporal pressure. In some embodiments, the individual-actuated signal comprises pressing of an actuator by the individual. In some embodiments, the implanted sensor is located to detect motor nerve fiber, a larger diameter afferent nerve, or any combination thereof. In some embodiments, the implanted sensor is located to detect a pudendal nerve, a sacral nerve, a cavernous nerve, a pelvic plexus nerve, a splanchnic nerve, or any combination thereof. In some embodiments, the episode of sexual dysfunction comprises a response from the individual to promote the increase in the intracorporal pressure of the erectile tissue. In some embodiments, the episode of sexual dysfunction is associated with an inadequate intracorporal pressure of the erectile tissue. In some embodiments, the response is inadequate to induce or sustain an erection of the erectile tissue or a combination thereof. In some embodiments, the response is associated with insufficient rigidity of the erectile tissue. In some embodiments, the parameter is associated with the individual getting aroused, having sexual intercourse, or any combination thereof. In some embodiments, the parameter has diurnal variation. In some embodiments, the diurnal variation comprises a temporal or a circadian variation. In some embodiments, the sensor comprises a tumescence sensor on or near the penis configured to sense the erection. In some embodiments, the tumescence sensor comprises a pressure sensor, a temperature sensor, a blood flow sensor, a stretch sensor, or a combination thereof. In some embodiments, the tumescence sensor comprises a cuff around the penis. In some embodiments, the individual uses or previously used a medicament to treat sexual dysfunction. In some embodiments, the medicament comprises a PDE5-inhibitor. In some embodiments, the medicament is ineffective in treating sexual dysfunction in the individual.

Provided herein are non-transitory computer readable storage mediums including software for treating an episode of sexual dysfunction of an individual, configured to cause a processor to: (a) receive from an implanted sensor a measurement of a parameter associated with an episode of sexual dysfunction; (b) analyze the measurement to determine at least one of an intensity, a frequency, or a duration of an adapted electrical stimulation; and (c) cause an implanted stimulator electrode to provide an adapted electrical stimulation to the individual, wherein the adapted electrical stimulation, alone or together with a response of the individual, is configured to the episode of sexual dysfunction. In some embodiments, the sensor, implanted electrode, or a combination thereof, is implanted in a pelvic region and/or area of the individual. In some embodiments, the treating the episode of sexual dysfunction comprises promoting an increase in intracorporal pressure of an erectile tissue during the episode of sexual dysfunction. In some embodiments, the software comprises a machine learning model, and wherein the machine learning model is configured to classify the measurement and generate at least one stimulation pattern configured to treat the episode of sexual dysfunction when the at least one stimulation pattern is provided by the implanted electrode to the individual. In some embodiments, the at least one stimulation pattern is provided to a pudendal nerve, a sacral nerve, a cavernous nerve, a splanchnic nerve, a pelvic plexus nerve, or any combination thereof. In some embodiments, the sexual dysfunction comprises a dysfunction in an erection of an erectile tissue. In some embodiments, the dysfunction of the erection of the erectile tissue comprises a dysfunction of at least one of initiation, induction, or sustentation of the erection of the erectile tissue. In some embodiments, the dysfunction of the erection is measured by tumescence of the erectile tissue. In some embodiments, the dysfunction of the erection is measured by rigidity of the erectile tissue. In some embodiments, the tumescence is measured by intracorporeal pressure of the erectile tissue. In some embodiments, the erectile tissue comprises a penis or a clitoris. In some embodiments, the sexual dysfunction comprises a dysfunction in achieving arousal. In some embodiments, the dysfunction in achieving arousal comprises a dysfunction in dopamine release in striatum. In some embodiments, the adapted electrical stimulation increases dopamine release in striatum. In some embodiments, the dysfunction in achieving arousal comprises a dysfunction in nitric oxide (NO) release in the erectile tissue. In some embodiments, the adapted electrical stimulation increases nitric oxide (NO) release in the erectile tissue. In some embodiments, the sexual dysfunction comprises a dysfunction in achieving ejaculation. In some embodiments, the sexual dysfunction comprises a dysfunction in achieving orgasm. In some embodiments, the sexual dysfunction comprises vaginal dryness. In some embodiments, the adapted electrical stimulation increases vaginal lubrication. In some embodiments, the implanted electrode comprises a first stimulator implanted at or adjacent to a first anatomical site. In some embodiments, the implanted electrode further comprises a second stimulator implanted at or adjacent to a second anatomical site. In some embodiments, the first anatomical site comprises at least one of a post-ganglionic nerve, or a pre-ganglionic nerve, or a combination thereof. In some embodiments, the first anatomical site comprises at least one of a pudendal nerve, a cavernous nerve, a sacral nerve, a pelvic plexus nerve, a splanchnic nerve, or any combination thereof. In some embodiments, the second anatomical site comprises at least one of a pudendal nerve, a cavernous nerve, a sacral nerve, or a splanchnic nerve. In some embodiments, the first anatomical site comprises an afferent nerve, an efferent motor nerve, or any combination thereof of the erectile tissue. In some embodiments, the second anatomical site comprises an afferent nerve, an efferent motor nerve, or any combination thereof. In some embodiments, the first anatomical site and the second anatomical site are on different nerves. In some embodiments, the adapted electrical stimulation is configured to initiate tumescence of the erectile tissue. In some embodiments, the adapted electrical stimulation is targeted to the pudendo-cavernosal reflex loop. In some embodiments, the adapted electrical stimulation is provided to pudendal afferent nerves. In some embodiments, the adapted electrical stimulation is targeted to the bulbocavernosus reflex loop. In some embodiments, the adapted electrical stimulation is provided to efferent motor fibers from a pudendal nerve. In some embodiments, the adapted electrical stimulation stimulates an ischiocavernosus muscle, a bulbocavernosus muscle, or any combination thereof. In some embodiments, the adapted electrical stimulation is configured to initiate, induce, or sustain an increase in intracorporal pressure of the erectile tissue or a combination thereof. In some embodiments, the adapted electrical stimulation initiates, induces, or sustains an erection of the erectile tissue, or a combination thereof. In some embodiments, the adapted electrical stimulation, alone or together with a response of the individual, promotes an increase in the intracorporal pressure of the erectile tissue. In some embodiments, the adapted electrical stimulation vary over time. In some embodiments, the adapted electrical stimulation comprises a first stimulation pattern and a second stimulation pattern, wherein the first stimulation pattern is provided by the first stimulator and the second stimulation pattern is provided by the second stimulator. In some embodiments, the first and second stimulation patterns vary over time. In some embodiments, the first and second stimulation patterns differ in intensity, frequency, phase, pulse width, or any combination thereof. In some embodiments, the method comprises using software configured to generate the first and second stimulation patterns based on at least the parameter. In some embodiments, the first and second stimulation patterns are configured to promote the increase in intracorporal pressure based on at least the parameter. In some embodiments, the sensor and the stimulator electrode are electrically coupled to a processor. In some embodiments, the sensor is configured to transmit the parameter to the processor. In some embodiments, the parameter comprises a first parameter and a second parameter, wherein the second parameter is later in time than the first parameter, and wherein the adapted electrical stimulation is sustained, modified, or cancelled based on the second parameter. In some embodiments, the second parameter comprises an ending parameter, and wherein the adapted electrical stimulation is reduced or ended after detecting the ending parameter. In some embodiments, the ending parameter comprises an individual-actuated signal, an EMG signal of the pelvic floor associated with ejaculation, an ENG signal associated with ejaculation, a pressure signal associated with ejaculation, or any combination thereof. In some embodiments, the parameter comprises a physiological signal, an EMG signal, an ENG signal, a digital signal, an individual-actuated signal, a pressure signal, a motion signal, an orientation signal, a posture signal, a global positioning system (GPS) signal, a speed signal, a location signal, a time of day signal, a time interval signal, or any combination thereof. In some embodiments, the sensor comprises a sensor electrode, a receiver, a pressure sensor, an accelerometer, a gyroscope, a magnetometer, a global positioning system (GPS), or any combination thereof. In some embodiments, the receiver comprises a controller receiver, digital receiver, radio receiver, or any combination thereof. In some embodiments, the sensor is configured to detect an activity of a muscle of the individual from an EMG signal, an ENG signal, a digital signal, an individual-initiated signal, a pressure signal, a muscle contraction event signal, or any combination thereof. In some embodiments, the muscle comprises a pelvic floor muscle, an ischiocavernosus muscle, a bulbocavernosus muscle, or any combination thereof. In some embodiments, the individual-actuated signal comprises a signal associated with a desire to increase intracorporal pressure. In some embodiments, the individual-actuated signal comprises pressing of an actuator by the individual. In some embodiments, the implanted sensor is located to detect motor nerve fiber, a larger diameter afferent nerve, or any combination thereof. In some embodiments, the implanted sensor is located to detect a pudendal nerve, a sacral nerve, a cavernous nerve, a pelvic plexus nerve, a splanchnic nerve, or any combination thereof. In some embodiments, the episode of sexual dysfunction comprises a response from the individual to promote the increase in the intracorporal pressure of the erectile tissue. In some embodiments, the episode of sexual dysfunction is associated with an inadequate intracorporal pressure of the erectile tissue. In some embodiments, the response is inadequate to induce or sustain an erection of the erectile tissue or a combination thereof. In some embodiments, the response is associated with insufficient rigidity of the erectile tissue. In some embodiments, the parameter is associated with the individual getting aroused, having sexual intercourse, or any combination thereof. In some embodiments, the parameter has diurnal variation. In some embodiments, the diurnal variation comprises a temporal or a circadian variation. In some embodiments, the sensor comprises a tumescence sensor on or near the penis configured to sense the erection. In some embodiments, the tumescence sensor comprises a pressure sensor, a temperature sensor, a blood flow sensor, a stretch sensor, or a combination thereof. In some embodiments, the tumescence sensor comprises a cuff around the penis. In some embodiments, the individual uses or previously used a medicament to treat sexual dysfunction. In some embodiments, the medicament comprises a PDE5-inhibitor. In some embodiments, the medicament is ineffective in treating sexual dysfunction in the individual.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** shows an exemplary embodiment of an open-loop bioelectronic system comprising an implantable pulse generator. The system delivers a predefined stimulation protocol and does not receive input from the subject.
**FIG. 2** shows an exemplary embodiment of a closed-loop bioelectronic system comprising a two-way neural interface comprising a sensor configured to capture a neural response of the individual and a processing module configured to interpret the neural response. The system can deliver an adapted stimulation based on the neural response.
**FIGS. 3A-3C** show anatomical structures and pathways in which the bioelectronic system is configured to interface with.
**FIG. 4A** shows an exemplary embodiment of the devices and methods described herein targeting a peripheral nerve, such as a pudendal nerve, by placing the sensor and stimulator electrodes near the nerve. The sensor captures a bio-signal associated with an episode of pain to be analyzed and classified, and the stimulator delivers an adapted electrical stimulation, wherein the adapted electrical stimulation was generated according to analyzed parameters comprising the bio-signal.
**FIG. 4B** shows exemplary embodiments of depicting a bio-signal and a bio-stimulator electrical stimulation.
**FIG. 5** shows an exemplary embodiment of a system block diagram for the devices and methods described herein configured to implement slow- and fast-adapting algorithms. API is an application programming interface; MICS is the Medical Information and Communication band.
**FIGS. 6A-6F** show a flowchart of a method of closed-loop operation of the device of the disclosure.
**FIGS. 7A-7B** show an example embodiment of the patient controller module, as described in some embodiments herein.
**FIG. 8** shows a flow diagram for purposeful patient contraction and manual operation of the devices and systems, as described in some embodiments herein.
**FIG. 9** shows a flow diagram for the closed loop system operation, as described in some embodiments herein.
**FIG. 10** shows a flow diagram for signal processing and threshold detection of signals associated with sexual dysfunction, as described in some embodiments herein.
**FIGS. 11A-11E** show patient purposeful muscle contraction, Valsalva maneuver, and coughing EMG data acquired and processed with the methods of the disclosure, as described in some embodiments herein.
**FIGS. 12A-12B** show flow diagrams for detecting a purposeful or intent based contraction by the patient and providing electrical stimulation to treat a sexual dysfunction event, as described in some embodiments herein.
**FIG. 13** shows a flow diagram for training on-board machine learning classifier of the devices and systems of the disclosure, as described in some embodiments herein.

### DETAILED DESCRIPTION

Sexual dysfunction is a very common problem with a large effect on quality of life, preventing a person from experiencing satisfaction from a sexual activity. Sexual dysfunction is a complex condition, often involving physical and psychological causes. Sexual dysfunction may occur during one or more phases of the sexual response cycle, which includes excitement (*i.e.*, desire and arousal), plateau, orgasm, and resolution. Often, sexual dysfunction is classified as a desire disorder (lack of sexual desire or interest in sex), an arousal disorder (inability to become physically aroused or excited during sexual activity, an orgasm disorder (delay or absence of orgasm), or a pain disorder (pain during intercourse), or a combination of these disorders.

A number of symptoms are associated with sexual dysfunction. In both men and women, symptoms of sexual dysfunction include but are not limited to a lack of interest in or desire for sex, inability to become aroused, and pain with intercourse. In women, some symptoms associated with sexual dysfunction include but are not limited to inability to achieve orgasm, inadequate vaginal lubrication before and during intercourse, and inability to relax the pelvic floor muscles surrounding the vagina to allow intercourse.

In some embodiments, woman (female) sexual dysfunction (FSD) may comprise hypoactive sexual desire disorder (HSDD), female sexual arousal disorder (FSAD), female orgasmic disordered, sexual pain disorder, or any combination thereof. In some cases, HSDD is characterized by a reduced or absent interest in sexual activity. In some instances, FSAD is characterized by an inability to attain or maintain sexual excitement. In some cases, female orgasmic disorder is characterized by the difficult of a woman to attain orgasm despite sufficient arousal. In some instances, sexual pain disorder is characterized by pain during sexual intercourse. In some cases, HSDD and FSAD may be combined into a single diagnosis of female sexual interest/arousal disorder (FSIAD). In some cases, FSIAD may be characterized as sexual dysfunction of a woman present for at least six months and/or has caused a source of personal distress for the woman experiencing sexual dysfunction.

In some cases, FSAD test measures: desire, subjective arousal, lubrication, orgasm, sexual satisfaction, pain, or any combination thereof. In some instances, FSAD test may comprise a minimum score of 2 and a maximum score of 36. In some instances, each measured area of the test may comprise one or more items and/or questions. For example, desire may comprise 2 questions, arousal may comprise 4 questions, lubrication may comprise 4 questions, orgasm may comprise 3 questions, sexual satisfaction may comprise 3 questions, and pain may comprise 3 items. Each tested category of FSAD may be scored on a Likert-type scale ranging from 0 to 6. In some cases, the score of each tested category is summed and multiplied by a domain factor ratio. In some cases, the domain factor ratios for the tested categories may comprise 0.6 for desire, 0.3 for arousal, 0.3 for lubrication, 0.4 for orgasm, 0.4 for sexual satisfaction, and 0.4 for pain. A FSAD score of up to about 26 may indicate FSD experienced by a woman.

In some cases, HSDD is measured by analyzing the Female Sexual Function Index-Desire Doman (FSFI-D) and the Female Sexual Distress Scale-Revised (FSDS-R). In some cases, the FSFI-D comprises a self-rated measure of a women's sexual function. In some cases, the FSFI-D measures the frequency and degree of sexual desire of a woman. In some instances, the FSFI-D may comprise a score range from about 1.2 to about 6.0, where a score of 6 indicates higher sexual desire. In some cases, FSFI-D comprises a 19-item questionnaire that evaluates sexual functioning of women. In some cases, FSFI-D comprises six subcategories that assess: desire, arousal, lubrication, orgasm, satisfaction, and/or pain. In some instances, the FSFI-D score minimum comprises a score of 2 and the FSFI-D score maximum comprises a score of 36. The FSFI-D score may comprise a sum of the six subcategories, each of which has a maximum score of 6. Each subcategory may comprise questions scored from 0-5 (for subcategories arousal, lubrication, organism, and/or pain) or 1-5 (for subcategories of desire and/or satisfaction). The scores for each category may be summed and multiplied by 0.3 (arousal, lubrication), 0.4 (orgasm, satisfaction, pain) or 0.6 (desire).

In some cases, HSDD may be measured and/or asses by sexual interest and desire inventory (SIDI-F). In some cases, FSDS-R comprises a questionnaire resulting in a women's self-reported sexual distress. In some instances, the FSDS-R comprises 14 questions. In some cases, the FSDS-R comprises a score range from 0 to 52, with a score of 52 indicating more sexually related distress. 13 items and/or questions of the FSDS-R may comprise a rating of each of the terms of a given question or item in terms of frequency from 0 (never) to 4 (always). A FSDS-R score of at least about 11 may indicate a woman experiencing FSD.

In some cases, SIDI-F comprises a 19-item scale that assess the intensity and frequency of sexual desire of sexual functioning in women. In some instances the 19 items may comprise 3 items for satisfaction, 4 items for arousal, 4 items for lubrication, 3 items for orgasm, and 5 items that combine frequency and intensity of certain aspects of sexual functioning. In some cases, each item is scored on a 5-point Likert scale ranging from 1 to 5, higher scores indicating greater levels of sexual functioning on a respective item. In some cases, SIDI-F may comprise a minimum score of 0 and a maximum score of 51. In some instances, a total score of 33 or less indicates presence of HSDD.

In some cases, the individual to receive an implanted device and/or system, as described elsewhere herein, to treat sexual dysfunction comprises a female adult woman with an age of at least 18 years of age. In some cases, the individual may comprise a woman who is sexually active at least 1 time per month. In some cases, the individual may comprise a woman who obtained a SFI score of up to about 26.55. In some cases, the individual may comprise a woman who is neurologically stable and ambulatory.

In men, some symptoms associated with sexual dysfunction include but are not limited to erectile dysfunction (ED), retarded ejaculation, and premature ejaculation. In some cases, ED refers to the inability to achieve or maintain an erection suitable for intercourse. In some cases, a retarded ejaculation refers to absent or delayed ejaculation despite sufficient sexual stimulation. In some cases, a premature ejaculation refers to an inability to control the timing of ejaculation.

ED is the most common form of sexual dysfunction in men. It is estimated that 322 million men worldwide will be affected by ED by 2025. ED has various etiologies, including but not limited to vascular, hormonal, and neurogenic causes. Individuals having neurogenic causes of ED include individuals with spinal cord injuries (SCI) and men after radical prostatectomy. Global incidence of SCI ranges from 40-80 new case per million of population per year, with 20-30 million men affected in the U.S. alone. A significant proportion of SCI individuals experiencing ED are young, where ED may significantly affect the quality of life.

In some cases, ED is diagnosed, screened, and/or monitored with one or more questionnaires and/or item-based tests. In some cases, the one or more questionnaires and/or item based tests comprise the Erection Hardness Score (EHS), sexual health inventory for men (SHIM), international index of erectile function (IIEF), male sexual health questionnaire, or any combination thereof. In some cases, color duplex doppler ultrasonography of the penis tissue is used to measure blood inflow into the penis as a measure of ED. In some instances, an axial rigidity of a penis is measured by Rigi scan.

In some cases, the IIEF comprises 15 questions that quantify 5 domains e.g., sexual desire, erectile function, intercourse satisfaction, ejaculatory and/or orgasmic function, overall sexual satisfaction, or any combination thereof. In some cases, the erectile function domain quantifies ED severity on a scale of 5-30. In some cases, scores of 26-30 indicate normal erectile function, scores of 18-25 indicate mild ED, scores of 11-17 indicate moderate ED, and scores up to 10 indicate severe ED. In some cases, the male sexual health questionnaire may comprise 25 questions divided into subclasses for erection, ejaculation, and satisfaction.

In some cases, the EHS comprises a single-time questionnaire that prompts men to rate erection hardness on a scale that ranges from 0 (penis does not enlarge) to 4 (penis is complete hard and fully rigid). In some cases, the SHIM comprises a questionnaire of 5 questions scored from 1 to 5. In some instances, total scores of SHIM of 22-25 are interpreted as no ED, scores of 17-21 as mild ED, scores of 12-16 as mild-to-moderate ED, scores of 8-11 as moderate ED, and scores of 5-7 as severe ED.

In some embodiments, the individual to receive an implanted device and/or system, as described elsewhere herein, to treat sexual dysfunction comprises a male individual with an age between 40 and 75 years of age. In some instances, the male individual comprises known or suspected vasculogenic erectile dysfunction based on the male individual's clinical history. In some cases, the male individual comprises a male individual who is in an active sexual relationship with at least 2 sexual arousal and/or intercourse attempts per month for at least one month prior to implantation. In some cases, the male individual comprises a male individual suffering for ED for at least 6 months. In some cases, the male individual comprises an IIEF erectile function domain score of about 17 to about 25 and an erection hardness domain score of at least 1. In some cases, the male individual comprises an IIEF score of at least 26 prior to a prostatectomy. In some cases, the male individual comprises a male individual undergoing a prostatectomy. In some cases, the male individual undergoing prostatectomy may receive one or more implanted electrode leads (e.g., stimulator electrode), sensors, stimulator, or any combination thereof, without the male individual exhibiting ED prior to the prostatectomy. In some cases, the male individual comprises testosterone levels between 300 and 100ng/dL within one month prior to receiving the implanted device and/or system, as described elsewhere herein. In some cases, the male individual comprises Hbg A1c of at least 8% within one prior to receiving the implanted device and/or system, as described elsewhere herein. In some cases, the male individual comprises an indication for nerve-sparing prostatectomy surgery. In some cases, the male individual comprises cancer at stage T1c or T2a. In some cases, the male individual comprises a gleason score of 6 or 7.

In some embodiments, to achieve an erection of the erectile tissue, contributions from both nervous and vascular components may be needed. In some embodiments, a normal erection relies on two reflex loops, pudendo-cavernosal reflex loop (for tumescence) and bulbocavernosus reflex loop (for rigidity). In some embodiments, an adequate nerve activity comprises release of nitric oxide (NO), leading to increase in cGMP, which in turn induce smooth muscle relaxation in the penile corpora. In some embodiments, the nervous arrangement for an erection is complex and relies on both the somatic nervous systems (via pudendal nerve (PN)) and autonomic nervous systems (via cavernous nerve (CN)).

Usually, sexual dysfunction is treated using one or more of approaches, including but not limited to medication, mechanical aid, psychotherapy, and behavioral treatments. In some cases, the medication, also referred herein as medicament, comprises a hormone, shot, pill, or cream; or a phosphodiesterase type 5 (PDE5) inhibitor to increase blood flow to the penis; or injection papaverine, phentolamine, and/or Prostaglandin E1 (PGE1) at or near the erectile tissue. Often, PDE5 inhibitors are a first line treatment and include but are not limited to sildenafil, tadalafil, vardenafil, avanafil, lodenafil, udenafil, and mirodenafil. Although PDE5 inhibitors are effective in treating sexual function, discontinuation rates may be high, reported as high as 50% after 1-2 years of use. In some cases, intracavernosal injections with papaverine, phentolamine, and/or PGE1 may be administered to the patient if medication is ineffective. Pharmaceutical treatments for female sexual dysfunction are also limited in providing treatment to female sexual interest/arousal disorder FSIAD, HSDD, FSAD, or any combination thereof. In some cases, intracavernosal injections result in a high incidence of adverse effects, such as priapism, injection site pain, bruising. In some cases, high incidences of adverse effects lead to patient non-compliance. In some cases, mechanical aids include but are not limited to vacuum devices, penile implants, vaginal dilators, and vibrators. In some cases, penile prosthesis implantation result in significant complications, such as infection, erosion and pain. As such, the effectiveness of treatment may be limited, especially for sexual dysfunction with a physical etiology and when the patient compliance or response drops.

In some cases, electrical stimulation has been used as a treatment for sexual dysfunction with limited success. Sacral nerve modulation has been used as a treatment for sexual dysfunction with very limited efficacy, with a reported successful intercourse in 20-30% of patients. In some cases, sacral anterior root stimulation (SARS) has been used to treat ED. However, SARS procedure may be quite invasive as rhizotomy is required and is only indicated in patients with complete SCI.

In some cases, the difficulty in treating sexual dysfunction by electrical stimulation may be due to the complex organization of the nervous system involved in sexual function, such as erection, including but not limited to the pudendal and cavernous nerves. As such, electrical stimulation by electrodes at multiple nerves involved in sexual function, such as pudendal and cavernous nerves, may restore sexual function, such as erection. In some cases, electrical stimulation at both pudendal and cavernous nerves may be able to treat sexual dysfunction due to neurogenic (e.g, spinal cord injury, post-prostatectomy) or combined neurogenic/vascular etiology (e.g., diabetes mellitus, idiopathic). In some cases, the erection produced by electrostimulation may be potentiated by PDE5 inhibitor (PDE5i). In some cases, the mechanisms of action may be directly linked to PDE5i as PDE5i inhibits the degradation of the CGM by inhibiting NOS. In some cases, the potential side effect of electrostimulation of somatic nerves are reduced due to the anatomy of somatic nerves. In some cases, the potential side effect of electrostimulation of somatic nerves are reduced as the proportion of somatic nerves surrounding the prostate apex represent <5% of the autonomic nerves.

Provided herein are devices, systems, and methods for treating a symptom of sexual dysfunction in an individual using electrical nerve stimulation. The systems, methods, and devices, described herein are directed to treating sexual dysfunction by targeted nerve peripheral stimulation and restoration and/or augmentation of reflex activity involved in sexual function. In some embodiments, adapted stimulation to the target nerves are provided by an implanted stimulator with an underlying physiological rationale to target both somatic nervous system (e.g., PN) and autonomic nervous system (e.g., CN), which are is involved in erection and sexual function. In some embodiments, adapted stimulation to the target nerves are provided by an implanted stimulator to target an autonomic nerve (e.g., CN), a somatic nerve (e.g., PN), or a combination thereof. In some instances, the devices, systems, and methods described herein are configured to restore erection in case of neurogenic (e.g., spinal cord injury, post-prostatectomy) and combined neurogenic/vascular etiology (e.g., diabetes mellitus, idiopathic). In some embodiments, the systems, methods, and devices, comprise a closed-loop configuration for providing the electrical stimulation.

Described herein are devices, systems, and methods for treating a symptom of sexual dysfunction in an individual, the method comprising: (a) implanting a sensor and a stimulator electrode within the body of the individual; (b) detecting with the implanted sensor a parameter associated with an episode of sexual dysfunction; and (c) providing an adapted electrical stimulation with the implanted electrode to treat the episode of sexual dysfunction, wherein at least one of an intensity, a frequency, or a duration of the adapted electrical stimulation varies according to the parameter detected in step (b).

### Physiology

Penile and clitoral erection may be controlled by spinal autonomic centers, the activity of which may be dependent on input from supraspinal centers and genitalia. In some cases, an erection can be a culmination of a number of successful nerve reflexes that initiate a vascular event. In some cases, a combination of neurovascular cavernosal reactivity, venous occlusion, and rhythmic perineal muscle contraction are needed for maintenance of erection and rigidity of the erectile tissue.

In some cases, an erection occurs through various mechanisms, including but not limited to a reflexogenic mechanism, a psychologic mechanism, and during REM sleep. During sexual activities, the reflexogenic and psychologic mechanisms both contribute to the erectile response. In men, by stroking the penis, somatic afferent receptors may be stimulated. In some cases, the somatic afferent receptors may converge and form bundles that enter a main branch of the pudendal nerve via the dorsal nerve of the penis (DNP) on the dorsal surface of the penis and the perineal nerves on the ventral surface and frenulum. In women, somatic afferent receptors may be stimulated by clitoral stimulation.

Usually, an erectile tissue, such as a penis and a clitoris, has a dual innervation, involving somatic and autonomic innervation. In some embodiments, the devices, systems, and methods provided herein target a somatic nerve, an autonomic nerve, or a combination thereof.

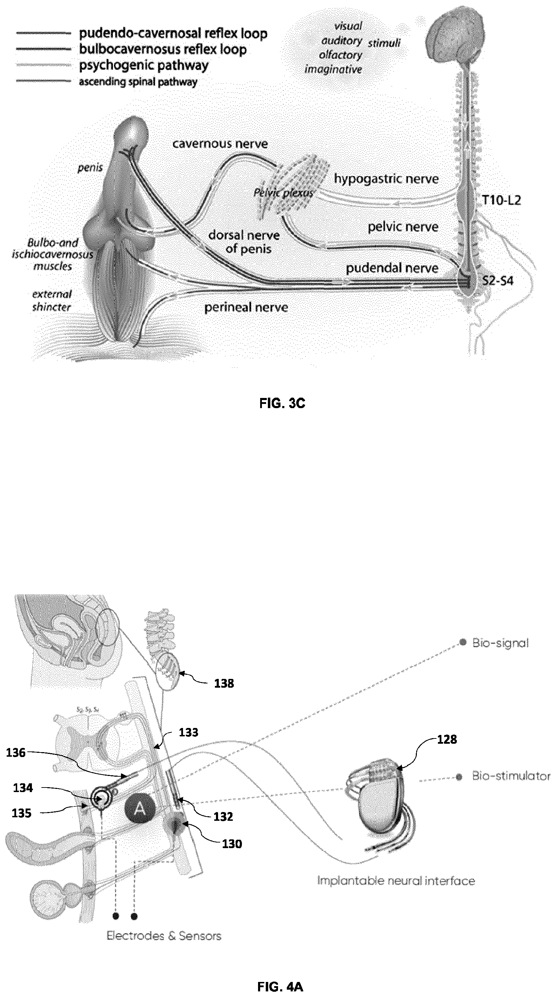

With the somatic nerve involvement in sexual function, the dorsal nerve (DN) of the penis and clitoris may be considered a very important nerve in sexual function and satisfaction. In some embodiments, the DN is a somatic nerve. In some embodiments, the DN is a continuation from the pudendal nerve (PN), arising from the sacral spinal segments S2-S4 and runs beneath the levator ani muscle. In some embodiments, the cutaneous nerves to the penis and scrotum arise from the dorsal and posterior branch of the PN. In some embodiments, the PN supplies the ischiocavernosus and bulbocavernosus muscles. In some embodiments, the penile or clitoral afferent nerves interact with the autonomic centers located in the sacral intermediolateral cell columns to modulate reflexogenic erection and micturition. In some embodiments, the efferent motor neurons travel via the pudendal branches that innervate striated perineal muscles (e.g., pelvic floor muscle, ischiocavernosus muscle, bulbocavernosus muscle). In some embodiments, the major nerve supply to the levator ani muscle includes the levator ani nerve (from S4) and contributions from the pudendal nerve. As shown in FIG. 4A, Onuf's nucleus 138 (S2-S4) is the center of pelvic somatomotor integration which joins nerves and becomes the pudendal nerve.

In some embodiments, the cavernosal nerve travels along the posterolateral aspect of the prostate and starts converging in the prostatic apex when approaching the membranous urethra at the 3 and 9 clock positions, before crossing the pelvic floor muscle near the urethra. In some embodiments, the fibers merge and form the cavernosal nerve, which traverses within the penile structures. In some embodiments, the electrodes are placed in the pelvic floor area, in close proximity to the urethra and due to the cavernosal nerve path. In some embodiments, electrical stimulation by the electrodes in the pelvic floor area along the cavernosal nerve, a somatic nerve, may result in reduced side effect. In some embodiments, the reduced side effect may be due to the proportion of somatic nerves surrounding the prostate apex, which represent <5% of the autonomic nerves, comprising both sensory and motor nerves.

In some embodiments, the autonomic nerve involvement in sexual function comprises efferent autonomic fibers to the penis from the cavernous nerve (CN) on each side of the prostate. In some embodiments, the CN leaves the pelvis between the transverse perineal muscles and membranous urethra before passing beneath the pubic arch to supply both corpus cavernosum. In some embodiments, the stimulation of the pelvic plexus and CN induce erection of the erectile tissue (i.e., penis or clitoris), whereas activation of the sympathetic trunk causes detumescence. In some embodiments, the autonomic nerves are a conduit for brain-induced and reflexogenic instructions to reach the erectile tissue (e.g., efferent system).

The pelvic plexus may comprise one or more nerves that that innervate tissue involved with urination and/or defecation in a pelvis of an individual. In some instances, one or more nerves of the pelvic plexus may comprise the splanchnic nerve, hypogastric nerve, autonomic plexus nerve, or any combination thereof nerves. The pelvic plexus may comprise one or more parasympathetic and/or sympathetic nerves.

### Implant Locations

Described herein are devices, systems, and methods for treating a symptom of sexual dysfunction in an individual by providing an adaptive electrical stimulation based on a detected parameter associated with sexual dysfunction in the individual. In some embodiments, treating a symptom of sexual dysfunction comprises preventing and/or reducing the severity of insufficient penile or clitoral erection. Provided herein are devices and systems for treating a symptom of sexual dysfunction in the individual comprising a sensor and an electrode that are configured to be implanted in proximity to a pudendal nerve, a cavernous nerve, a splanchnic nerve, a sacral nerve, a pelvic plexus nerve, or any branches or combination thereof in the individual. In some aspects, one or more sensors and one or more electrodes can be placed at one or more anatomical sites unilaterally or bilaterally. In some aspects, the anatomical site comprises a post-ganglionic nerve, or a pre-ganglionic nerve, or a combination thereof. In some aspects, an implanted electrode comprises a first stimulator implanted at or adjacent to a first anatomical site which comprises at least one of a pudendal nerve, a cavernous nerve, a splanchnic nerve, and a sacral nerve. In some aspects, an implanted electrode comprises a second stimulator implanted at or adjacent to the first anatomical site or a second anatomical site which comprises at least one of a pudendal nerve, a cavernous nerve, a splanchnic nerve, a sacral nerve, a pelvic plexus nerve, or any combination thereof. In some embodiments, the one or more stimulator electrodes or one or more sensors may be implanted unilaterally and/or bilaterally based at least on the sexual dysfunction individual has experienced or may experience. In some cases, the one or more stimulator electrodes and/or one or more sensors may be implanted unilaterally at or adjacent a first region of a nerve. In some embodiments, the one or more stimulator electrodes and/or one or more sensors may be implanted bilaterally at or adjacent to a first region nerve and a second region of a nerve where the first and second region are spatially independent regions of the nerve. In some cases, unilateral pain may be treated by implanting one or more electrode leads (e.g., stimulator electrode and sensor electrode) at or adjacent to a trunk and distal pudendal nerve, pudendal and sacral nerve, pudendal and pelvic autonomic nerve, or any combination thereof. In some instances, bilateral pain may be treated by implanting one or more electrode leads (e.g., stimulator electrode and sensor electrode) at or adjacent the pudendal nerve bilaterally, the sacral nerve bilaterally, pelvic autonomic nerve bilaterally. In some aspects, the first and/or second anatomical site comprises an afferent nerve, an efferent motor nerve, or any combination thereof. In some aspects, the first anatomical site and the second anatomical site are on different nerves.

In some cases, the one or more stimulator electrodes may be placed at or near the pudendal nerve or the sacral nerve. In some cases, the stimulator electrode and/or the sensor may be implanted in proximity to the pudendal nerve, sacral nerve, cavernous nerve, splanchnic nerve and/or pelvic plexus nerve unilaterally or bilaterally. In some cases, the stimulator electrode may be implanted at or near the pelvic floor muscle. In some cases, the stimulator electrode may be implanted to stimulate motor nerve fibers and branches thereof (e.g., pudendal nerve, cavernous nerve, splanchnic nerve, sacral nerve, levator ani nerve). Alternatively, or in combination, the stimulator electrode may be implanted within, proximate, or adjacent to the muscle (e.g., pelvic floor muscle, striated perineal muscles, ischiocavernosus muscle, bulbocavernosus muscle, levator ani muscle).

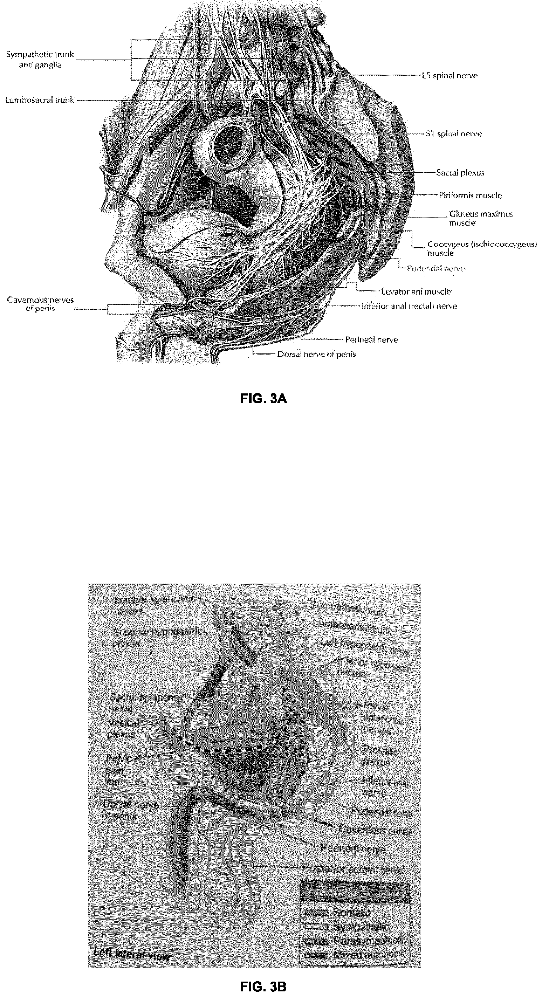

In some instances, the devices, systems, and methods described herein involve providing an electrical stimulation to one or more reflex loops including the pudendo-cavernosal reflex loop, which is associated with tumescence of an erectile tissue, and the bulbocavernosus reflex loop, which is associated with rigidity of the erectile tissue (see FIG. 3D). In some instances, the pudendal nerve circuitry is a mixed nerve, composed of both somatic (sensory and motor) and autonomic components. In some instances, the nerves are nitric oxide synthase (NOS) positive. In some instances, the NOS activity is considered autonomic in origin and a critical component of sexual and eliminative reflexology.

In some instances, an electrical stimulation is configured to target sensory stimulation and motor stimulation. In some instances, the electrical stimulation is configured to target to at least one of the pudendo-cavernosal reflex loop and/or the bulbocavernosus reflex loop (see **FIG. 3C**). In some instances, the electrical stimulation is configured to be provided to at least one of pudendal afferent nerves and efferent motor fibers from a pudendal nerve. In some instances, the electrical stimulation is configured to stimulate a pelvic floor muscle, an ischiocavernosus muscle, a bulbocavernosus muscle, or any combination thereof. In some instances, the adapted stimulation is configured to initiate tumescence of the erectile tissue. In some instances, for sensory stimulation, the electrical stimulation is configured to initiate tumescence of the erectile tissue by stimulating the pudendal afferents and activate of the pudendo-cavernosal reflex loop. In some instances, for motor stimulation, the electrical stimulation is configured to induce and sustain rigidity of an erection by stimulation efferent motor fibers from the pudendal nerve that supply the ischiocavernosus muscle and bulbocavernosus muscle and activate the bulbocavernosus reflex loop.

In some instances, the devices, systems, and methods described herein involve stimulating the pudendo-cavernosal reflex loop. In some instances, penile or clitoral afferents play a direct role in tumescence of the erectile tissue via the pudendo-cavernosal reflex. In some instances, the somatic-autonomic reflex involves the dorsal nerve (DN) of the penis and clitoris (DNP/C) and the perineal nerve as the afferent, and the cavernosal nerve (CN) as the efferent limb. In some instances, genital stimuli are conveyed via the DNP/C and perineal nerve to the dorsal horn of the lumbosacral gray commissure. In some instances, they stimulate autonomic centers via interneurons. In some instances, the efferent CN sends signals back to the genitalia to initiate penile corporal and clitoral/labial vasodilation. In some instances, the bioelectronic system is configured to stimulate the DNP/C leading to intracavernosal pressure elevation and penile or clitoral erection. In some instances, the bioelectronic system is configured to modulate the parasympathetic output of the pudendo-cavernosal reflex loop with bilateral stimulation of the CN.

In some instances, the devices, systems, and methods described herein involve stimulating the bulbocavernosus reflex loop. In some instances, the bulbocavernosus reflex loop is a spinal reflex involving somatic afferent and somatic efferent neurons of the PN. In some instances, the afferent limb comprises DNP/C and perineal nerve, which join fibers to relay their message to the Onufs nucleus. In some instances, the efferent motor neurons travel via the PN branches that supply the ischiocavernosus muscle (ICM) and the bulbocavernosus muscle (BCM). In some instances, a contraction of the ICM produces the rigid-erection phase. In some instances, a rhythmic contraction of the BCM aids in ejaculation. In some instances, the bioelectronic system is configured to modulate the output of the bulbocavernosus reflex loop.

In some instances, the devices and systems described herein increase the intracorporal pressure (ICP) of the erectile tissue by stimulating one or more nerves of the individual. In some instances, the one or more nerves comprises the pudendal nerve, cavernosal nerve, splanchnic nerve, sacral nerve, or any combinations or branches thereof. In some instances, the stimulator described herein, provides an electrical stimulation at the nerve, via a stimulator electrode at a frequency and/or frequency ranges, described herein. In some instances, the electrical stimulation increases intracorporal pressure of the erectile tissue. In some instances, the intracorporal pressure ranges from about 1 mm Hg to about 200 mm Hg, about 5 mm Hg to about 100 mm Hg, or about 5 mm Hg to about 50 mm Hg. In some instances, the intracorporal pressure in a flaccid state of the erectile tissue is close that venous system. In some instances, the ICP of the flaccid state ranges from about 1 mm Hg to about 10 mm Hg, or about 5 mm Hg to about 7 mm Hg. In some instances, the ICP in a latent or filling phase when an erection is initiated is close to venous pressure. In some instances, the partial pressure of oxygen in a latent or filling phase when an erection increases from the flaccid state. In some instances, the ICP increases rapidly during transition from the latent phase to the tumescence phase. In some instances, the ICP reaches an equilibrium level during the tumescence phase that is close to the mean systolic arterial pressure. In some instances, the ICP during the full erection phase is close to that of the systolic pressure. In some instances, the corpoveno-occlusive mechanism is fully activated during the erection phase, .

The devices and systems described herein comprise one or more sensors or sensor electrodes, one or more stimulator electrodes, a processor, a power source, or any combination thereof. In some instances, the one or more stimulator electrodes, one or more sensors, a processor, a power source, or any combination thereof are implanted into the body of the individual. In some instances, the one or more stimulator electrodes, one or more sensors, processor, power source, or any combination thereof may be placed superficially on the body of the individual. In some instances, the processor, power source, or any combination thereof are implanted into the body of the individual. In some instances, the device comprises a wireless transmission module capable of transmitting and receiving wireless data wireless by and between a remote device (e.g., a mobile phone, a tablet, a computer, etc.) and the device described herein. Alternatively or in combination, the device comprises a hermetically sealed connector placed on the individual's skin superficially that may electrically couple to a remote device by a cable. In some instances, the individual or a healthcare professional may manually modify or change the one or more sensors or one or more stimulator electrode parameters through a graphical user interface on the remote device. In some embodiments, the device automatically modifies the setting of the sensor and/or the stimulator electrode. In some embodiments, the device may automatically modify the setting of the sensor and/or the stimulator electrode based on a parameter detected by the sensor. In some embodiments, the setting may comprise one or more of sensitivity of the sensor or stimulator, activity of the sensor or stimulator, a length signal acquisition period, a stimulation pattern provided by a stimulator to the surrounding tissue.

### Adapted Stimulation

Described herein are devices, systems, and methods for treating a symptom of sexual dysfunction in an individual by providing an adapted stimulation to treat the episode of sexual dysfunction. In some instances, the sexual dysfunction comprises a dysfunction in an erection of an erectile tissue, such as a penis or a clitoris. In some instances, the dysfunction in erection comprises a dysfunction of at least one of initiation, induction, or sustentation of the erection of the erectile tissue. In some instances, the sexual dysfunction comprises a dysfunction in achieving one or more of arousal, ejaculation, in achieving orgasm, and vaginal dryness. In some instances, the dysfunction in achieving arousal comprises a dysfunction in dopamine release in striatum and/or a dysfunction in nitric oxide (NO) release in the erectile tissue. In some instances, the dysfunction of the erection is measured by tumescence of the erectile tissue or by rigidity of the erectile tissue. In some instances, the tumescence is measured by intracorporeal pressure of the erectile tissue. In some instances, a parameter to indicate a change in length and rigidity of the erectile tissue can be used. In some cases, erection is measured with color duplex doppler ultrasonography to measure blood inflow into the penis.

In some instances, the adapted stimulation, alone or together with a response of the individual, promotes an increase in an intracorporal pressure, also referred herein as intracorporeal pressure, of the erectile tissue. In some instances, the adapted stimulation is configured to increase dopamine release in striatum. In some instances, the adapted stimulation is configured to increase nitric oxide (NO) release in the erectile tissue. In some instances, the adapted stimulation is configured to augment a reflex activity of the individual experiencing an episode of sexual dysfunction. In some instances, the adapted stimulation is configured to initiate tumescence. In some instances, the adapted stimulation is configured to initiate, induce, or sustain an increase in an intracorporal pressure of the erectile tissue (e.g., penis or clitoris) or a combination thereof. In some instances, the adapted stimulation is configured to increase vaginal lubrication. In some instances, the adapted stimulation is configured to target both the somatic and autonomic system. In an example, the somatic system is targeted through the pudendal nerve and the autonomic system is targeted through the cavernous nerve. In some instances, the adapted stimulation is configured to initiate, induce, or sustain a penile or clitoral erection, or a combination thereof.

In some cases, the implanted sensor of the device or the system provided herein detects a signal that indicates that an individual may exhibit an episode of sexual dysfunction, such as an insufficient erection of the erectile tissue. In some cases, the implanted sensor detects a parameter associated with an episode of sexual dysfunction. In some cases, the implanted sensor detects a parameter associated with an intent for sexual activity. In some cases, the intent for sexual activity is insufficient for normal sexual function. In some cases, the determination by the device or the system of the episode of insufficient erection comprises one or more signals from one or more sensors, where the sensors may be at one or more anatomical sites. In some cases, the device may analyze the signal and classify the signal as a real-time or prospective episode of insufficient erection. In some cases, the device may generate an electrical stimulation that is modulated based on the classified episode of insufficient erection and may deliver a modulated electrical stimulation using one or more stimulator electrodes to the targeted nerve(s). In some cases, the modulation of the one or more electrical stimulation comprises changing the frequency of electric stimulation, amplitude of electrical stimulation, pulse width of electric stimulation, stimulator electrode configuration, or any combination thereof. In some cases, the electrode comprises one or more stimulation electrodes. In some cases, the electrode comprises one or more stimulation electrodes and one or more sensors. In some cases, the sensor comprises a tumescence sensor on or near the penis configured to sense an erectile function (also referred herein as an erection). In some cases, the tumescence sensor comprises a pressure sensor, a temperature sensor, a blood flow sensor, a stretch sensor, or a combination thereof. In some cases, the tumescence sensor detects one or more parameters associated with a change in penile length, girth, and/or rigidity. In some cases, a cuff can be configured to be placed around the penis and to sense a change in erection and/or a pressure. In some cases, the cuff comprises a pressure sensor configured to detect a change in the intracorporal pressure of the erectile tissue. In some cases, a patch with at least one or two dimensional stretches can be configured to detect a change in the intracorporal pressure and communicate it to an implantable pulse generator IPG or a patient controller module in communication with the IPG. In some cases, a condom with a stretch or strain sensor can be used to determine girth and length (not shown). In some cases, the stretches that are measured by the sensor serve as a surrogate for directly measuring intracorporal pressure. In some cases, the external sensor can communicate with the IPG or patient control module. In some cases, a condom or wearable cuff can activate the IPG to start stimulation. In some cases, the IPG or sensor can detect vibrations from a vibrator to start stimulation. In some cases, the embedded or external sensors can be used to calibrate parameters used for identifying the electrical stimulation which can be programed in the IPG.

In some instances, the sensor is configured to monitor internal environment, including but not limited to muscle functions. In some instances, the muscle function comprises a pelvic floor EMG in the different erectile states and patterns to recognize orgasm (i.e. characterized by repeated contractions of the pelvic floor). In some instances, the sensor can be combined with the individual's actuation to signal termination of stimulation at the time sexual act is finished. In some instances, the sensor comprises a sensor electrode, a receiver, a pressure sensor, an accelerometer, a gyroscope, a magnetometer, a global positioning system (GPS), or any combination thereof. In some instances, the sensor is configured to detect an activity of a muscle of the individual from an EMG signal, an ENG signal, a digital signal, an individual-initiated signal, a pressure signal, a muscle contraction event signal, or any combination thereof.

In some instances, the stimulation electrode and the sensor may be configured to switch between stimulating and sensing operations on demand, programmatically, user controlled, medical personal control, or any combination thereof. In some instances, the electrical stimulation may be modulated to improve the muscle and/or nerve response to prevent the episode of insufficient erection. In some instances, the modulated electrical stimulation results in improved muscle response, as measured by response time, muscle function, or other markers of insufficient penile or clitoral erection prevention, to prevent the potential episode of insufficient penile or clitoral erection. In some instances, the electrical stimulation may be modulated to reduce a severity and/or duration of an episode of insufficient erection. In some instances, an individual who exhibits an insufficient erection episode may receive an electrical stimulation provided by the devices, systems, and methods provided herein, in response to the insufficient erection episode.

In some embodiments, the electrode provides a basal stimulation to the target nerve before an adapted stimulation (e.g., an electrical stimulation having an adapted stimulation pattern) is provided. In some embodiments, the adapted electrical stimulation is provided by the same electrode that provided the basal stimulation. In some embodiments, the adapted electrical stimulation is provided by a different electrode than the one providing the basal stimulation. In some embodiments, the adapted stimulation provide at least one of: (i) a "boost" of the basal stimulation parameters (e.g., increase the amplitude of the basal stimulation); (ii) a switch to a different stimulation program; or (iii) a change from single to dual nerve stimulation, or a combination thereof.

In some cases, the electrical stimulation comprises a constant stimulation, a variable stimulation, a combination of a constant and a variable stimulation, or a combination of variable stimulations, that are provided and vary over a period of time. In some cases, the electrical stimulation increases automatically in amplitude during the sequence of erectile phases up to orgasm/ejaculation. In some cases, the period of time may comprise the period of time a subject may provide an intent based or purposeful muscle contraction to trigger an EMG threshold detection. In some cases, the period of time for the electrical stimulation may comprise about 1 second to about 30 seconds. In some cases, the adapted stimulation results in extended duration of the sexual activity than without the adapted stimulation. In some cases, the adapted stimulation ends before the end of sexual activity. In some cases, where stimulation ends before the end of sexual activity, the sexual dysfunction may be less severe, including but not limited to less disturbed erectile dysfunction or problem with vaginal dryness.

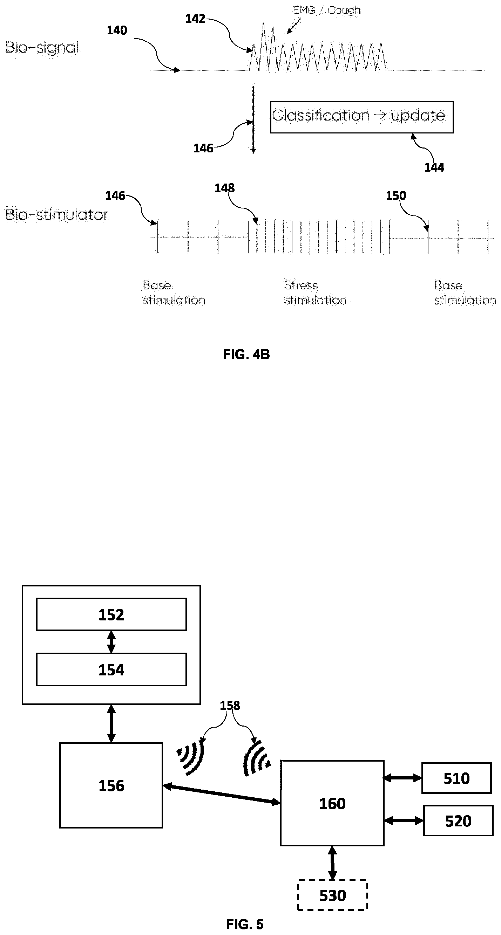

In some instances, the electrical stimulation is adapted to an input (e.g., signal indicating a symptom of sexual dysfunction) obtained from the individual. In some instances, the inputs are used to determine the adapted stimulation for starting a sexual activity, changing a stimulation paradigm during the sexual activity, and terminating the sexual activity. In some instances, inputs from the individual can be used to initiate excitement phase and to start the sexual activity when the individual does not have a change in arousal state and/or when the individual has a partial erection (e.g., tumescence without rigidity). In some instances, the adapted stimulation may vary over time, including but are limited to in the case of single nerve stimulation, going from single nerve to two nerve stimulation and in the case of combined stimulation, changing the parameters of one or both stimulation locations, changing the predominance in stimulation, and ending the stimulation maintaining the erection or reversing the intracorporal pressure. Examples of inputs from the individual include but are not limited to a sequence of taps on the IPG or a sensor, a remote or manual input on the patient control module, by sensing a sequence of voluntary contractions of the pelvic floor (i.e. via EMG), and by way of holding, waving, tapping, or using opposite poles of a magnet relative to a sensor or the IPG (see **FIG. 5**). Alternatively or in combination, event patterns can be automatically sensed and considered as inputs by sensing specific pattern of pelvic floor EMG patterns such as rhythmic pelvic floor contractions that accompany ejaculation and orgasms.

In some instances, the adapted stimulation comprises stimulation of a first nerve at the onset of sexual activity, which is followed by a patient actuation to induce a second nerve at a certain point of arousal. In some instances, the adapted stimulation comprises adding and/or changing the nerve that is stimulated by the methods, devices, and systems provided herein. In some instances, the adapted stimulation comprises a change in stimulation parameters.

### Parameters

In some cases, an implanted sensor of the device may be configured to sense one or more parameters, also referred herein as bio-signals, that may be associated with a symptom of sexual dysfunction. In some cases, the sexual dysfunction comprises a dysfunction of penile or clitoral erection. In some cases, a parameter is associated with the individual getting aroused, having sexual intercourse, or any combination thereof. In some cases, the parameter is associated with at least one of initiation, induction, or sustentation of the episode of sexual dysfunction. In some cases, the parameter comprises an electromyography (EMG) signal that may be predictive of an episode of penile or clitoral erection. In some cases, EMG signals comprise electrical activity of muscles, specifically from action potentials in muscle fibers. In some cases, the parameter comprises an electroneurogram (ENG). In some cases, an electroneurogram comprises electrical activity from one or more neurons and typically refers to recordings made from bundles of axons in peripheral nerves. In some cases, the parameter comprises a change of electric impedance caused by physical deformation of the sensor material. In some cases, the physical deformation comprises stretching, elongation, compression, or any combination thereof.

In some instances, the parameter comprises a change in pressure, velocity, acceleration, or 3-D spatial direction. In some instances, 3-D spatial direction may be determined by GPS signal. The GPS signal, in some cases, may indicate and recognize when the individual is in proximity to locations such as the individual's home. In some instances, the GPS signal may be configured to modulate the stimulator, described elsewhere herein, based on the GPS coordinates and/or GPS location of the subject. In some instances, modulating the stimulator comprises adjusting a detection parameter (e.g., signal intensity threshold) of the classifier, described elsewhere herein. In some cases, the GPS signal of a location and/or GPS coordinates may indicate locations and/or regions traveled to the subject that may impose a higher risk of a penile or clitoral erection event. In some cases, 3-D spatial direction may measure the posture of an individual. In some instances, changes in pressure may be measured by a pressure sensor. In some cases, the pressure sensor comprises a differential pressure sensor, absolute pressure sensor, or any combination thereof. In some cases, changes in velocity, acceleration, or changes in 3-D spatial direction may be measured by an accelerometer, gyroscope, magnetometer, or any combination thereof. In some instances, the device may provide an electrical stimulation using the one or more implanted stimulator electrodes that, together with the individual's preventative response, may prevent the episode of insufficient penile or clitoral erection.

In some cases, a parameter is based on activity detected during REM sleep as a new biomarker alongside tumescence measurements to help configure the adapted stimulation. In some cases, the parameter is based on nerve activity detected during REM sleep. In some cases, the parameter detected during REM sleep may serve as an input to determine whether an adapted stimulation is needed and what type of adapted stimulation may be needed to treat the symptom of sexual dysfunction in the individual. In some cases, the parameter detected during REM sleep may be used in providing the adapted stimulation to treat the individual when they are awake.

The devices, systems, and methods described herein may be capable of treating a symptom of sexual dysfunction. The devices, systems, and methods described herein may be capable of preventing and/or reducing the severity of an insufficient erection event by detecting an individual's erection parameter and adjusting an electrical stimulation by one or more stimulator electrodes based on the erection's characteristics. In some cases, the erection parameter characteristics comprise the changes in the amplitude, frequency, phase, or any combination thereof one or more EMG, ENG, accelerometer, gyroscope, magnetometer, pressure sensor signals, or any combination thereof. In some instances, the erection parameter may comprise physical movement of the individual, one or more EMG signals, or any combination thereof. By providing adaptive electrical stimulation in combination to the individual's response and/or basal stimulation, the devices, systems, and methods described herein may treat the symptom of sexual dysfunction.

### Device

Provided herein are devices and systems for treating a symptom of sexual dysfunction in an individual. The devices and systems provided herein comprise one or more sensors and one or more stimulator electrodes. The devices and systems provided herein further comprise a processor and a non-transitory computer readable storage medium. In some cases, the processor comprises a processor, memory, a user interface, a power source, or any combination thereof. The devices may be implantable. The surgical procedure to implant the device may be completed under awake sedation, general anesthesia, local anesthesia, twilight anesthesia, or any combination thereof. The devices may be implanted wholly or partly in an individual's pelvic region. In some cases, the devices may be implanted by one or more surgical instruments. In some instances, surgical instruments may comprise introducers, sheaths, directable probes, wires, needles, or any combination thereof. In some cases, the device may further comprise a transmitter electrically coupled to a processor capable of wirelessly transmitting and receiving data from a remote device, such as a mobile phone, a tablet, or a computer. In some cases, the device may be configured for open-loop configuration. In some cases, the device may be configured for a close-loop or feedback-controlled configuration. The devices described herein may be used to prevent an episode of insufficient penile or clitoral erection.

### Open Loop Configuration

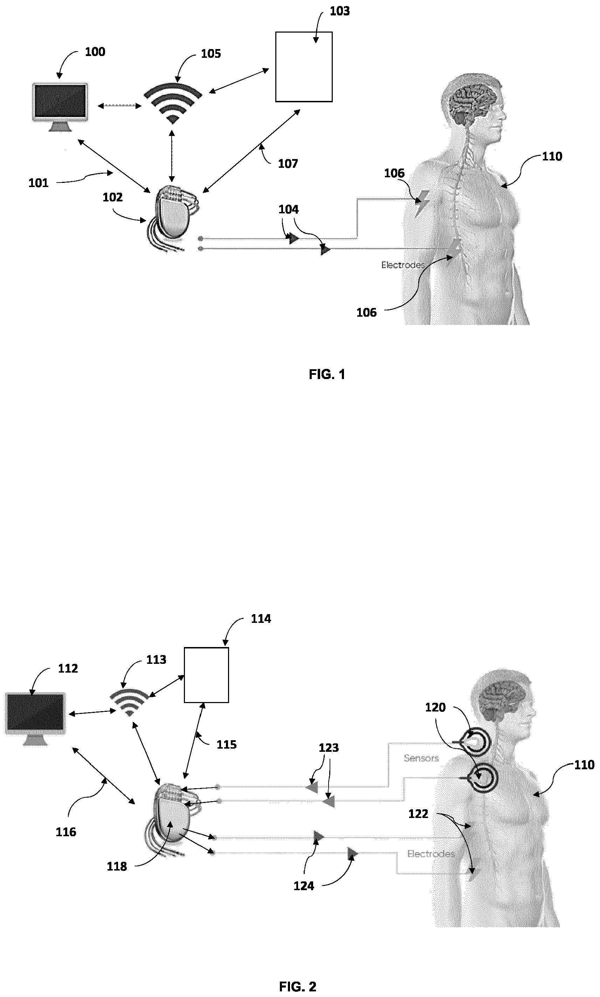

**FIG. 1** shows an exemplary embodiment of an open-loop bioelectronic system described herein configured to treat a symptom of sexual dysfunction in a pelvic region of an individual **110.** The system in an open loop configuration may comprise an implantable pulse generator IPG **102** comprising one or more stimulator electrodes **106,** and a power source. In some cases, the implantable pulse generator **102** may comprise a processor, and a wireless transmission module configured to execute software to administer a stimulation pattern **104.** In some cases, the stimulation pattern is an electrical stimulation pattern. In some cases, the power source may comprise a battery. In some instances, the battery may be a lithium polymer ion battery, lithium iodine, lithium manganese dioxide, lithium carbon monofluoride, or any combination thereof. In some cases, the battery may be wirelessly charged by an inductive charger. In some cases, the battery power source may be a single use.

The implantable pulse generator (IPG) **102** may deliver a predefined stimulation pattern **104** that has been set by a healthcare provider on a remote device **100** (e.g., a mobile phone, a tablet, a computer, etc.) and transmitted wirelessly **105** to the implantable pulse generator **102.** Alternatively or in combination, the implantable pulse generator **102** may deliver a predefined stimulation pattern **104** that has been set by a healthcare provider on a remote computing device **100** (e.g., a mobile phone, a tablet, a computer, etc.) and transmitted via a wired communication **101** to the implantable pulse generator **102.** In some instances, the healthcare provider may set the predefined electrical stimulation parameters through a graphical user interface on the remote device. In some cases, electrical stimulation parameters of a pulse generator **102** implanted in a pelvic region of the individual **110** may be modified or set by the individual with an external input device **103** (e.g., a mobile phone, a tablet, a computer, etc.) via a wireless communication **105** between the external input device **103** the implantable pulse generator **102.** In some cases, an individual **110** with an implantable pulse generator **102** may modify or set electrical stimulation parameters via an external input device **103** via a wired communication **107** of the implantable pulse generator **102.** In some instances, the stimulation pattern **104** of a pulse generator **102** implanted in a pelvic region of the individual 110, may be adjusted by the individual using a graphical user interface on the external input device **103.** In some cases, the stimulation pattern **104** parameters that may be adjusted comprise frequency, amplitude, pulse width, or any combination thereof.

In an aspect, the system is configured to deliver a predefined stimulation protocol and does not receive input from the subject.

### Closed Loop Configuration

**FIG. 2** shows an exemplary embodiment of a closed-loop bioelectronic system described herein configured to symptom of sexual dysfunction in a pelvic region of the individual **110.** The system comprises a two-way neural interface comprising a sensor configured to capture a neural response of the subject and a processing module configured to interpret the neural response. In an aspect, the system is configured to deliver an adapted stimulation based on the neural response. The system in a closed loop configuration may comprise an implantable pulse generator **118,** one or more stimulator electrodes **122,** one or more sensors **120,** and a power source. In an aspect, sensors are configured to capture one or more bio-signals **123** from a pelvic region of the individual **110.** In an aspect, the one or more bio-signals **123** can be used to identify and/or classify a parameter associated with a sexual dysfunction episode. Examples of parameters associated with a sexual dysfunction episode comprise an electrical parameter, a pressure parameter, a motion parameter, or any combination thereof. In some cases, the parameter associated with the sexual dysfunction episode comprises a physiological signal, EMG signal, ENG signal, digital signal, an individual-actuated signal based on a volitional signal or wanting the increase in intracorporal pressure, a pressure signal, muscle contraction event signal, a motion signal, an orientation signal, a posture signal, a global positioning system (GPS) signal, a speed signal, a location signal, a time of day signal, a time interval signal, or any combination thereof. In some cases, the parameter has diurnal variation.

In some cases, the parameter comprises a first parameter and a second parameter, where the second parameter is later in time than the first parameter. In an example, the adapted stimulation is sustained, modified, or cancelled based on the second parameter. In an example, the second parameter comprises an ending parameter, and the adapted stimulation can be reduced or ended after detecting the ending parameter. In some cases, the ending parameter comprises an individual-actuated signal, an EMG signal of the pelvic floor associated with ejaculation, an ENG signal associated with ejaculation, a pressure signal associated with ejaculation, or any combination thereof.

In some cases, the implantable pulse generator **118** may comprise a processor, and a wireless transmission module configured to execute software to detect, analyze myoelectric electromyograph (EMG) signals via one or more sensors **120** and administer an electrical stimulation pattern **124** or ("bio-simulation"). In some instances, the power source may comprise a battery. The battery may be rechargeable or single use. In some cases, the battery may be charged through inductive charging. In some instances, the system configured in a closed loop configuration may measure EMG signals via one or more sensors **120** to detect the onset of a sexual dysfunction event or a level of innate myoelectric electrical activity. In some instances, the system configured in a closed loop configuration may measure inertial signals such as rapid acceleration, shock, posture-orientation, or any combination thereof via the one or more sensors **120** to detect the onset of the sexual dysfunction event or a level of innate myoelectric electrical activity Once detected the implantable pulse generator **118** may provide an electrical stimulation pattern **124** to treat the sexual dysfunction event. In some cases, the threshold level for detecting the sexual dysfunction event may be modified and adjusted by the individual **110** via graphical user interface on an external input device **114** either via wireless communication **113** or a wired connection **115.**

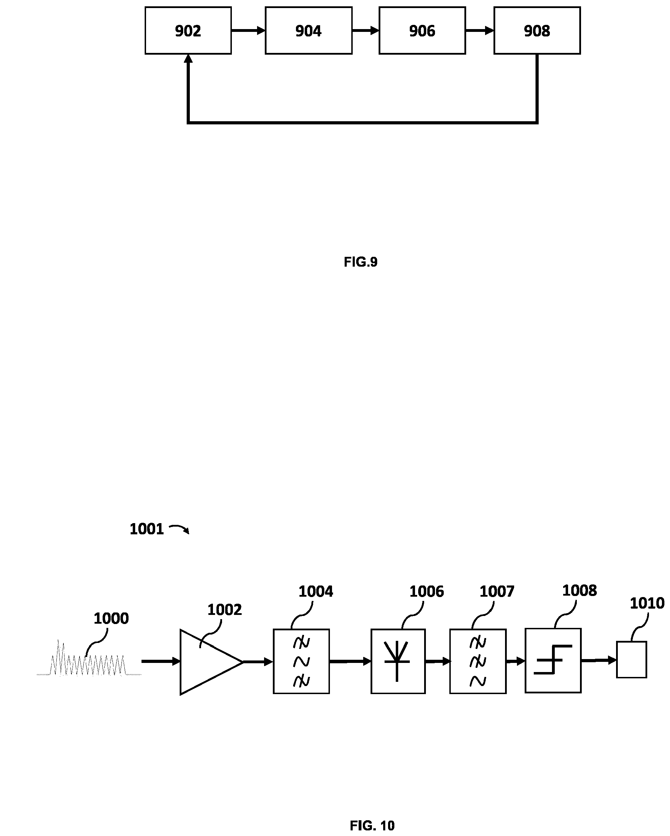

In some embodiments, a closed-loop configuration of the systems and methods described herein, may comprise a fully synchronized system, as shown in **FIG. 9****.** In some cases, an electrode **902,** comprising a sensor and/or stimulator electrode may detect an EMG, ENG, and/or pressure signal through circuitry **904** (e.g., analog to digital circuitry) and then may pass the detected signal to a classifier algorithm **906.** Once the classifier algorithm **906** has classified the detected EMG, ENG, and/or pressure signal as a sexual dysfunction event, the classifier may enable an electric stimulation **908** to be delivered to the patient. As shown in **FIG. 9****,** the electrical stimulation may be provided by the same electrode **902** that sensed the EMG and/or ENG signal initially. In some cases, the electrical stimulation may be provided by one or more different electrodes.

In some embodiments, a closed loop configuration of the system described herein may be configured to detect an individual's effort to counteract a symptom of sexual dysfunction. In some instances, the methods and systems described here may supplement the patient's effort with an electrical stimulation pattern via one or more stimulator electrodes **122** sufficient to prevent an episode of sexual dysfunction. In some cases, an individual's effort may comprise an EMG, ENG, pressure, acceleration, gyroscope, magnetometer, 3-D spatial, or any combination thereof signal at a threshold. In some instances, the threshold myoelectric signal may be detected by one or more sensors **120.** In some cases, the supplemental excitation, may comprise an excitation signal provided by the stimulator electrodes, described elsewhere herein, with parameters e.g., frequency, pulse width, and/or amplitude such that in combination with the detected individual's effort may prevent an episode of sexual dysfunction. In some instances, the supplemental excitation may comprise an excitation signal provided by the stimulator electrodes with parameters e.g., frequency, pulse width, and/or amplitude to prevent a sexual dysfunction episode in response to detecting the sexual dysfunction event. In some instances, the insufficient penile or clitoral erection event may comprise actions such as coughing, sneezing, laughing, or exercise, detectable by e.g., a gyroscope, accelerometer, and/or magnetometer, described elsewhere herein. In some instances, the one or more parameters of the supplemental excitation, may be determined on an individual subject basis and/or on a large-scale population of subjects with similar presentation of sexual dysfunction. For example, a given subject's supplemental excitation one or more parameters may be tuned and/or determined by whether such supplemental excitation signal prevented an episode of sexual dysfunction in real-time or after the sexual dysfunction event through a user interface of the device and systems, described elsewhere herein. In some instances, a given subject's supplemental excitation one or more parameters may be tuned to values and/or parameters found to prevent sexual dysfunction events in subjects with similar clinical presentation (e.g., age, type of sexual dysfunction, frequency of sexual dysfunction events, other subject clinical meta data, etc.).

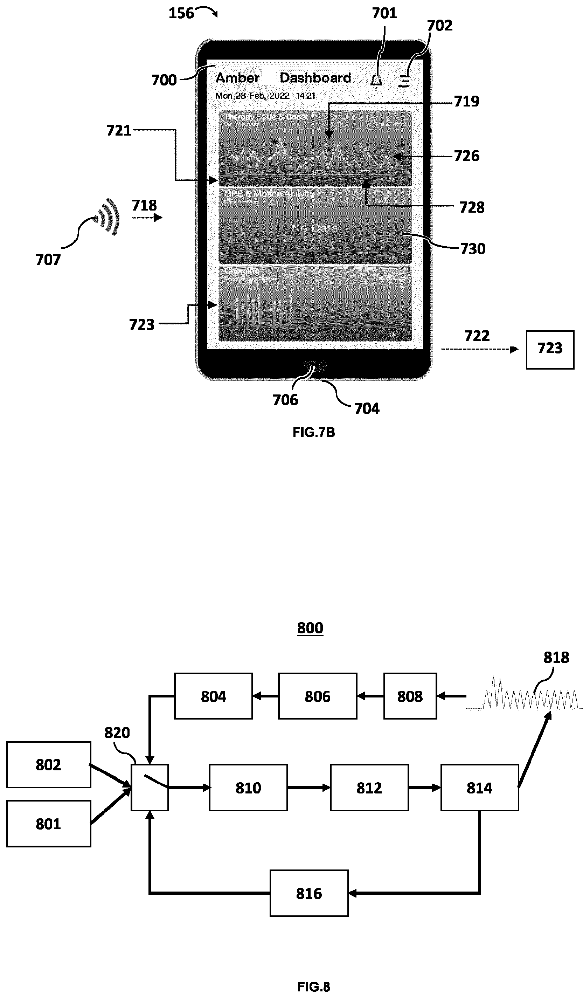

In some instances, the system and methods described herein may comprise system and methods **800** configured to provide an electrical stimulation to prevent a sexual dysfunction event based on a subject and/or patient's purposeful muscle contraction and/or movement, as seen in **FIG. 8****.** In some cases, the subject and/or patient **814** may, upon realizing that they may exhibit a sexual dysfunction event, induce movement and/or contraction of one or more muscles or muscle groups to trigger an EMG, ENG, pressure, acceleration, gyroscope, magnetometer, 3-D spatial, or any combination thereof signal **818.** In some instances, the induced movement and/or contraction of one or more muscle groups may be amplified **808,** classified (by a classifier) **806,** passed through a control logic algorithm **804,** and used as a trigger **820** to enable the flow of therapy and respective basal **802** and/or active **801,** described elsewhere herein, stimulation pattern parameters through the stimulator **810** and neural interface **812** to prevent a sexual dysfunction event. In some cases, the classifier may comprise a machine learning classifier. In some instances, the classifier may comprise an intensity threshold classifier, described elsewhere herein. In some cases, the patient and/or subject **814** may manually **816** enable the delivery of electrical stimulation via a button on the patient controller module **156,** described elsewhere herein.

In some cases, the detection threshold of the implantable pulse generator **118** or stimulator may be modified and tuned via a graphical user interface on an external input device **114.** The external input device **114** may be able to modify and tune the threshold of the implantable pulse generator **118** via a wireless communication **113** or a wired connection **115.** In some cases, the implantable pulse generator threshold may be tuned via a healthcare provider on a remote computing device **112** (e.g., a mobile phone, a tablet, a computer, etc.) and transmitted via a wired communication **116** to the implantable pulse generator **118.** In some cases, the implantable pulse generator threshold may be tuned via a healthcare provider on a remote computing device **112** (e.g., a mobile phone, a tablet, a computer, etc.) and transmitted via wireless communication **113** to the implantable pulse generator **118.** In some cases, the implantable pulse generator threshold may be tuned via the individual **110** on a remote computing device **112** (e.g., a mobile phone, a tablet, a computer, etc.) and transmitted via a wired communication **116** to the implantable pulse generator **118.** Alternatively or in combination, the implantable pulse generator threshold may be tuned via the individual **110** on a remote computing device **112** (e.g., a mobile phone, a tablet, a computer, etc.) and transmitted via wireless communication **113** to the implantable pulse generator **118.**

In some cases, the electrical stimulation pattern **124** provided via one or more stimulator electrodes **122** may be tuned and adjusted by the detected threshold level to supplement an individual's effort to prevent an episode of sexual dysfunction. In some instances, the adjusted electrical stimulation pattern **124** may be determined by mapping a detectable physiological signal representing effort and a provided electrical stimulation pattern **124** by, piecewise linear mapping, linear mapping, sigmoidal mapping, or any variations thereof. The electric stimulation pattern **124** may be tuned by modifying or changing the electrical stimulation pattern parameters comprising frequency, pulse-width, and amplitude. In some cases, the electrical stimulation pattern parameters of the implantable pulse generator **118** may be modified and tuned via a graphical user interface on an external input device **114.** The external input device **114** may be able to modify and tune the electrical stimulation pattern parameters of implantable pulse generator **118** via a wireless communication **113** or a wired connection **115.** In some cases, the electrical stimulation pattern parameters of implantable pulse generator **118** may be tuned via a healthcare provider on a remote computing device **112** (e.g., a mobile phone, a tablet, a computer, etc.) and transmitted via a wired communication **116** to the implantable pulse generator **118.** In some cases, the electrical stimulation pattern parameters of implantable pulse generator **118** may be tuned via a healthcare provider on a remote computing device **112** (e.g., a mobile phone, a tablet, a computer, etc.) and transmitted via wireless communication **113** to the implantable pulse generator **118.** In some cases, the electrical stimulation pattern parameters of implantable pulse generator **118** may be tuned by a machine learning model executed by the processor of the implantable pulse generator **118** based on input from the individual **110.** In some cases, the machine learning model may be configured to determine a whether or not a subject is at risk of an episode of sexual dysfunction based on muscle EMG signals detected by the one or more sensors. In some cases, the machine learning model may be trained to determine the presence or lack thereof an individual's effort, described elsewhere herein. For example, the machine learning model may be trained with one or more EMG signals characteristic of a subject's muscle contractions in particular EMG signals that lead to an episode of sexual dysfunction. In some cases, the information from individuals with normal sexual function may be used by the machine learning model to classify an episode of sexual dysfunction in an individual having symptoms of sexual dysfunction. In some cases, the information from individuals with normal sexual dysfunction may be used by the machine learning model to generate the adapted stimulation pattern to treat an individual having symptoms of sexual dysfunction. In some cases, the individual with normal sexual function may have another condition where the individual uses, devices and systems provided herein. In some cases, another condition comprises incontinence. In some cases, the incontinence comprises urinary incontinence.

In some embodiments, the electrical stimulation pattern parameters, as described elsewhere herein (e.g., frequency, amplitude, etc.), may be set or determined by a stimulation machine learning model. In some cases, the stimulation machine learning model may comprise a Bayesian optimization model. In some instances, the stimulation machine learning model may be trained with stimulation patterns that users of the devices and systems, described elsewhere herein, indicate as treating sexual dysfunction. In some cases, the stimulation machine learning model may be trained with the patient's type of sexual dysfunction. The stimulation machine learning algorithms may be trained in a cloud computing network and/or server in communication with the implantable and user-devices, described elsewhere herein, and re-distributed or downloaded to one or more users and/or patients. In effect users and/or patients may update and/or download new updates to the software of the devices and systems described herein. This aspect of the invention described herein provides an unexpected result of a patient specific and optimized electrical stimulation signal that would otherwise not be achievable with a traditional stimulator.

One or more machine learning algorithms may be used to construct the machine learning model, such as support vector machines that deploy stepwise backwards parameter selection and/or graphical models, both of which may have advantages of inferring interactions between parameters. For example, machine learning algorithms or other statistical algorithms may be used such as alternating decision trees (ADTree), decision stumps, functional trees (FT), logistic model trees (LMT), logistic regression, random forests (rf), receiver operational characteristic curves (ROC), linear regression, extreme gradient boosting (xgb), classification and regression trees, support vector machines (SVM), generalized additive model using splines (e.g., gamSpline), glmnet, multivariate adaptive regression splint (earth), neural network, k-means clustering, or any machine learning algorithm or statistical algorithm known in the art. One or more algorithms may be used together to generate an ensemble method, wherein the ensemble method may be optimized using a machine learning ensemble meta-algorithm such as boosting (e.g., AdaBoost, LPBoost, TotalBoost, Brown Boost, MadaBoost, LogitBoost, etc.) to reduce bias and/or variance.

In some embodiments, the machine learning algorithm may comprise a constrained machine learning algorithm configured to run on micro-processors. In some cases, the machine learning algorithm may comprise a machine learning algorithm operating on within a TinyML framework. In some instances, the machine learning algorithm, described elsewhere herein may be trained offline. The offline training may be completed on a server, cloud, or other dedicated computing clusters. In some cases, the offline trained machine learning algorithm could then be downloaded, deployed, and/or imported into the device to iteratively improve upon the device and system performance in preventing sexual dysfunction events.

One of ordinary skill would realize that such an off-line training structure is feasible and realized in related but different implementation. For example, such a machine learning architecture may be utilized in performing "wake up-word" text classification that are commonly seen in smartphone devices (e.g., "hey siri", "okay google", etc.). In some cases, the machine learning algorithms described herein may operate within a framework similar to the "wake up-words" speech machine learning classifier. In some cases, the machine learning algorithms described herein may operate on processing power and memory allocation determined to be sufficient for "wake up" speech machine learning classifiers. In some cases, the machine learning algorithms described herein may herein may operate with a user-initiated input. In some cases, the user-initiated input comprises a sequence of tapping motions on a sensor.

In some cases, the software, described elsewhere herein, may be executed by a processor located on the implanted stimulator. In some cases, the software located on the implanted stimulator may utilize a TinyML constrained machine learning model to accommodate the processing and memory parameters of the implanted stimulator. In some instances, the software may be executed offline on a cloud-based computing and/or dedicated computing cluster(s). In some cases, the offline processing workflow may comprise a high-speed (Bluetooth, Wi-Fi, medical implant communication systems, etc.) data transfer between the implanted stimulator and a local personal computing device (smartphone, tablet, laptop, etc.). The personal processing device may then communicate the implanted stimulator data to a one or more cloud and/or computer clusters that will then send back a resulting output, command, and/or notification to the device. In some cases, the command and/or notification may comprise a warning, alert, initiation of electrical stimulation, or any combination thereof. In some cases, the command may comprise the output of a machine learning classifier configured to determine a threshold intensity of EMG and/or ENG signals indicative of a sexual dysfunction event.

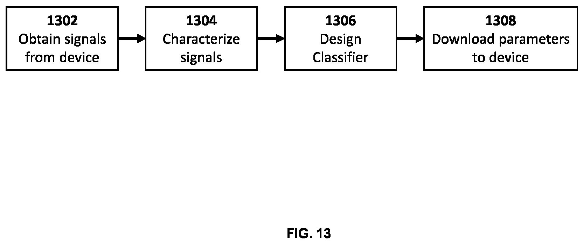

The machine learning models may be trained on one or more datasets. In some instances, the one or more datasets may comprise data generated from a user and/or subject, or data generated from a population or segment thereof. In some cases, the data generated from subject and/or the data generated from a population may comprise effort signals, excitation signals, that indicated a sexual dysfunction event, and prevented the sexual dysfunction event, respectively. In some cases, the devices, systems, and corresponding methods described herein, may record user data and/or input of the user when interacting with the systems and devices described elsewhere herein. In some cases, the data may comprise user labeled EMG, ENG, accelerometer, gyroscope, or any combination thereof sensors, as described elsewhere herein, that lead to a sexual dysfunction event. In some cases, these signals may be obtained from the device **1302,** and used in characterizing **1304** and training a machine learning classifier **1306,** as seen in **FIG. 13****.** In some instances, the trained machine learning classifiers trained on or more datasets may then be downloaded to each patient's device **1308** to further improve the machine learning classifier's accuracy. In some cases, the machine learning models may be configured to sense subject effort and/or providing sufficient excitation based on e.g., parameters of frequency, amplitude, and/or pulse-width as described elsewhere herein. In some instances, the datasets of one or more individuals may be pooled together as a training dataset where the subjects show characteristics of similarity between clinical presentation and parameters of excitatory/sensory input. In some cases, clinical presentation may comprise clinical a sexual dysfunction type, subject clinical meta data, e.g., gender, age, past medical history, current medications taken, past surgical intervention, etc. In some cases, a pooled training datasets may be utilized for an individual during the initial period of training a device implanted into a subject.

In some cases, the one or more machine learning models, described elsewhere herein, may be trained on raw and/or processed signals measured by the devices, sensors, and systems, described elsewhere herein. In some cases, the processed signals may comprise original raw signals that have been filtered to optimize the signal-to-noise ratio of the raw signal. In some cases, the filter may comprise a high-pass, low-pass, band-pass, notch, or any combination thereof filters. In some instances, the one or more machine learning models may alternatively or in addition to, be trained on user feedback regarding prior excitation signal parameters and whether or not such excitation signal parameters prevented a sexual dysfunction event.

In some aspects, the disclosure provides a method of processing detected signals **1001,** described elsewhere herein to determine a sexual dysfunction event precursor signal intensity thresholds, as seen in **FIG. 10****.** In some cases, the EMG, ENG, accelerometer, gyroscope, magnetometer, pressure sensor signals, or any combination thereof signals **1000** may be detected through an amplifier circuit **1002.** In some cases, the amplifier circuit may comprise an operational amplifier circuit.

In some instances, the amplifier circuit may be configured to amplify signals from about 10 microvolt (µV) to about 1,000 µV. In some instances, the amplifier circuit may be configured to amplify signals from about 10 µV to about 50 µV, about 10 µV to about 100 µV, about 10 µV to about 150 µV, about 10 µV to about 300 µV, about 10 µV to about 500 µV, about 10 µV to about 700 µV, about 10 µV to about 900 µV, about 10 µV to about 1,000 µV, about 50 µV to about 100 µV, about 50 µV to about 150 µV, about 50 µV to about 300 µV, about 50 µV to about 500 µV, about 50 µV to about 700 µV, about 50 µV to about 900 µV, about 50 µV to about 1,000 µV, about 100 µV to about 150 µV, about 100 µV to about 300 µV, about 100 µV to about 500 µV, about 100 µV to about 700 µV, about 100 µV to about 900 µV, about 100 µV to about 1,000 µV, about 150 µV to about 300 µV, about 150 µV to about 500 µV, about 150 µV to about 700 µV, about 150 µV to about 900 µV, about 150 µV to about 1,000 µV, about 300 µV to about 500 µV, about 300 µV to about 700 µV, about 300 µV to about 900 µV, about 300 µV to about 1,000 µV, about 500 µV to about 700 µV, about 500 µV to about 900 µV, about 500 µV to about 1,000 µV, about 700 µV to about 900 µV, about 700 µV to about 1,000 µV, or about 900 µV to about 1,000 µV. In some instances, the amplifier circuit may be configured to amplify signals from about 10 µV, about 50 µV, about 100 µV, about 150 µV, about 300 µV, about 500 µV, about 700 µV, about 900 µV, or about 1,000 µV. In some instances, the amplifier circuit may be configured to amplify signals from at least about 10 µV, about 50 µV, about 100 µV, about 150 µV, about 300 µV, about 500 µV, about 700 µV, or about 900 µV. In some instances, the amplifier circuit may be configured to amplify signals from at most about 50 µV, about 100 µV, about 150 µV, about 300 µV, about 500 µV, about 700 µV, about 900 µV, or about 1,000 µV.

In some cases, the amplifier circuit may be configured to amplify signals with a frequency of about 1 Hz to about 1,500 Hz. In some cases, the amplifier circuit may be configured to amplify signals with a frequency of about 1 Hz to about 20 Hz, about 1 Hz to about 40 Hz, about 1 Hz to about 80 Hz, about 1 Hz to about 100 Hz, about 1 Hz to about 150 Hz, about 1 Hz to about 200 Hz, about 1 Hz to about 250 Hz, about 1 Hz to about 500 Hz, about 1 Hz to about 750 Hz, about 1 Hz to about 1,000 Hz, about 1 Hz to about 1,500 Hz, about 20 Hz to about 40 Hz, about 20 Hz to about 80 Hz, about 20 Hz to about 100 Hz, about 20 Hz to about 150 Hz, about 20 Hz to about 200 Hz, about 20 Hz to about 250 Hz, about 20 Hz to about 500 Hz, about 20 Hz to about 750 Hz, about 20 Hz to about 1,000 Hz, about 20 Hz to about 1,500 Hz, about 40 Hz to about 80 Hz, about 40 Hz to about 100 Hz, about 40 Hz to about 150 Hz, about 40 Hz to about 200 Hz, about 40 Hz to about 250 Hz, about 40 Hz to about 500 Hz, about 40 Hz to about 750 Hz, about 40 Hz to about 1,000 Hz, about 40 Hz to about 1,500 Hz, about 80 Hz to about 100 Hz, about 80 Hz to about 150 Hz, about 80 Hz to about 200 Hz, about 80 Hz to about 250 Hz, about 80 Hz to about 500 Hz, about 80 Hz to about 750 Hz, about 80 Hz to about 1,000 Hz, about 80 Hz to about 1,500 Hz, about 100 Hz to about 150 Hz, about 100 Hz to about 200 Hz, about 100 Hz to about 250 Hz, about 100 Hz to about 500 Hz, about 100 Hz to about 750 Hz, about 100 Hz to about 1,000 Hz, about 100 Hz to about 1,500 Hz, about 150 Hz to about 200 Hz, about 150 Hz to about 250 Hz, about 150 Hz to about 500 Hz, about 150 Hz to about 750 Hz, about 150 Hz to about 1,000 Hz, about 150 Hz to about 1,500 Hz, about 200 Hz to about 250 Hz, about 200 Hz to about 500 Hz, about 200 Hz to about 750 Hz, about 200 Hz to about 1,000 Hz, about 200 Hz to about 1,500 Hz, about 250 Hz to about 500 Hz, about 250 Hz to about 750 Hz, about 250 Hz to about 1,000 Hz, about 250 Hz to about 1,500 Hz, about 500 Hz to about 750 Hz, about 500 Hz to about 1,000 Hz, about 500 Hz to about 1,500 Hz, about 750 Hz to about 1,000 Hz, about 750 Hz to about 1,500 Hz, or about 1,000 Hz to about 1,500 Hz. In some cases, the amplifier circuit may be configured to amplify signals with a frequency of about 1 Hz, about 20 Hz, about 40 Hz, about 80 Hz, about 100 Hz, about 150 Hz, about 200 Hz, about 250 Hz, about 500 Hz, about 750 Hz, about 1,000 Hz, or about 1,500 Hz. In some cases, the amplifier circuit may be configured to amplify signals with a frequency of at least about 1 Hz, about 20 Hz, about 40 Hz, about 80 Hz, about 100 Hz, about 150 Hz, about 200 Hz, about 250 Hz, about 500 Hz, about 750 Hz, or about 1,000 Hz. In some cases, the amplifier circuit may be configured to amplify signals with a frequency of at most about 20 Hz, about 40 Hz, about 80 Hz, about 100 Hz, about 150 Hz, about 200 Hz, about 250 Hz, about 500 Hz, about 750 Hz, about 1,000 Hz, or about 1,500 Hz.

In some cases, the amplified signal may then be passed to a filter **1004.** In some cases, the filter may comprise a low-pass, high-pass, band-pass, notch, or any combination thereof filter.

In some cases, the filter may be configured to filter the frequency band of about 1 Hz to about 70 Hz. In some cases, the filter may be configured to filter the frequency band of about 1 Hz to about 5 Hz, about 1 Hz to about 10 Hz, about 1 Hz to about 15 Hz, about 1 Hz to about 20 Hz, about 1 Hz to about 25 Hz, about 1 Hz to about 40 Hz, about 1 Hz to about 50 Hz, about 1 Hz to about 60 Hz, about 1 Hz to about 70 Hz, about 5 Hz to about 10 Hz, about 5 Hz to about 15 Hz, about 5 Hz to about 20 Hz, about 5 Hz to about 25 Hz, about 5 Hz to about 40 Hz, about 5 Hz to about 50 Hz, about 5 Hz to about 60 Hz, about 5 Hz to about 70 Hz, about 10 Hz to about 15 Hz, about 10 Hz to about 20 Hz, about 10 Hz to about 25 Hz, about 10 Hz to about 40 Hz, about 10 Hz to about 50 Hz, about 10 Hz to about 60 Hz, about 10 Hz to about 70 Hz, about 15 Hz to about 20 Hz, about 15 Hz to about 25 Hz, about 15 Hz to about 40 Hz, about 15 Hz to about 50 Hz, about 15 Hz to about 60 Hz, about 15 Hz to about 70 Hz, about 20 Hz to about 25 Hz, about 20 Hz to about 40 Hz, about 20 Hz to about 50 Hz, about 20 Hz to about 60 Hz, about 20 Hz to about 70 Hz, about 25 Hz to about 40 Hz, about 25 Hz to about 50 Hz, about 25 Hz to about 60 Hz, about 25 Hz to about 70 Hz, about 40 Hz to about 50 Hz, about 40 Hz to about 60 Hz, about 40 Hz to about 70 Hz, about 50 Hz to about 60 Hz, about 50 Hz to about 70 Hz, or about 60 Hz to about 70 Hz. In some cases, the filter may be configured to filter the frequency band of about 1 Hz, about 5 Hz, about 10 Hz, about 15 Hz, about 20 Hz, about 25 Hz, about 40 Hz, about 50 Hz, about 60 Hz, or about 70 Hz. In some cases, the filter may be configured to filter the frequency band of at least about 1 Hz, about 5 Hz, about 10 Hz, about 15 Hz, about 20 Hz, about 25 Hz, about 40 Hz, about 50 Hz, or about 60 Hz. In some cases, the filter may be configured to filter the frequency band of at most about 5 Hz, about 10 Hz, about 15 Hz, about 20 Hz, about 25 Hz, about 40 Hz, about 50 Hz, about 60 Hz, or about 70 Hz.

After passing the signal through the filter, the systems and methods described herein, may rectify **1006** the filtered signal. In the process of rectifying the signal, as understood by one of ordinary skill in the art, the signal will convert the alternating current detected signal to a direct current signal. The rectified signal may be additionally filtered with a low pass filter **1007** that may smooth the rectified signal. The signal may be subjected to a threshold detector **1008,** where the threshold detector determines the onset of a sexual dysfunction event from a threshold intensity value of the rectified and smooth processed signals **1000** of EMG, ENG, accelerometer, gyroscope, magnetometer, pressure sensor signals, or any combination thereof. If a sexual dysfunction event is determined by the threshold detector **1008,** the system may enable the delivery of electrical stimulation **1010,** as described elsewhere herein.

In some cases, training may be supervised training. In some cases, training may be unsupervised training. In some instances, the data set may be a retrospective data set. Alternatively or in combination, the data set may be prospectively developed dataset, and the machine learning model may be iteratively improved over time.

In some aspects, the disclosure provided herein may comprise a method to train a machine learning model with a data set that comprises sensed signal profiles and excitation signals that have and have not prevented sexual dysfunction events. The method may comprise the steps of: preprocessing, training, and predicting.

The method may extract training data from a database, or intake new data, described elsewhere herein. The preprocessing step may apply one or more transformations to standardize the training data or new data for the training step or the prediction step. The preprocessed training data may be passed to the training step, which may construct a machine learning model based on training data. The training step may further comprise a validation step, configured to validate the trained machine learning model using any appropriate validation algorithm (e.g., Stratified K-fold cross-validation). In some cases, the k-fold cross-validation may comprise at least 1-fold, 2, folds, 3 folds, 4 folds, 5 folds, 6 folds, 7 folds, 8 folds, 9 folds, or 10 folds. In some cases, the k-fold cross-validation may comprise up to 1-fold, 2 folds, 3 folds, 4 folds, 5 folds, 6 folds, 7 folds, 8 folds, 9 folds, or 10 folds.

The preprocessing step may apply one or more transformations to the training data to clean and normalize the data. The preprocessing step may be configured to discard parameters which contain spurious data or contain very few observations. The preprocessing module can be further configured to standardize the encoding of parameter values. The preprocessing step may recognize the encoding variation for the same value and standardize the dataset to have a uniform encoding for a given parameter value. The processing step may thus reduce irregularities in the input data for the training and prediction steps, thereby improving the robustness of the training and prediction steps.

The training step may utilize a machine learning algorithm or other algorithm to construct and train a machine learning model to be used in the association of an excitation stimulation, sensed signal profile, and the presence or lack thereof a sexual dysfunction event. A machine learning model may be constructed to capture, based on the training data, the statistical relationship, if any, between excitation stimulation parameters, sensed signal profiles, and the presence or lack thereof a sexual dysfunction event.

The machine learning algorithm may have an accuracy greater than about 60%, 70%, 80%, 85%, 90%, 95%, or 99%. The machine learning algorithm may have a positive predictive value greater than about 60%, 70%, 75%, 80%, 85%, 90%, 95%, or 99%. The machine learning algorithm may have a negative predictive value greater than about 60%, 70%, 80%, 90%, 95%, or 99%.

Machine learning data analysis, machine learning model training, or any combination thereof may be performed using one or more of many programming languages and platforms known in the art, such as R, Weka, Python, and/or MATLAB, for example.

The use of such closed-loop bioelectronic systems may potentially provide improved electrical stimulation devices to prevent or reduce insufficient penile or clitoral erection. In some instances, individuals having the implanted device may provide feedback to the parameters provided positive outcomes, negative outcomes, or neutral outcomes. In some cases, positive outcomes may comprise preventing a sexual dysfunction event. In some instances, negative outcomes may comprise not preventing a sexual dysfunction event, producing pain, or any combination thereof. In some cases, neutral outcomes may comprise not preventing a sexual dysfunction event, not producing pain, or any combination thereof. In some cases, the positive outcomes, negative outcomes, neutral outcomes, sensor data, or any combination thereof from a plurality of individuals having the implanted device may be used in tuning algorithms to suggest changes to sensor thresholds and electrical stimulation patterns **124.**

### Electrical Simulation Pattern

In some cases, the electric stimulation pattern provided by an implantable pulse generator and one or more stimulator electrodes may be modified or changed to suit the needs of the individual in need thereof preventing an episode of sexual dysfunction. In some cases, the one or more stimulator electrodes **122** may output an electric stimulation pattern **124** or adapted stimulation in response to what is detected by the one or more sensors **120.** In some cases, the electric stimulation pattern **124** may comprise one or more electrical signals. For example, the electric stimulation pattern **124** may comprise a continuous wave signal (e.g., an electric stimulation signal with a constant frequency in time) and a burst or beating signal superimposed onto the continuous wave signal. In some cases, the burst or beating signal may only be enabled for a short duration of time compared to the continual temporal aspect of the continuous wave signal. In some instances, the combination of the continuous wave signal and/or a burst or beating signal may increase a pain threshold of a subject allowing the stimulator to provide higher amplitude electric stimulation burst pattern to prevent sexual dysfunction events. In some cases, the adapted stimulation can vary over time and arousal or erectile states.

In some instances, the frequency of the electric stimulation pattern may be modified or changed. The frequency pattern may comprise a constant profile, swept profile, beating profile, burst profile, chirped profile, monophasic profile, biphasic profile, or any combination thereof. In some instances, the constant profile is comprised of excitation values at a constant amplitude with a frequency value of 0 Hz. In some cases, a swept profile may comprise a signal with time varying frequency of excitation. In some instances, a beating profile may comprise a combination of one or more excitation signals of varying frequency. In some cases, the burst profile may comprise a signal with a constant frequency that is enveloped by a square, delta, sine, or any combination thereof envelope functions. In some instances, a monophasic profile may comprise an excitation signal with only positive or negative amplitude (e.g., signal with values from 0 to -5V or 0 to 5V only) with a constant frequency. In some cases, the beating or burst profile may comprise an electric simulation pattern that is provided to a patient and/or subject for during an on-state for a first period of time and is not provided to a patient and/or subject during an off-state for a second period of time. In some cases, beating or bust profiles may provide an excitation signal that may provide for a lengthier period of muscle excitation without suffering muscle fatigue.

In some cases, the on-state and/or off-state may comprise about 0.1 second (s) to about 6.5 s. In some cases, the on-state and/or off-state may comprise about 0.1 s to about 0.5 s, about 0.1 s to about 1 s, about 0.1 s to about 1.2 s, about 0.1 s to about 1.5 s, about 0.1 s to about 2 s, about 0.1 s to about 2.5 s, about 0.1 s to about 3 s, about 0.1 s to about 3.5 s, about 0.1 s to about 4 s, about 0.1 s to about 5 s, about 0.1 s to about 6.5 s, about 0.5 s to about 1 s, about 0.5 s to about 1.2 s, about 0.5 s to about 1.5 s, about 0.5 s to about 2 s, about 0.5 s to about 2.5 s, about 0.5 s to about 3 s, about 0.5 s to about 3.5 s, about 0.5 s to about 4 s, about 0.5 s to about 5 s, about 0.5 s to about 6.5 s, about 1 s to about 1.2 s, about 1 s to about 1.5 s, about 1 s to about 2 s, about 1 s to about 2.5 s, about 1 s to about 3 s, about 1 s to about 3.5 s, about 1 s to about 4 s, about 1 s to about 5 s, about 1 s to about 6.5 s, about 1.2 s to about 1.5 s, about 1.2 s to about 2 s, about 1.2 s to about 2.5 s, about 1.2 s to about 3 s, about 1.2 s to about 3.5 s, about 1.2 s to about 4 s, about 1.2 s to about 5 s, about 1.2 s to about 6.5 s, about 1.5 s to about 2 s, about 1.5 s to about 2.5 s, about 1.5 s to about 3 s, about 1.5 s to about 3.5 s, about 1.5 s to about 4 s, about 1.5 s to about 5 s, about 1.5 s to about 6.5 s, about 2 s to about 2.5 s, about 2 s to about 3 s, about 2 s to about 3.5 s, about 2 s to about 4 s, about 2 s to about 5 s, about 2 s to about 6.5 s, about 2.5 s to about 3 s, about 2.5 s to about 3.5 s, about 2.5 s to about 4 s, about 2.5 s to about 5 s, about 2.5 s to about 6.5 s, about 3 s to about 3.5 s, about 3 s to about 4 s, about 3 s to about 5 s, about 3 s to about 6.5 s, about 3.5 s to about 4 s, about 3.5 s to about 5 s, about 3.5 s to about 6.5 s, about 4 s to about 5 s, about 4 s to about 6.5 s, or about 5 s to about 6.5 s. In some cases, the on-state and/or off-state may comprise about 0.1 s, about 0.5 s, about 1 s, about 1.2 s, about 1.5 s, about 2 s, about 2.5 s, about 3 s, about 3.5 s, about 4 s, about 5 s, or about 6.5 s. In some cases, the on-state and/or off-state may comprise at least about 0.1 s, about 0.5 s, about 1 s, about 1.2 s, about 1.5 s, about 2 s, about 2.5 s, about 3 s, about 3.5 s, about 4 s, or about 5 s. In some cases, the on-state and/or off-state may comprise at most about 0.5 s, about 1 s, about 1.2 s, about 1.5 s, about 2 s, about 2.5 s, about 3 s, about 3.5 s, about 4 s, about 5 s, or about 6.5 s.

In some instances, an electrical stimulation pattern provided during an on-state may comprise an oscillating electric stimulation pattern. In some cases, the oscillating electric stimulation pattern may comprise one or more frequencies. In some cases, the frequency of the oscillating electrical stimulation may be chosen based upon prior knowledge of how similar subjects respond with a particular frequency or range of frequencies of the oscillating electrical stimulation pattern. In some instances, the electrical stimulation is basal stimulation. In some instances, the electrical stimulation is adapted stimulation. In some instances, the frequency of the electrical stimulation pattern may be about 1 Hz to about 60 Hz, about 0.1 Hz to 100 Hz, or about 0.1 Hz to 200 Hz. In some instances, the frequency of the electrical stimulation pattern may be about 1 Hz, about 5 Hz, about 10 Hz, about 15 Hz, about 25 Hz, about 30 Hz, about 35 Hz, about 40 Hz, about 45 Hz, about 50 Hz, about 55 Hz, about 60 Hz, about 70 Hz, about 80 Hz, about 90 Hz, or about 100 Hz. In some instances, the frequency of the electrical stimulation pattern may be at least about 1 Hz, about 5 Hz, about 10 Hz, about 15 Hz, about 25 Hz, about 30 Hz, about 35 Hz, about 40 Hz, about 45 Hz, about 50 Hz, about 55 Hz, about 60 Hz, about 70 Hz, about 80 Hz, about 90 Hz, or about 100 Hz. In some instances, the frequency of the electrical stimulation pattern may be at most about 5 Hz, about 10 Hz, about 15 Hz, about 25 Hz, about 30 Hz, about 35 Hz, about 40 Hz, about 45 Hz, about 50 Hz, about 55 Hz, about 60 Hz, about 70 Hz, about 80 Hz, about 90 Hz, or about 100 Hz. In some embodiments, the frequency refers to mean frequency of the electrical stimulation pattern. In some embodiments, the frequency refers to the median frequency. In some embodiments, the frequency refers to the maximum frequency.

In some cases, a burst signal with an on-state and/or an off-state, described elsewhere herein may be provided to the subject to prevent fatigue on the one or more muscles innervated by the subject's pudendal nerve. In some cases, the burst electrical stimulation pattern may comprise one or more frequencies described elsewhere herein.

In some instances, the amplitude of the electric stimulation pattern may be modified or changed. In some instances, the electrical stimulation is basal stimulation. In some instances, the electrical stimulation is adapted stimulation. In some instances, the amplitude of the electrical stimulation pattern may be about 0 volt (V) to about 55 V, about 0 V to about 100 V, about 0.1 V to about 60 V, or about 0.01 V to about 100 V. In some instances, the amplitude of the electrical stimulation pattern may be about 1 V, about 5 V, about 10 V, about 50 V, about 55 V, about 60 V, about 65 V, or about 100 V. In some instances, the amplitude of the electrical stimulation pattern may be at least about 1 V, about 5 V, about 10 V, about 50 V, about 55 V, about 60 V, about 65 V, or about 100 V. In some instances, the amplitude of the electrical stimulation pattern may be at most about 5 V, about 10 V, about 50 V, about 55 V, about 60 V, about 65 V, or about 100 V. In some embodiments, the amplitude refers to the mean amplitude. In some embodiments, the amplitude refers to the median amplitude. In some embodiments, the amplitude refers to the maximum amplitude. In some embodiments, the amplitude refers to peak to peak amplitude.

In some instances, the amplitude of the electrical stimulation pattern may be about 0.05 milliampere (mA) to about 100 mA. In some instances, the electrical stimulation is basal stimulation. In some instances, the electrical stimulation is adapted stimulation. In some instances, the amplitude of the electrical stimulation pattern may be about 0.05 mA to about 1 mA, about 0.05 mA to about 10 mA, about 0.05 mA to about 50 mA, or about 0.05 mA to 100 mA. In some instances, the amplitude of the electrical stimulation pattern may be about 0.05 mA, 1 mA, 10 mA, 50 mA, or 100 mA. In some instances, the amplitude of the electrical stimulation pattern may be at least about 0.05 mA, 1 mA, 10 mA, 50 mA, or100 mA. In some instances, the amplitude of the electrical stimulation pattern may be at most about 1 mA, 10 mA, 50 mA, 100 mA, 150 mA, or 200 mA. In some embodiments, the amplitude refers to the mean amplitude. In some embodiments, the amplitude refers to the median amplitude. In some embodiments, the amplitude refers to the maximum amplitude.

In some instances, the pulse width of the electric stimulation pattern may be modified or changed. In some instances, the electrical stimulation is basal stimulation. In some instances, the electrical stimulation is adapted stimulation. In some instances, the pulse width of the electrical stimulation pattern may be about 10 µs to about 1000 µs, about 10 µs to about 700 µs, about 10 µs to about 500 µs, about 50 µs to about 500 µs, about 50 µs to about 400 µs, or about 50 µs to about 300 µs. In some instances, the pulse width of the electrical stimulation pattern may be about 60 µs, about 90 µs, about 120 µs, about 150 µs, about 180 µs, about 210 µs, about 240 µs, about 270 µs, about 300 µs, about 330 µs, about 360 µs, about 390 µs, about 400 µs, or about 500 µs. In some instances, the pulse width of the electrical stimulation pattern may be at least about 60 µs, about 90 µs, about 120 µs, about 150 µs, about 180 µs, about 210 µs, about 240 µs, about 270 µs, about 300 µs, about 330 µs, about 360 µs, about 400 µs, or about 500 µs. In some instances, the pulse width of the electrical stimulation pattern may be at most about 90 µs, about 120 µs, about 150 µs, about 180 µs, about 210 µs, about 240 µs, about 270 µs, about 300 µs, about 330 µs, about 360 µs, about 400 µs, about 500 µs, or about 1000 µs. In some embodiments, the pulse width refers to the mean pulse width. In some embodiments, the pulse width refers to the median pulse width. In some embodiments, the pulse width refers to the maximum pulse width.

### Combined Treatment

In some cases, the methods, devices, and systems provided herein may be used in combination with other approaches to treat symptoms of sexual dysfunction. In some cases, treatment for sexual dysfunction comprises one or more of medication, mechanical aid, electrical stimulation, psychotherapy, and behavioral treatments. In some cases, the medication comprises a hormone, shot, pill, or cream; or a phosphodiesterase type 5 (PDE5) inhibitor to increase blood flow to the penis; or injection papaverine, phentolamine, and/or Prostaglandin E1 (PGE1) at or near the erectile tissue. In some cases, the penile or clitoral engorgement produced by electrostimulation can be potentiated by a medicament to treat sexual dysfunction. In some cases, the medication alone is not sufficient to treat the episode of sexual dysfunction. In some cases, the medication in combination with the methods, devices, and systems provided herein have a synergistic effect. In some instances, less medication is needed for efficacy when an individual is treated with methods, devices, and systems provided herein than without methods, devices, and systems provided herein. In some cases, PDE5 inhibitors comprise one or more of sildenafil, tadalafil, vardenafil, avanafil, lodenafil, udenafil, and mirodenafil. Although PDE5 inhibitors are effective in treating sexual function, discontinuation rates may be high, reported as high as 50% after 1-2 years of use. In some cases, intracavernosal injections with papaverine, phentolamine, and/or PGE1 may be administered to the patient if medication is ineffective. In some cases, intracavernosal injections result in a high incidence of adverse effects, such as priapism, injection site pain, bruising. In some cases, high incidences of adverse effects lead to patient non-compliance. In some cases, mechanical aids include but are not limited to vacuum devices, penile implants, vaginal dilators, and vibrators. In some cases, penile prosthesis implantation result in significant complications, such as infection, erosion and pain.

In some cases, the adjunct treatment of sexual dysfunction may be used before the methods, devices, and systems provided herein. In some cases, the adjunct treatment of sexual dysfunction may be used about 1 hour, 6 hours, 12 hours, 24 hours, 2 day, 3 day, 4 day, 5 day, 6 day, 7 day before the methods, devices, and systems provided herein. In some cases, the adjunct treatment of sexual dysfunction may be used after the methods, devices, and systems provided herein. In some cases, the adjunct treatment of sexual dysfunction may be used about1 hour, 6 hours, 12 hours, 24 hours, 2 day, 3 day, 4 day, 5 day, 6 day, 7 day after the methods, devices, and systems provided herein. In some cases, the adjunct treatment of sexual dysfunction may be used concurrently with the methods, devices, and systems provided herein.

### Sensors for Use with Embodiments of the Device Described Herein

In some instances, a sensor for use with embodiments of the device described herein may be configured to senses a parameter associated with a response of an individual intending to prevent an episode of sexual dysfunction or that indicates the individual may have an episode of sexual dysfunction. In some cases, the sensor may be configured to sense a contraction of a muscle of the individual that results in a change in intracorporal pressure of the erectile tissue. In some cases, a sensor may be configured to sense bulk motion or anatomical stress of an individual. In some instances, the sensor may comprise a sensor configured to detect electromyography (EMG) signals. In some cases, the sensor may be configured to sense myoelectric activity. In some instances, an EMG signal threshold may determine that a contraction of at least one pelvic muscle has occurred. In some cases, the strength of the EMG signal may be proportional to the strength of the contraction of at least one pelvic muscle. In some instances, the sensor detects an action potential signal. In some cases, the device comprises an amplifier to amplify the signal obtained by the sensor, to facilitate analysis and classification of the signal by the processor.

In some cases, the sensor may be implanted within the pelvis of the individual. In some instances, the sensor may be implanted at or adjacent to one or more pudendal nerves. In some cases, the sensor may be implanted at or adjacent to one or more sacral nerves. In some cases, the sensor may be implanted within or adjacent to one or more of the pelvic muscles. In some cases, the sensor may be implanted within or adjacent to one or more of the pelvic plexus nerves. In some instances, the sensor may be implanted at or adjacent to pelvic nerves and muscles. In some instances, the devices described herein may comprise a plurality of sensors. In some cases, the devices described herein may comprise one or more sensors. In some instances, the devices described herein may comprise a different sensor, or a second sensor. In some cases, the sensor may detect the signal from a muscle area innervated by a first pudendal nerve and the different or second sensor may detect signal from a muscle area innervated by a second pudendal nerve. In some instances, the sensor may detect a signal from a muscle area innervated by a first sacral nerve and the different or second sensor detects a signal from a muscle area innervated by a second sacral nerve.

In some instances, the sensor may comprise a casing and a lead. In some instances, the casing may be made of titanium, titanium alloy, tantalum, or any combination thereof. In some cases, the lead may be made of a metal alloy. In some embodiments, a sensor and a stimulator electrode may be positioned on a single lead. In some instances, the lead may be electrically coupled to one or more sensors or one or more stimulator electrodes. In some embodiments, a sensor and a stimulator electrode are positioned on separate leads. In some cases, the one or more sensors may comprise bioelectrical sensors.

### Electrodes

In some cases, the sensor and the stimulator electrode may be located on a single lead. In some instances, one or more sensors and one or more stimulator electrodes may be located on a single lead. In some cases, one sensor and one stimulator electrode may be located on a single lead. In some instances, the sensor and the stimulator electrode may be located on separate leads. In some cases, the sensor and the stimulator electrode may each be located on its own lead. In some cases, the one or more sensors and one or more stimulator electrodes may be in a linear geometry, triangular geometry, square geometry, hexagonal geometry, or a general polygonal geometry. In some cases, the electrodes located on a single lead may be spaced by a distance. In some cases, the spacing provides the capability to stimulate multiple locations along the length of the nerve. In some instances, the sensor may comprise casing and a lead. In some instances, the casing may be made of titanium or a titanium alloy. In some cases, a lead may be made of a metal alloy. In some instances, the electrode leads can be wires, conical or split electrode leads, a wrapping cuff, or paddle electrode.

In some cases, the electrodes may be separated by a distance of at least about 1 mm, about 1.5 mm, about 2 mm, about 2.5 mm, about 5 mm, about 10 mm, about 20 mm, about 30 mm, about 40 mm, about 50 mm, or about 60 mm. In some cases, the electrodes may be separated by a distance of at most about 1.5 mm, about 2 mm, about 2.5 mm, about 5 mm, about 10 mm, about 20 mm, about 30 mm, about 40 mm, about 50 mm, about 60 mm, or about 80 mm.

In some instances, the device may comprise one or more leads. In some cases, the device may comprise at least two leads. In some instances, the device may comprise at least 1, 2, 3, 4, 5, 6, 7, 8, 9, or 10 leads. In some cases, the device may comprise at most 1, 2, 3, 4, 5, 6, 7, 8, 9, or 10 leads. In some instances, the device may comprise 1, 2, 3, 4, 5, 6, 7, 8, 9, or 10 leads. In some cases, each lead may comprise one or more electrodes. In some cases, the one or more electrodes may comprise one or more sensor and/or stimulation electrodes. In some cases, the one or more electrodes on a lead may comprise about 1 electrode to about 10 electrodes. In some cases, the one or more electrodes on a lead may comprise about 1 electrode to about 2 electrodes, about 1 electrode to about 3 electrodes, about 1 electrode to about 4 electrodes, about 1 electrode to about 5 electrodes, about 1 electrode to about 6 electrodes, about 1 electrode to about 7 electrodes, about 1 electrode to about 8 electrodes, about 1 electrode to about 9 electrodes, about 1 electrode to about 10 electrodes, about 2 electrodes to about 3 electrodes, about 2 electrodes to about 4 electrodes, about 2 electrodes to about 5 electrodes, about 2 electrodes to about 6 electrodes, about 2 electrodes to about 7 electrodes, about 2 electrodes to about 8 electrodes, about 2 electrodes to about 9 electrodes, about 2 electrodes to about 10 electrodes, about 3 electrodes to about 4 electrodes, about 3 electrodes to about 5 electrodes, about 3 electrodes to about 6 electrodes, about 3 electrodes to about 7 electrodes, about 3 electrodes to about 8 electrodes, about 3 electrodes to about 9 electrodes, about 3 electrodes to about 10 electrodes, about 4 electrodes to about 5 electrodes, about 4 electrodes to about 6 electrodes, about 4 electrodes to about 7 electrodes, about 4 electrodes to about 8 electrodes, about 4 electrodes to about 9 electrodes, about 4 electrodes to about 10 electrodes, about 5 electrodes to about 6 electrodes, about 5 electrodes to about 7 electrodes, about 5 electrodes to about 8 electrodes, about 5 electrodes to about 9 electrodes, about 5 electrodes to about 10 electrodes, about 6 electrodes to about 7 electrodes, about 6 electrodes to about 8 electrodes, about 6 electrodes to about 9 electrodes, about 6 electrodes to about 10 electrodes, about 7 electrodes to about 8 electrodes, about 7 electrodes to about 9 electrodes, about 7 electrodes to about 10 electrodes, about 8 electrodes to about 9 electrodes, about 8 electrodes to about 10 electrodes, or about 9 electrodes to about 10 electrodes. In some cases, the one or more electrodes on a lead may comprise about 1 electrode, about 2 electrodes, about 3 electrodes, about 4 electrodes, about 5 electrodes, about 6 electrodes, about 7 electrodes, about 8 electrodes, about 9 electrodes, or about 10 electrodes. In some cases, the one or more electrodes on a lead may comprise at least about 1 electrode, about 2 electrodes, about 3 electrodes, about 4 electrodes, about 5 electrodes, about 6 electrodes, about 7 electrodes, about 8 electrodes, or about 9 electrodes. In some cases, the one or more electrodes on a lead may comprise at most about 2 electrodes, about 3 electrodes, about 4 electrodes, about 5 electrodes, about 6 electrodes, about 7 electrodes, about 8 electrodes, about 9 electrodes, or about 10 electrodes.

In some cases, each electrode may comprise a length, whereby the length may provide localized excitation of one or more nerves of the sacral or pudendal nerve. In some cases, each electrode may comprise a length of about 0.1 millimeter (mm) to about 2 mm. In some cases, each electrode may comprise a length of about 0.1 mm to about 0.3 mm, about 0.1 mm to about 0.5 mm, about 0.1 mm to about 0.7 mm, about 0.1 mm to about 0.8 mm, about 0.1 mm to about 1 mm, about 0.1 mm to about 1.2 mm, about 0.1 mm to about 1.4 mm, about 0.1 mm to about 1.5 mm, about 0.1 mm to about 2 mm, about 0.3 mm to about 0.5 mm, about 0.3 mm to about 0.7 mm, about 0.3 mm to about 0.8 mm, about 0.3 mm to about 1 mm, about 0.3 mm to about 1.2 mm, about 0.3 mm to about 1.4 mm, about 0.3 mm to about 1.5 mm, about 0.3 mm to about 2 mm, about 0.5 mm to about 0.7 mm, about 0.5 mm to about 0.8 mm, about 0.5 mm to about 1 mm, about 0.5 mm to about 1.2 mm, about 0.5 mm to about 1.4 mm, about 0.5 mm to about 1.5 mm, about 0.5 mm to about 2 mm, about 0.7 mm to about 0.8 mm, about 0.7 mm to about 1 mm, about 0.7 mm to about 1.2 mm, about 0.7 mm to about 1.4 mm, about 0.7 mm to about 1.5 mm, about 0.7 mm to about 2 mm, about 0.8 mm to about 1 mm, about 0.8 mm to about 1.2 mm, about 0.8 mm to about 1.4 mm, about 0.8 mm to about 1.5 mm, about 0.8 mm to about 2 mm, about 1 mm to about 1.2 mm, about 1 mm to about 1.4 mm, about 1 mm to about 1.5 mm, about 1 mm to about 2 mm, about 1.2 mm to about 1.4 mm, about 1.2 mm to about 1.5 mm, about 1.2 mm to about 2 mm, about 1.4 mm to about 1.5 mm, about 1.4 mm to about 2 mm, or about 1.5 mm to about 2 mm. In some cases, each electrode may comprise a length of about 0.1 mm, about 0.3 mm, about 0.5 mm, about 0.7 mm, about 0.8 mm, about 1 mm, about 1.2 mm, about 1.4 mm, about 1.5 mm, or about 2 mm. In some cases, each electrode may comprise a length of at least about 0.1 mm, about 0.3 mm, about 0.5 mm, about 0.7 mm, about 0.8 mm, about 1 mm, about 1.2 mm, about 1.4 mm, or about 1.5 mm. In some cases, each electrode may comprise a length of at most about 0.3 mm, about 0.5 mm, about 0.7 mm, about 0.8 mm, about 1 mm, about 1.2 mm, about 1.4 mm, about 1.5 mm, or about 2 mm.

In some cases, the one or more leads may comprise a length between the proximal tip of the lead to the distal end electrically coupled to the stimulator. In some cases, the length of the lead may vary based upon the anatomy of the individual or subject receiving the implanted device and leads. In some cases, the lead length may comprise a length, whereby the lead electrodes may be placed near and/or adjacent to the sacral and/or pudendal nerve yet reach the placement of the stimulator in buttock fat pockets of the subject. In some instances, the one or more leads may comprise a length of about 20 centimeters (cm) to about 50 cm. In some instances, the one or more leads may comprise a length of about 20 cm to about 25 cm, about 20 cm to about 30 cm, about 20 cm to about 35 cm, about 20 cm to about 40 cm, about 20 cm to about 45 cm, about 20 cm to about 50 cm, about 25 cm to about 30 cm, about 25 cm to about 35 cm, about 25 cm to about 40 cm, about 25 cm to about 45 cm, about 25 cm to about 50 cm, about 30 cm to about 35 cm, about 30 cm to about 40 cm, about 30 cm to about 45 cm, about 30 cm to about 50 cm, about 35 cm to about 40 cm, about 35 cm to about 45 cm, about 35 cm to about 50 cm, about 40 cm to about 45 cm, about 40 cm to about 50 cm, or about 45 cm to about 50 cm. In some instances, the one or more leads may comprise a length of about 20 cm, about 25 cm, about 30 cm, about 35 cm, about 40 cm, about 45 cm, or about 50 cm. In some instances, the one or more leads may comprise a length of at least about 20 cm, about 25 cm, about 30 cm, about 35 cm, about 40 cm, or about 45 cm. In some instances, the one or more leads may comprise a length of at most about 25 cm, about 30 cm, about 35 cm, about 40 cm, about 45 cm, or about 50 cm.

In some cases, the one or more leads may comprise a diameter. In some cases, the diameter of the lead may vary based upon the anatomy of the individual or subject receiving the implanted device and leads. In some cases, the lead diameter may comprise a diameter, whereby the diameter provides a form factor for minimally invasive placement of the lead in the subject. In some cases, the diameter of the lead may comprise a diameter at which the lead will resist breakage. In some cases, the one or more leads may have an outer diameter of about 0.1 mm to about 2 mm. In some cases, the one or more leads may have an outer diameter of about 0.1 mm to about 0.2 mm, about 0.1 mm to about 0.3 mm, about 0.1 mm to about 0.5 mm, about 0.1 mm to about 0.8 mm, about 0.1 mm to about 1 mm, about 0.1 mm to about 1.2 mm, about 0.1 mm to about 1.4 mm, about 0.1 mm to about 1.5 mm, about 0.1 mm to about 2 mm, about 0.2 mm to about 0.3 mm, about 0.2 mm to about 0.5 mm, about 0.2 mm to about 0.8 mm, about 0.2 mm to about 1 mm, about 0.2 mm to about 1.2 mm, about 0.2 mm to about 1.4 mm, about 0.2 mm to about 1.5 mm, about 0.2 mm to about 2 mm, about 0.3 mm to about 0.5 mm, about 0.3 mm to about 0.8 mm, about 0.3 mm to about 1 mm, about 0.3 mm to about 1.2 mm, about 0.3 mm to about 1.4 mm, about 0.3 mm to about 1.5 mm, about 0.3 mm to about 2 mm, about 0.5 mm to about 0.8 mm, about 0.5 mm to about 1 mm, about 0.5 mm to about 1.2 mm, about 0.5 mm to about 1.4 mm, about 0.5 mm to about 1.5 mm, about 0.5 mm to about 2 mm, about 0.8 mm to about 1 mm, about 0.8 mm to about 1.2 mm, about 0.8 mm to about 1.4 mm, about 0.8 mm to about 1.5 mm, about 0.8 mm to about 2 mm, about 1 mm to about 1.2 mm, about 1 mm to about 1.4 mm, about 1 mm to about 1.5 mm, about 1 mm to about 2 mm, about 1.2 mm to about 1.4 mm, about 1.2 mm to about 1.5 mm, about 1.2 mm to about 2 mm, about 1.4 mm to about 1.5 mm, about 1.4 mm to about 2 mm, or about 1.5 mm to about 2 mm. In some cases, the one or more leads may have an outer diameter of about 0.1 mm, about 0.2 mm, about 0.3 mm, about 0.5 mm, about 0.8 mm, about 1 mm, about 1.2 mm, about 1.4 mm, about 1.5 mm, or about 2 mm. In some cases, the one or more leads may have an outer diameter of at least about 0.1 mm, about 0.2 mm, about 0.3 mm, about 0.5 mm, about 0.8 mm, about 1 mm, about 1.2 mm, about 1.4 mm, or about 1.5 mm. In some cases, the one or more leads may have an outer diameter of at most about 0.2 mm, about 0.3 mm, about 0.5 mm, about 0.8 mm, about 1 mm, about 1.2 mm, about 1.4 mm, about 1.5 mm, or about 2 mm.

In some cases, the one or more leads described elsewhere herein may comprise an internal stylet and/or mandrel configured to provide rigidity to the lead for implantation and/or insertion to a subject. In some cases, the internal stylet may be removed from the lead once the lead has been inserted and implanted. In some instances, the one or more leads described elsewhere herein may be sterilizable with conventional methods of sterilization used in the medical field, e.g., gas sterilization, steam sterilization, UV sterilization, etc.

In some instances, the one or more leads, described elsewhere herein, may be electrically coupled to the electric stimulator, described elsewhere herein. In some instances, the one or more leads may be coupled to the stimulator and may at a later point in time be uncoupled from the stimulator. In some instances, the one or more leads may couple to the electric stimulator with a quick release electrical coupling. In some cases, the one or more leads may be coupled to the electric stimulator by a set screw fastener, whereby a lead is inserted into a hollow cylindrical geometry in electrical communication with the electric stimulator internal circuitry. The lead may then be fastened i.e., held in tension against the inner wall of the hollow cylindrical geometry, to the conductive hollow cylindrical geometry with a non-conductive machine set screw. The one or more leads may be placed into the electric stimulator prior to or during the surgical implantation procedure.

In some instances, the sensor and the stimulator electrode may be operatively coupled to a processor and a non-transitory computer readable medium that comprises software. In some cases, the sensor may be calibrated by the individual using an external input device that interfaces with the software. In some instances, the software may be configured to record a signal from the sensor. In some cases, the software may be configured to adjust the sensor in response to the signal.

In some instances, the stimulator electrode may provide an electrical stimulation to the pudendal nerve. In some cases, the stimulator electrode may provide an electrical stimulation to the sacral nerve. In some instances, the stimulator electrode may provide an electrical stimulation to one or more of the nerves innervating the pelvic muscles. In some cases, the electrode may comprise one or more electrodes or leads e.g., a first and second electrode. In some instances, the electrode may comprise one or more stimulator electrodes. In some cases, an electrode may comprise a first stimulator electrode configured to stimulate one pudendal nerve, and a second stimulator electrode configured to stimulate another spatially independent region of the same pudendal nerve. In some cases, the first stimulator electrode may stimulate the main trunk of the pudendal nerve and the second stimulator electrode may stimulate the distal nerve of the pudendal nerve. In some instances, the distal nerve of the pudendal nerve comprises branches thereof the distal pudendal nerve. In some cases, the first stimulator electrode may stimulate the trunk of the pudendal nerve and the second stimulator electrode may stimulate a main branch of the pudendal nerve e.g., dorsal genital nerve. In some cases, the stimulator electrode may provide an electrical stimulation to one or more of the nerves innervating the pelvic muscles. In some instances, the stimulator electrode may provide an electrical stimulation to one or more of the nerves innervating the erectile tissue. In some instances, the stimulator electrode may provide an electrical stimulation to one or more of the nerves innervating the erectile tissue. In some cases, the stimulator electrode may provide an electrical stimulation to one or more of the nerves innervating the penis or clitoris. In some instances, the devices described herein may comprise a plurality of stimulator electrodes. In some instances, the devices described herein may comprise one or more stimulator electrodes. In some instances, the devices described herein may comprise a different stimulator electrode, or a second stimulator electrode. In some cases, the stimulator electrode may stimulate a first pudendal nerve and the different or second stimulator electrode stimulates a second pudendal nerve. In some cases, the stimulator electrode may stimulate a first sacral nerve and the different or second stimulator electrode stimulates a second sacral nerve.

In some instances, the stimulator electrode of the device may provide a constant electrical stimulation. In some cases, the stimulator electrode of the device may provide a constant electrical stimulation at a lower intensity level than the electrical stimulation provided to prevent an episode of sexual dysfunction. In some cases, constant electrical stimulation may comprise constant frequency, amplitude, current, or any combination thereof. In some instances, the intensity or duration of the electrical stimulation provided may prevent an episode of sexual dysfunction varies according to the individual's response to a possible episode of sexual dysfunction that is sensed by the sensor. In some cases, the individual's response to prevent a possible episode of sexual dysfunction that is sensed by the sensor may be insufficient on its own to prevent the episode of sexual dysfunction and the electrical stimulation delivered by the stimulator electrode of the device provides sufficient stimulation, together with the response, to prevent the episode of sexual dysfunction. In some cases, the individual's response to prevent a possible episode of sexual dysfunction that may be sensed by the sensor combined with the electrical stimulation delivered by the stimulator electrode of the device provides sufficient stimulation to trigger the action potential of the muscles responsible for the sexual dysfunction, resulting in contraction of the muscle. In some cases, the combined stimulation from the individual and the device may result in contraction of a muscle.

### Device Anchors

In some cases, the devices may be anchored when implanted by one the one or more surgical device. In some instances, anchoring of the devices may be achieved by sliding the device over the lead, described elsewhere herein, and then compressing it onto the lead using ligatures such that it is immobile. These ligatures may be used to fix the anchoring device to native adjacent tissue such as ligament or periosteum. In some instances, the device may comprise groves for the purpose of aligning compression ligatures. In some instances, the anchoring device may comprise a torque system to compress the device onto the lead such that it is immobile. In some instances, the device may be compressed at a single point onto the lead. In some instances, the device is compressed at two or more points onto the lead. The electric stimulator may comprise radio-opaque markers that may permit visualization of the electric stimulator under x-ray e.g., fluoroscopy during and/or after implantation. In some instances, fluoroscopy may be used alone or in combination with EMG sensor readings of pelvic muscles to verify placement or to adjustment placement of sensor leads, stimulator electrode leads, and/or the stimulator.

### Electrical Signal

**FIG. 4B** depicts the sensed myoelectric EMG signal and a corresponding electrical stimulation ("Bio-stimulator") provided by the devices disclosed herein. In some cases, the myoelectric EMG signal ("Bio-signal") **140** may comprise electrical fluctuations **142** corresponding to contractile activity of the muscle near the sensor. In some cases, the electrical fluctuations **142** may represent a cough, sudden movement, change in inertia, or any combination thereof from an electromyography (EMG) reading. In some instances, the bio-signal captured by the sensor may be analyzed and classified **144** to identify any sexual dysfunction events from an EMG reading. In some cases, the identified sexual dysfunction event may initiate a process **146** by which the implantable electric stimulator may deliver an electric stimulation pattern **148** to via one or more stimulator electrodes to a one or more pudendal nerve to prevent an episode of sexual dysfunction. In some cases, the stimulator electrode may deliver an electrical stimulation by the one or more stimulator electrodes **122** that is configured to supplement the innate reflex detected by the one or more sensors **120** to account for the sexual dysfunction event otherwise leading to a sexual dysfunction event.

In some cases, the electric stimulator may comprise a width and length to be readily implantable in a patient. In some cases, the electric stimulator may comprise a width and length to accommodate circuitry and/or other system level components, described elsewhere herein.

In some instances, the electric stimulator may comprise a width and length to provide sufficient space for a battery, where the battery may comprise a lifetime after which the battery may be replaced. In some cases, the battery lifetime may comprise about 1 year to about 15 years.

In some cases, the electric stimulator battery may require charging once in about 1 day to about 14 days. In some cases, the electric stimulator battery may require charging once in about 1 day, about 2 days, about 3 days, about 4 days, about 5 days, about 6 days, about 7 days, about 8 days, about 9 days, about 10 days, about 11 days, about 12 days, about 13 days, or about 14 days. In some cases, the electric stimulator battery may require charging once in at least about 1 day, about 2 days, about 3 days, about 4 days, about 5 days, about 6 days, about 7 days, about 8 days, about 9 days, about 10 days, or about 11 days. In some cases, the electric stimulator battery may require charging once in at most about 2 days, about 3 days, about 4 days, about 5 days, about 6 days, about 7 days, about 8 days, about 9 days, about 10 days, about 11 days, or about 12 days.

In some instances, the electric stimulator may comprise a width of about 1 mm to about 50 mm. In some instances, the electric stimulator may comprise a width of about 1 mm to about 5 mm, about 1 mm to about 10 mm, about 1 mm to about 15 mm, about 1 mm to about 20 mm, about 1 mm to about 25 mm, about 1 mm to about 30 mm, about 1 mm to about 35 mm, about 1 mm to about 40 mm, about 1 mm to about 45 mm, about 1 mm to about 50 mm, about 5 mm to about 10 mm, about 5 mm to about 15 mm, about 5 mm to about 20 mm, about 5 mm to about 25 mm, about 5 mm to about 30 mm, about 5 mm to about 35 mm, about 5 mm to about 40 mm, about 5 mm to about 45 mm, about 5 mm to about 50 mm, about 10 mm to about 15 mm, about 10 mm to about 20 mm, about 10 mm to about 25 mm, about 10 mm to about 30 mm, about 10 mm to about 35 mm, about 10 mm to about 40 mm, about 10 mm to about 45 mm, about 10 mm to about 50 mm, about 15 mm to about 20 mm, about 15 mm to about 25 mm, about 15 mm to about 30 mm, about 15 mm to about 35 mm, about 15 mm to about 40 mm, about 15 mm to about 45 mm, about 15 mm to about 50 mm, about 20 mm to about 25 mm, about 20 mm to about 30 mm, about 20 mm to about 35 mm, about 20 mm to about 40 mm, about 20 mm to about 45 mm, about 20 mm to about 50 mm, about 25 mm to about 30 mm, about 25 mm to about 35 mm, about 25 mm to about 40 mm, about 25 mm to about 45 mm, about 25 mm to about 50 mm, about 30 mm to about 35 mm, about 30 mm to about 40 mm, about 30 mm to about 45 mm, about 30 mm to about 50 mm, about 35 mm to about 40 mm, about 35 mm to about 45 mm, about 35 mm to about 50 mm, about 40 mm to about 45 mm, about 40 mm to about 50 mm, or about 45 mm to about 50 mm. In some instances, the electric stimulator may comprise a width of about 1 mm, about 5 mm, about 10 mm, about 15 mm, about 20 mm, about 25 mm, about 30 mm, about 35 mm, about 40 mm, about 45 mm, or about 50 mm. In some instances, the electric stimulator may comprise a width of at least about 1 mm, about 5 mm, about 10 mm, about 15 mm, about 20 mm, about 25 mm, about 30 mm, about 35 mm, about 40 mm, or about 45 mm. In some instances, the electric stimulator may comprise a width of at most about 5 mm, about 10 mm, about 15 mm, about 20 mm, about 25 mm, about 30 mm, about 35 mm, about 40 mm, about 45 mm, or about 50 mm.

In some instances, the electric stimulator may comprise a length of about 1 mm to about 50 mm. In some instances, the electric stimulator may comprise a length of about 1 mm to about 5 mm, about 1 mm to about 10 mm, about 1 mm to about 15 mm, about 1 mm to about 20 mm, about 1 mm to about 25 mm, about 1 mm to about 30 mm, about 1 mm to about 35 mm, about 1 mm to about 40 mm, about 1 mm to about 45 mm, about 1 mm to about 50 mm, about 5 mm to about 10 mm, about 5 mm to about 15 mm, about 5 mm to about 20 mm, about 5 mm to about 25 mm, about 5 mm to about 30 mm, about 5 mm to about 35 mm, about 5 mm to about 40 mm, about 5 mm to about 45 mm, about 5 mm to about 50 mm, about 10 mm to about 15 mm, about 10 mm to about 20 mm, about 10 mm to about 25 mm, about 10 mm to about 30 mm, about 10 mm to about 35 mm, about 10 mm to about 40 mm, about 10 mm to about 45 mm, about 10 mm to about 50 mm, about 15 mm to about 20 mm, about 15 mm to about 25 mm, about 15 mm to about 30 mm, about 15 mm to about 35 mm, about 15 mm to about 40 mm, about 15 mm to about 45 mm, about 15 mm to about 50 mm, about 20 mm to about 25 mm, about 20 mm to about 30 mm, about 20 mm to about 35 mm, about 20 mm to about 40 mm, about 20 mm to about 45 mm, about 20 mm to about 50 mm, about 25 mm to about 30 mm, about 25 mm to about 35 mm, about 25 mm to about 40 mm, about 25 mm to about 45 mm, about 25 mm to about 50 mm, about 30 mm to about 35 mm, about 30 mm to about 40 mm, about 30 mm to about 45 mm, about 30 mm to about 50 mm, about 35 mm to about 40 mm, about 35 mm to about 45 mm, about 35 mm to about 50 mm, about 40 mm to about 45 mm, about 40 mm to about 50 mm, or about 45 mm to about 50 mm. In some instances, the electric stimulator may comprise a length of about 1 mm, about 5 mm, about 10 mm, about 15 mm, about 20 mm, about 25 mm, about 30 mm, about 35 mm, about 40 mm, about 45 mm, or about 50 mm. In some instances, the electric stimulator may comprise a length of at least about 1 mm, about 5 mm, about 10 mm, about 15 mm, about 20 mm, about 25 mm, about 30 mm, about 35 mm, about 40 mm, or about 45 mm. In some instances, the electric stimulator may comprise a length of at most about 5 mm, about 10 mm, about 15 mm, about 20 mm, about 25 mm, about 30 mm, about 35 mm, about 40 mm, about 45 mm, or about 50 mm.

In some instances, the electric stimulator may comprise a height of about 0.5 mm to about 5.5 mm. In some instances, the electric stimulator may comprise a height of about 0.5 mm to about 1 mm, about 0.5 mm to about 1.5 mm, about 0.5 mm to about 2 mm, about 0.5 mm to about 2.5 mm, about 0.5 mm to about 3 mm, about 0.5 mm to about 3.5 mm, about 0.5 mm to about 4 mm, about 0.5 mm to about 4.5 mm, about 0.5 mm to about 5 mm, about 0.5 mm to about 5.5 mm, about 1 mm to about 1.5 mm, about 1 mm to about 2 mm, about 1 mm to about 2.5 mm, about 1 mm to about 3 mm, about 1 mm to about 3.5 mm, about 1 mm to about 4 mm, about 1 mm to about 4.5 mm, about 1 mm to about 5 mm, about 1 mm to about 5.5 mm, about 1.5 mm to about 2 mm, about 1.5 mm to about 2.5 mm, about 1.5 mm to about 3 mm, about 1.5 mm to about 3.5 mm, about 1.5 mm to about 4 mm, about 1.5 mm to about 4.5 mm, about 1.5 mm to about 5 mm, about 1.5 mm to about 5.5 mm, about 2 mm to about 2.5 mm, about 2 mm to about 3 mm, about 2 mm to about 3.5 mm, about 2 mm to about 4 mm, about 2 mm to about 4.5 mm, about 2 mm to about 5 mm, about 2 mm to about 5.5 mm, about 2.5 mm to about 3 mm, about 2.5 mm to about 3.5 mm, about 2.5 mm to about 4 mm, about 2.5 mm to about 4.5 mm, about 2.5 mm to about 5 mm, about 2.5 mm to about 5.5 mm, about 3 mm to about 3.5 mm, about 3 mm to about 4 mm, about 3 mm to about 4.5 mm, about 3 mm to about 5 mm, about 3 mm to about 5.5 mm, about 3.5 mm to about 4 mm, about 3.5 mm to about 4.5 mm, about 3.5 mm to about 5 mm, about 3.5 mm to about 5.5 mm, about 4 mm to about 4.5 mm, about 4 mm to about 5 mm, about 4 mm to about 5.5 mm, about 4.5 mm to about 5 mm, about 4.5 mm to about 5.5 mm, or about 5 mm to about 5.5 mm. In some instances, the electric stimulator may comprise a height of about 0.5 mm, about 1 mm, about 1.5 mm, about 2 mm, about 2.5 mm, about 3 mm, about 3.5 mm, about 4 mm, about 4.5 mm, about 5 mm, or about 5.5 mm. In some instances, the electric stimulator may comprise a height of at least about 0.5 mm, about 1 mm, about 1.5 mm, about 2 mm, about 2.5 mm, about 3 mm, about 3.5 mm, about 4 mm, about 4.5 mm, or about 5 mm. In some instances, the electric stimulator may comprise a height of at most about 1 mm, about 1.5 mm, about 2 mm, about 2.5 mm, about 3 mm, about 3.5 mm, about 4 mm, about 4.5 mm, about 5 mm, or about 5.5 mm.

In some instances, the electric stimulator may comprise a mass of about 1 g to about 18 g. In some instances, the electric stimulator may comprise a mass of about 1 g to about 3 g, about 1 g to about 6 g, about 1 g to about 8 g, about 1 g to about 10 g, about 1 g to about 12 g, about 1 g to about 14 g, about 1 g to about 16 g, about 1 g to about 17 g, about 1 g to about 18 g, about 3 g to about 6 g, about 3 g to about 8 g, about 3 g to about 10 g, about 3 g to about 12 g, about 3 g to about 14 g, about 3 g to about 16 g, about 3 g to about 17 g, about 3 g to about 18 g, about 6 g to about 8 g, about 6 g to about 10 g, about 6 g to about 12 g, about 6 g to about 14 g, about 6 g to about 16 g, about 6 g to about 17 g, about 6 g to about 18 g, about 8 g to about 10 g, about 8 g to about 12 g, about 8 g to about 14 g, about 8 g to about 16 g, about 8 g to about 17 g, about 8 g to about 18 g, about 10 g to about 12 g, about 10 g to about 14 g, about 10 g to about 16 g, about 10 g to about 17 g, about 10 g to about 18 g, about 12 g to about 14 g, about 12 g to about 16 g, about 12 g to about 17 g, about 12 g to about 18 g, about 14 g to about 16 g, about 14 g to about 17 g, about 14 g to about 18 g, about 16 g to about 17 g, about 16 g to about 18 g, or about 17 g to about 18 g. In some instances, the electric stimulator may comprise a mass of about 1 g, about 3 g, about 6 g, about 8 g, about 10 g, about 12 g, about 14 g, about 16 g, about 17 g, or about 18 g. In some instances, the electric stimulator may comprise a mass of at least about 1 g, about 3 g, about 6 g, about 8 g, about 10 g, about 12 g, about 14 g, about 16 g, or about 17 g. In some instances, the electric stimulator may comprise a mass of at most about 3 g, about 6 g, about 8 g, about 10 g, about 12 g, about 14 g, about 16 g, about 17 g, or about 18 g.

In some instances, the electric stimulator may be sterilizable with conventional methods of sterilization used in the medical field, e.g., gas sterilization, steam sterilization, UV sterilization, etc.

In some cases, the one or more stimulator electrodes may provide a constant electrical stimulation, or base stimulation 146 and 150 for an individual experiencing sexual dysfunction. In some cases, constant electrical stimulation may comprise constant frequency, amplitude, current, or any combination thereof. In some instances, the stimulator electrode may provide a temporary electrical stimulation lasting the duration of an insufficient penile or clitoral erection **148** episode for an individual experiencing insufficient penile or clitoral erection. In some cases, the stimulator electrode may provide a constant electrical stimulation (i.e., basal stimulation pattern) with a temporary activated stimulation (i.e., activation stimulation pattern) lasting the duration of an insufficient penile or clitoral erection episode for an individual experiencing insufficient penile or clitoral erection.

In some instances, the device disclosed herein may comprise a non-transitory computer readable medium that comprises software. In some cases, the software may be configured to record a signal from the one or more sensors. In some cases, the software may be configured to process the recorded signal from the one or more sensors to determine whether an electrical stimulation pattern may need to be delivered to the one or more stimulator electrodes innervating the one or more pudendal nerves. In some instances, the software may be configured to adjust parameters of the sensor in response to the observed signal. In some cases, the recorded signal of the sensor by the software may observe a signal that saturates the sensor's dynamic range. In some cases, the gain of the sensor may be adjusted by the software to allow for sufficient monitoring and thresholding of the myoelectric EMG signals of the individual.

### Nerve Stimulation

**FIG. 4A** shows an exemplary embodiment of the devices and methods described herein implanted in an individual. Onufs nucleus **138** (S2-S4) is the center of pelvic somatomotor integration which joins nerves and becomes the pudendal nerve **133.** In some cases, the implanted device **128** may target the pudendal nerve **133** by placing at least one sensor **134** and at least one electrode **136** and may target the cavernous nerve **135** by placing at least one sensor **130** and at least one electrode **132.** The sensors **130** and **134** may capture the bio-signal (A) to classify any sexual dysfunction events, and the stimulator electrodes **132** and **136** may deliver the bio-simulation, which is adapted from the base stimulation to account for the sexual dysfunction event, to act on the target muscle.

In some cases, providing electrical stimulation to the pudendal nerve instead of the sacral nerve may provide a greater precision as the pudendal nerve or branches thereof is lower than the sacral nerve and closer to the organs and tissues involved in sexual dysfunction than the sacral nerves. The sensors of the device capture the bio-signal to classify any erectile dysfunction event, and the stimulator electrodes of the device deliver the electrical stimulation, which is adapted from the base stimulation to account for the sexual dysfunction event, to act on one or more target muscles. Other embodiments of stimulation are described elsewhere in this disclosure.

### System

**FIG. 5** shows an exemplary embodiment of a system block diagram for the devices and methods described herein with the slowand fast-adapting algorithms. In some cases, the systems disclosed herein may comprise a plurality of sub-modules. In some instances, the sub-modules may comprise: an offline analysis module **152,** a clinician control module **154,** a patient controller module **156,** a pulse generator **160,** one or more electrode leads **510,** and one or more sensors **520** or any combination thereof. In some cases, the one or more electrode leads may comprise one or more stimulator and/or sensors. In some cases, the IPG 160 can be controlled by a remote or magnet **530** as described above. In some cases, the IPG 160 or other control modules can communicate with a wearable patch configured to detect a change in intracorporal pressure.

In some instances, the system can comprise an actuator or constrictor to compress the dorsal veins of the penis to assist erection. In some instances, the cuff and/or condom can be configured to compress the dorsal veins of the penis to assist erection.

In some cases, the offline analysis module **152** may comprise a data repository, an analysis software a visualization software, or any combination thereof. In some instances, the offline analysis module may be used to retrospectively analyze and graphically visualize an individual's implant performance to prevent episodes of sexual dysfunction. The off-line analysis module **152** may be programmatically coupled to the clinician control module through an application programming interface (API). In some instances, the clinician control module may comprise software that may tune or change the electrical stimulation patterns of the individual's electrical stimulation implant.

In some cases, the clinical control module **154** may comprise stimulation management and device monitor software, stimulation programming map, classification configuration dashboard, streaming data collection dashboard, or any combination thereof. In some cases, a health care personnel may assist an individual with an electrical implant by updating or modifying their electrical implant parameters through the clinical control module **154.** In some cases, the clinical control module may be utilized to initialize an individual's electrical implant after implantation through an USB interface to the patient controller module **156.**

In some cases, the patient controller module **156** may comprise a direct interface to control aspects of their electrical stimulator as described herein. In some instances, the patient controller module **156** may comprise: medical information and communication band (MICS) communication platform, manual electrical stimulator control, enable or disable algorithm functionality, algorithm patient alerts, an inductive or wired charger for the implantable pulse generator rechargeable battery, or any combination thereof.

The pulse generator **160** can be an implantable pulse generator or external pulse generator. In some cases, the patient controller module **156** may be configured to wirelessly and/or inductively charge the implantable pulse generator rechargeable battery with a recharger of the patient controller module **156.** In some cases, the patient controller module **156** may magnetically couple to the implantable pulse generator **160** from outside the subject's skin. In some cases, the magnetic coupling of the controller module **156** to the implantable pulse generator **160** may be made such that the coupling is ergonomic for the subject such that the subject may conduct him/herself as if the controller module **156** is not magnetically coupled to the implantable pulse generator **160.** In some cases, the patient controller module **156** may comprise a battery that may be recharged through wireless inductive charging via the recharger and/or wired charging. In some cases, the patient controller module **156** may comprise a rechargeable lithium-ion battery. In some instances, the recharger may comprise one or more inductive coils used when charging the patient controller module **156** and/or when using the patient controller module **156** to charge the implantable pulse generator **160.** In some cases, the patient controller module, shown as shown in **FIG. 7B** may be configured **722** to accept additional memory storage **723.** In some cases, the additional memory storage may be used to transport patient data and/or information between patient/subject and provider.

In some cases, the patient controller module **156** may directly or automatically control the implantable pulse generator **160.** In some cases, the implantable pulse generator **160** may comprise a corresponding MICS-telemetry communication platform to that of the MICS communication platform of the patient controller module, enabling the communication between the two devices over an ad hoc Wi-Fi or wireless network **158.** In some instances, the implantable pulse generator **160** may further comprise a three-axis accelerometer biopotential amplifier that may be electrically coupled to one or more electrodes leads **510.**

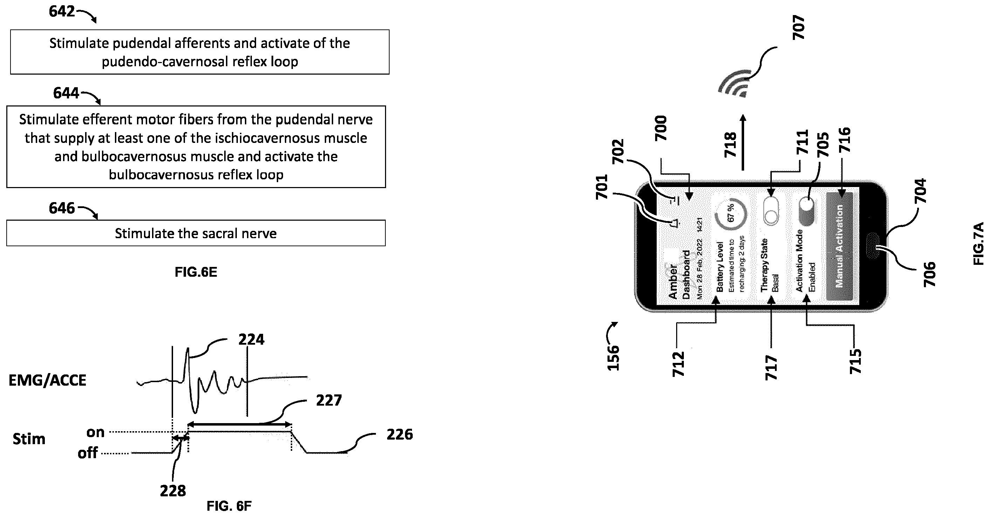

In some cases, the one or more electrode leads may comprise one or more stimulator and/or sensors. In some cases, a biopotential amplifier may be electrically coupled to a computation sub module comprising a classifier, control policy, real-time clock scheduler, microprocessor, or any combination thereof. In some instances, the biopotential amplifier, classifier, control policy, real-time clock scheduler, and microprocessor, or any combination thereof, may process and interpret detected myoelectric EMG signals in the patient to determine the necessary electrical stimulation pattern provided by the actuator to the one or more stimulator electrodes to prevent an episode of sexual dysfunction in an individual.
In some instances, the patient controller module **156** may comprise a user interface, ports, indicators, or any combination thereof as seen in **FIGS. 7A-7B****.** The user interface and/or ports of the patient controller module may comprise an input charging socket **704,** keypad navigation button **706,** stimulation indicator, communication indicator, output charging adapter port, battery level indicator in both percentage and number of days **712,** manual excitation over-ride button **716,** or any combination thereof. In some cases, the input charging socket may be configured to accept a USB A, B, and/or C, Firewire, any micro versions thereof, or any combinations thereof. connections. In some instances, the patient controller module **156** may be connected to a power converter to through a corresponding cable adapted to the input charging socket to charge the patient controller module **156.**

### User Interface

The patient controller module **156** may comprise a user interface **700** where the user interface may comprise one or more user interface objects **(701, 702, 705, 711, 712, 715, 716, 717, 721, 730, 723),** and/or views as seen in **FIG. 7A-7B****.** In some cases, the user interface **700** may comprise a touch screen display configured to receive touch or pressing input from a user, patient, and/or medical care personnel. In some cases, a user, patient, and/or medical care personnel may press and/or interact with button **716** based mixed graphic and text indicators, and/or switch mixed graphic and text indicators **(705, 711).** In some cases, the user, patient, and/or medical care personnel may double tap a user interface object to enable an emergency state. In some instances, the emergency state may enable the implanted stimulator to provide electrical stimulation immediately in response to the double tap command. In some cases, a parameter or setting value of the user interface objects may be modified and/or changed by tilting the patient controller module. In some cases, tilting the patient controller module in a first direction may increase the parameter and/or setting value of the user interface object whereas tilting the patient controller module in a second direction opposite the first direction may decrease the parameter and/or setting value.

In some cases, the user interface between devices such as smart phones and tablets or other personal computing device may comprise a scaled version of the user interface. In some cases, the different user interface views e.g., the view shown in **FIG. 7A** and **FIG. 7B** may display varying user interface objects. In some cases, the user interface objects may comprise one or more buttons **716,** switches **(711,716),** and/or graphical or image based representation of data **(721,730, 723).**

In some cases, users may customize the user interface object with a selection of one or more user interface objects (e.g., buttons, switch button to enable various device operation modes, graphical displays of device data, etc.). In some cases, the user views may be a predetermine set of views with set user interface object. In some instances, the user may customize and/or create one or more views accessible by a menu icon **702.** In some cases, the menu icon **702** may be configured to display one or more submenu options. In some instances, the one or more submenu options may comprise personal identification, account information, device registration, customer support, or any combination thereof submenus. In some cases, one submenu may comprise information of how to connect the device platform to pre-existing health care providers. In some cases, the user interface may comprise a notification object **701.** The notification object may display a unique or highlighted state if a particular notification of device performance, detection of an sexual dysfunction event, or any combination thereof is to be provided to the user of the device. A user may interact with a press the notification object to view, in the form of a pop-up dialogue, the particular notification.

In some cases, the one or more user interface objects may comprise text and/or mixed text and vector objects representations of the various API function calls and/or sub-user interface views, as seen in **FIGS. 7A-7B****.** In some cases, the user interface may comprise mixed text and vector objects that permit the subject or user to activate **705** or de-activate **711** electrical stimulation of the device **716,** adjust device parameters **715,** indicate therapy state **717,** view device measured EMG signals **721,** view stimulator electrode electrical signal characteristics (e.g., frequency, amplitude, pulse width, etc.) delivered, view a medical portal to submit user data to a health care provider, log resulting sexual dysfunction events **719** overlaid on top of measured ENG/EMG signals, or any combination thereof. In some cases, the user interface may further comprise a battery **712** and wireless communication connectivity indicator for the users and/or subjects to visualize patient controller module **156** operating properties.

In some cases, the keypad navigation button **706** may be configured to navigate between various user interface views e.g., the user interface views provided in **FIG. 7A** and **FIG. 7B**

In some instances, the patient controller module **156** may comprise visual indicators **(715, 717, 712),** configured to indicate whether the implanted electrical stimulator is outputting electrical stimulation and/or the presence or lack thereof connectivity with a second or third device. In some cases, the patient controller module may comprise a device adjustment parameter, where the device adjustment parameter may comprise a stimulation indicator, or an activation of a stimulation mode **715.** In some cases, the stimulation indicator may be in electrical communication with a processor, described elsewhere herein, configured to display a visual indicator when the stimulator is providing an electrical stimulation to a subject. In some instances, the patient controller module may comprise a connectivity indicator. In some cases, the connectivity indicator may be in electrical communication with a processor, described elsewhere herein, and configured to provide a visual indicator when the patient controller module is connected to one or more discrete devices, data servers, local WIFI or ad-hoc WIFI networks, Bluetooth, medical implant communication system (MICS), or any combination thereof. In some cases, the connectivity indicator may indicate the wireless connection with the implanted electrical stimulator. In some cases, the connectivity indicator may comprise one or more states. In some instances, a first state my comprise a solid image indicator, where such a solid image indicator may notify a user, subject, individual, and/or health care personnel, a successfully established communication pairing between the patient controller module and a third device, server, etc. A second state may comprise a flashing image indicator, where such a flashing image indicator indicates a paired communication state between the patient controller module and a third device, server, etc. In some cases, the image indicator may comprise the universal symbol for Bluetooth that may be observed on smart devices and/or devices with Bluetooth connectivity. In some cases, the image indicator may comprise a graphic of the universal symbol indicator for Wi-Fi (e.g., a quarter circle of concentric rings) seen commonly on smart devices and/or devices with Wi-Fi connectivity.

In some cases, device data (e.g., EMG/ENG, accelerometer, gyroscopic, magnetometer, 3-D spatial movement, global positioning system (GPS) data, or any combination thereof) may be transmitted **718** over Wi-Fi, Bluetooth, MICS, or other ad-hoc networks between one or more devices, as described elsewhere herein.

**FIG. 7B** shows a different user interface view than that of the **FIG. 7A****.** In some cases, the user interface of **FIG. 7B** may comprise one or more user interface objects **(721, 730, 723),** where each user interface object displays device data received **718** through wireless transmission **707** as described above. In some cases, one of the user interface objects may comprise a graphical therapy object **721.** The graphical therapy object may display detected EMG/ENG signals **726** and corresponding stimulation profiles **728.** In some cases, the graphical therapy object may display leak events **719** of where the user indicated an sexual dysfunction event but where the device did not provide stimulation. Another user interface object may comprise a GPS and motion activity object **730.** In some instances, the motion activity object **730** may display GPS and motion data of the subject over time. The GPS and motion data may be useful considerations when improving the classifier described elsewhere herein. Another user interface object may comprise a charging indicator object **723.** In some cases, the charging indicator object may display charge capacitance of the implanted stimulator over a period of time. In some cases, the charging indicator object may be used to monitor the health of the battery of the implanted stimulator. A user interacting with the display view shown in FIG. 7B may pinch, swipe, or otherwise interact with the data of each user interface object **(721, 730, 723)** to view other temporal regions of data or to zoom in on a particular scale of a measurement. In some cases, through the menu object **702** a user and/or subject may export their medical data to one or more provides.

### Method of Treatment

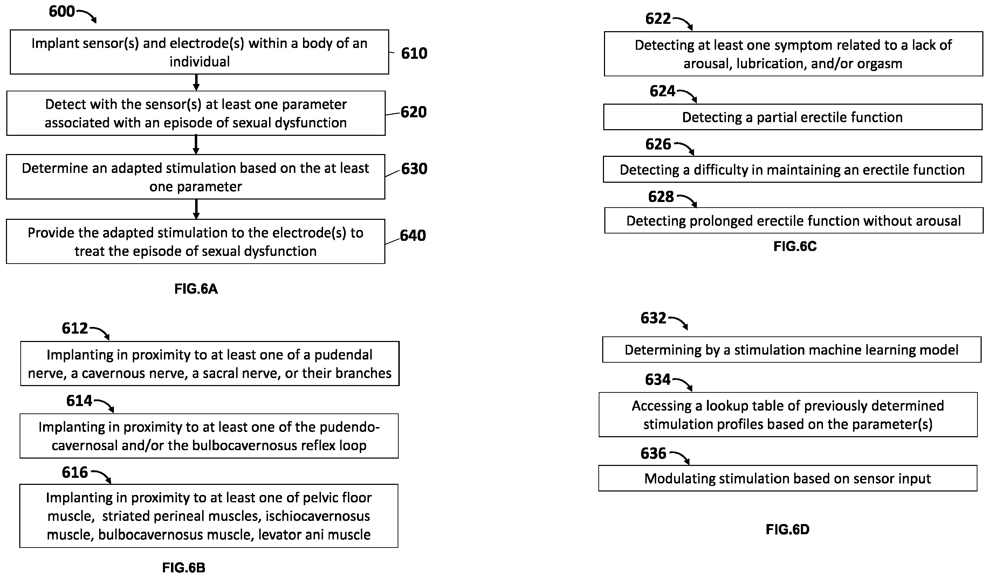

**FIG. 6A** illustrates a workflow of a method **600** for treating a symptom of sexual dysfunction in an individual. The method may comprise the steps of (a) implanting one or more sensors and one or more stimulator electrodes within a body of the individual **610;** (b) detecting with the sensor(s) at least one parameter associated with an episode of sexual dysfunction **620;** and (c) determining an adapted stimulation based on the at least one parameter **630;** providing the adapted stimulation to the implanted electrode(s) to treat the episode of sexual dysfunction **640.** Often, the methods described herein prevent the episode of insufficient penile or clitoral erection.

In some instances, step (a) of implanting one or more sensors and one or more stimulator electrodes within a body of the individual 610 can comprise implanting at the one or more anatomical sites unilaterally or bilaterally as mentioned above. In some instances, step (a) can comprise implanting in proximity to at least one of a pudendal nerve, a cavernous nerve, a splanchnic nerve, a sacral nerve, a pelvic plexus nerve, or any branches or combination thereof **(612);** implanting in proximity to at least one of the pudendo-cavernosal and/or the bulbocavernosus reflex loop **(614);** implanting in proximity to at least one of pelvic floor muscle, striated perineal muscles, ischiocavernosus muscle, bulbocavernosus muscle, levator ani muscle **(616).** In some instances, multiple implantation sites may be targeted including combinations of two electrode leads interfacing with different nerves including pudendal and cavernosous nerves, pudendal and sacral nerves, and cavernosous and sacral nerves, pudendal and splanchnic nerves, cavernosous and splanchnic nerves, and sacral and splanchnic nerves.

In some instances, step (b) detecting with the sensor(s) at least one parameter associated with an episode of sexual dysfunction **620** can comprise detecting at least one symptom related to a lack of arousal, lubrication, and/or orgasm **(622);** detecting a partial erection such as tumescence without rigidity **(624);** detecting a difficulty maintaining an erection **(626);** detecting prolonged erection in the absence of sexual desire **(628)** (see **FIG. 6C****).** In some instances, the step (b) can comprise detecting a response from the individual to prevent the sexual dysfunction event, to determine an initiation of stimulation to start sexual activity, and to determine a wanting of termination of the stimulation. In an aspect, the sensor and/or parameter is configured to detect a volitional and/or reflex activity of the individual experiencing an episode of sexual dysfunction.

In an example, a sensor could be configured to detect no penile or clitoral changes when the individual is aroused. In some instances, detecting a partial erection such as tumescence without rigidity comprises detecting a low intracorporal pressure as determined by a predetermined threshold. In an example, the individual can indicate to a sensor or the patient controller module that they are aroused whereby a parameter of the episode can be sensed. In an example, the individual can use a magnet by way of holding, waving, tapping, or using opposite poles of the magnet to indicate to control functions the IPG (i.e. they are experiencing an episode of sexual dysfunction). In an aspect, using a magnet would prevent from other types of tapping patterns that could get confused with volitional control of the device.

In some instances, step (c) of determining an adapted stimulation based on the at least one parameter can comprise system and methods **800** described above. In an aspect, providing the adapted stimulation comprises varying at least one of an intensity, a frequency, or a duration of the adapted stimulation according to the parameter detected in step (b). In some instances, step (c) of determining an adapted stimulation can be determined by a stimulation machine learning model as described above **(632);** accessing a lookup table of previously determined stimulation profiles based on the parameter **(634);** modulating stimulation based on sensor input **(636).** In an example, modulating stimulation based on sensor input can comprise interpreting a rhythmic pattern of rubbing, vibration, stroking and adjusting the adapted stimulation on a 1:1 or other multiple of the rhythmic pattern. In an aspect, the adapted stimulation is configured to augment a reflex activity of the individual experiencing an episode of sexual dysfunction including neurogenic and combined neurogenic/vascular etiology causes.

In some instances, step (d) of providing an adapted stimulation with the implanted electrode to treat the episode of sexual dysfunction **(640)** can comprise providing a sensory stimulation and a motor stimulation (see **FIG. 6E****).** In an example, the adapted stimulation is configured to providing a sensory stimulation by initiating penile or clitoral tumescence by stimulating penile or clitoral pudendal afferents and activating the pudendo-cavernosal reflex loop **(642).** In an example, the adapted stimulation is configured to provide a motor stimulation by inducing and sustaining rigidity of an erection by stimulating efferent motor fibers from the pudendal nerve that supply the ischiocavernosus muscle and bulbocavernosus muscle and activate the bulbocavernosus reflex loop **(644).** In an example, the adapted stimulation is configured to provide a sensory and/or motor stimulation by initiating penile or clitoral tumescence and/or by sustain rigidity of erection by stimulating the sacral nerve **(646).** In an aspect, pudendal stimulation can result in somatic nerve stimulation both for sensory and/or motor effects and the cavernosus nerve for autonomic function.

In some cases, the insufficient penile or clitoral erection may comprise at least one of insufficient penile or clitoral erection.

In some cases, the method may comprise a step of providing a constant electrical stimulation at a lower intensity level than the electrical stimulation provided in step (c). The constant electrical stimulation provided at a lower intensity may assist individual's suffering from sexual dysfunction. In some instances, the intensity or duration of the electrical stimulation provided in step (c) may vary according to the response that is sensed in step (b). In some cases, the time period between sensing a parameter **224** and providing an electrical stimulation **226** may be described as a response time **228,** as shown in **FIG. 6F****.** In some cases, the stimulation may be provided for a duration of stimulation **227.**

In some cases, the duration of stimulation **227** may comprise about 1 second to about 30 seconds. In some cases, the duration of stimulation **227** may comprise about 1 second to about 2 seconds, about 1 second to about 3 seconds, about 1 second to about 4 seconds, about 1 second to about 5 seconds, about 1 second to about 10 seconds, about 1 second to about 12 seconds, about 1 second to about 14 seconds, about 1 second to about 16 seconds, about 1 second to about 20 seconds, about 1 second to about 25 seconds, about 1 second to about 30 seconds, about 2 seconds to about 3 seconds, about 2 seconds to about 4 seconds, about 2 seconds to about 5 seconds, about 2 seconds to about 10 seconds, about 2 seconds to about 12 seconds, about 2 seconds to about 14 seconds, about 2 seconds to about 16 seconds, about 2 seconds to about 20 seconds, about 2 seconds to about 25 seconds, about 2 seconds to about 30 seconds, about 3 seconds to about 4 seconds, about 3 seconds to about 5 seconds, about 3 seconds to about 10 seconds, about 3 seconds to about 12 seconds, about 3 seconds to about 14 seconds, about 3 seconds to about 16 seconds, about 3 seconds to about 20 seconds, about 3 seconds to about 25 seconds, about 3 seconds to about 30 seconds, about 4 seconds to about 5 seconds, about 4 seconds to about 10 seconds, about 4 seconds to about 12 seconds, about 4 seconds to about 14 seconds, about 4 seconds to about 16 seconds, about 4 seconds to about 20 seconds, about 4 seconds to about 25 seconds, about 4 seconds to about 30 seconds, about 5 seconds to about 10 seconds, about 5 seconds to about 12 seconds, about 5 seconds to about 14 seconds, about 5 seconds to about 16 seconds, about 5 seconds to about 20 seconds, about 5 seconds to about 25 seconds, about 5 seconds to about 30 seconds, about 10 seconds to about 12 seconds, about 10 seconds to about 14 seconds, about 10 seconds to about 16 seconds, about 10 seconds to about 20 seconds, about 10 seconds to about 25 seconds, about 10 seconds to about 30 seconds, about 12 seconds to about 14 seconds, about 12 seconds to about 16 seconds, about 12 seconds to about 20 seconds, about 12 seconds to about 25 seconds, about 12 seconds to about 30 seconds, about 14 seconds to about 16 seconds, about 14 seconds to about 20 seconds, about 14 seconds to about 25 seconds, about 14 seconds to about 30 seconds, about 16 seconds to about 20 seconds, about 16 seconds to about 25 seconds, about 16 seconds to about 30 seconds, about 20 seconds to about 25 seconds, about 20 seconds to about 30 seconds, or about 25 seconds to about 30 seconds. In some cases, the duration of stimulation **227** may comprise about 1 second, about 2 seconds, about 3 seconds, about 4 seconds, about 5 seconds, about 10 seconds, about 12 seconds, about 14 seconds, about 16 seconds, about 20 seconds, about 25 seconds, or about 30 seconds. In some cases, the duration of stimulation **227** may comprise at least about 1 second, about 2 seconds, about 3 seconds, about 4 seconds, about 5 seconds, about 10 seconds, about 12 seconds, about 14 seconds, about 16 seconds, about 20 seconds, or about 25 seconds. In some cases, the duration of stimulation **227** may comprise at most about 2 seconds, about 3 seconds, about 4 seconds, about 5 seconds, about 10 seconds, about 12 seconds, about 14 seconds, about 16 seconds, about 20 seconds, about 25 seconds, or about 30 seconds.

In some cases, the response time **228** may comprise about 60 µs to about 100 µs. In some cases, the response time **228** may comprise about 60 µs to about 65 µs, about 60 µs to about 70 µs, about 60 µs to about 75 µs, about 60 µs to about 80 µs, about 60 µs to about 85 µs, about 60 µs to about 90 µs, about 60 µs to about 95 µs, about 60 µs to about 100 µs, about 65 µs to about 70 µs, about 65 µs to about 75 µs, about 65 µs to about 80 µs, about 65 µs to about 85 µs, about 65 µs to about 90 µs, about 65 µs to about 95 µs, about 65 µs to about 100 µs, about 70 µs to about 75 µs, about 70 µs to about 80 µs, about 70 µs to about 85 µs, about 70 µs to about 90 µs, about 70 µs to about 95 µs, about 70 µs to about 100 µs, about 75 µs to about 80 µs, about 75 µs to about 85 µs, about 75 µs to about 90 µs, about 75 µs to about 95 µs, about 75 µs to about 100 µs, about 80 µs to about 85 µs, about 80 µs to about 90 µs, about 80 µs to about 95 µs, about 80 µs to about 100 µs, about 85 µs to about 90 µs, about 85 µs to about 95 µs, about 85 µs to about 100 µs, about 90 µs to about 95 µs, about 90 µs to about 100 µs, or about 95 µs to about 100 µs.In some cases, the response time **228** may comprise about 60 µs, about 65 µs, about 70 µs, about 75 µs, about 80 µs, about 85 µs, about 90 µs, about 95 µs, or about 100 µs. In some cases, the response time 228 may comprise at least about 60 µs, about 65 µs, about 70 µs, about 75 µs, about 80 µs, about 85 µs, about 90 µs, or about 95 µs. In some cases, the response time **228** may comprise at most about 65 µs, about 70 µs, about 75 µs, about 80 µs, about 85 µs, about 90 µs, about 95 µs, or about 100 µs. Through the development of iteratively trained machine learning classifiers the response time may be minimized, and treatment of sexual dysfunction may be realized.

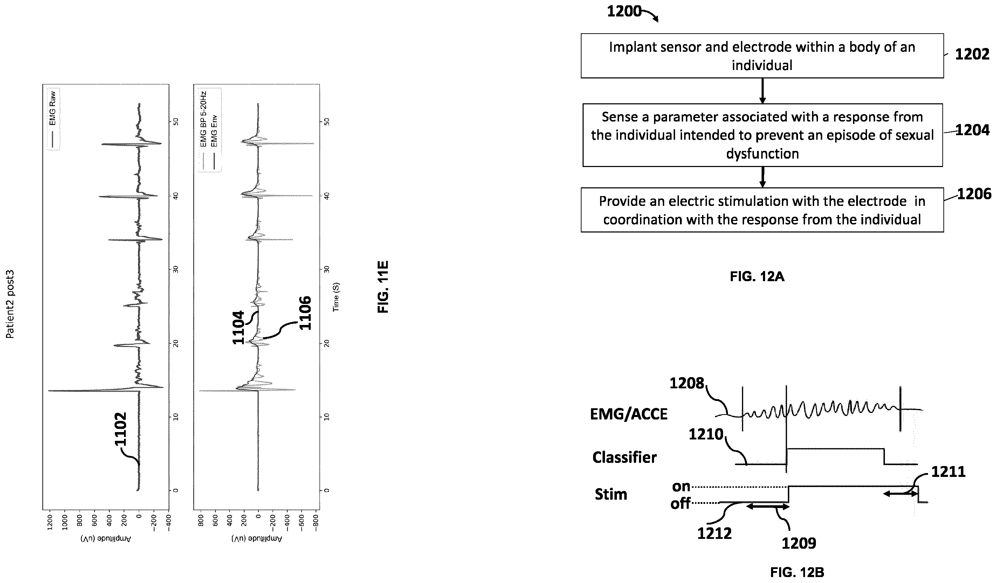

**FIGS. 12A-12B** illustrate a workflow of a method **1200** for treatment of sexual dysfunction in an individual. The method may comprise the steps of (a) implanting a sensor and stimulator electrode within a body of an individual **1202;** (b) sensing a parameter associated with a response from the individual for preventing an episode of sexual dysfunction, where the response is user induced stimulus **1204;** and (c) providing an electric stimulation with the stimulator electrode in coordination with the response from the individual to prevent the sexual dysfunction episode **1206.** In some cases, the user induced stimulus may be intended to prevent an episode of sexual dysfunction. Additional steps and examples of a method for treating a symptom of sexual dysfunction in an individual are described in method **600** relation to **FIG. 6A** which is incorporated herein.

As shown graphically in **FIG. 12B****,** a detected EMG signal **1208** may be analyzed by a classifier **1210,** described elsewhere herein, to determine when the EMG, ENG, accelerometer, gyroscope, magnetometer, pressure sensor signals or any combination thereof signals have crossed a pre-determined threshold, described elsewhere herein. Once the classifier **1210** has determined that the signal **1208** does represent a sexual dysfunction event, the processor, described elsewhere herein, may enable stimulation **1212** to treat the symptom of sexual dysfunction. In some cases, stimulation **1212** may comprise an extension of stimulation **1211** that may extend beyond the time the classifier **1210** determines there to be a sexual dysfunction event. In some cases, the extension of stimulation **1211** may comprise a duration of time equal to the duration the subject continues to purposefully or with intent produce a muscle contraction.

In some cases, the extension of stimulation **1211** may comprise about 1 s to about 30 s. In some cases the extension of stimulation **1211** may comprise about 1 s to about 3 s, about 1 s to about 5 s, about 1 s to about 8 s, about 1 s to about 10 s, about 1 s to about 12 s, about 1 s to about 15 s, about 1 s to about 18 s, about 1 s to about 20 s, about 1 s to about 22 s, about 1 s to about 24 s, about 1 s to about 30 s, about 3 s to about 5 s, about 3 s to about 8 s, about 3 s to about 10 s, about 3 s to about 12 s, about 3 s to about 15 s, about 3 s to about 18 s, about 3 s to about 20 s, about 3 s to about 22 s, about 3 s to about 24 s, about 3 s to about 30 s, about 5 s to about 8 s, about 5 s to about 10 s, about 5 s to about 12 s, about 5 s to about 15 s, about 5 s to about 18 s, about 5 s to about 20 s, about 5 s to about 22 s, about 5 s to about 24 s, about 5 s to about 30 s, about 8 s to about 10 s, about 8 s to about 12 s, about 8 s to about 15 s, about 8 s to about 18 s, about 8 s to about 20 s, about 8 s to about 22 s, about 8 s to about 24 s, about 8 s to about 30 s, about 10 s to about 12 s, about 10 s to about 15 s, about 10 s to about 18 s, about 10 s to about 20 s, about 10 s to about 22 s, about 10 s to about 24 s, about 10 s to about 30 s, about 12 s to about 15 s, about 12 s to about 18 s, about 12 s to about 20 s, about 12 s to about 22 s, about 12 s to about 24 s, about 12 s to about 30 s, about 15 s to about 18 s, about 15 s to about 20 s, about 15 s to about 22 s, about 15 s to about 24 s, about 15 s to about 30 s, about 18 s to about 20 s, about 18 s to about 22 s, about 18 s to about 24 s, about 18 s to about 30 s, about 20 s to about 22 s, about 20 s to about 24 s, about 20 s to about 30 s, about 22 s to about 24 s, about 22 s to about 30 s, or about 24 s to about 30 s. In some cases, the extension of stimulation **1211** may comprise about 1 s, about 3 s, about 5 s, about 8 s, about 10 s, about 12 s, about 15 s, about 18 s, about 20 s, about 22 s, about 24 s, or about 30 s. In some cases, the extension of stimulation **1211** may comprise at least about 1 s, about 3 s, about 5 s, about 8 s, about 10 s, about 12 s, about 15 s, about 18 s, about 20 s, about 22 s, or about 24 s. In some cases, the extension of stimulation **1211** may comprise at most about 3 s, about 5 s, about 8 s, about 10 s, about 12 s, about 15 s, about 18 s, about 20 s, about 22 s, about 24 s, or about 30 s.

In some instances, there may be a delay **1209** between the start of sexual dysfunction event in the raw EMG, ENG, accelerometer, gyroscope, magnetometer, pressure sensor, or any combination thereof signal data, and the onset of the electrical stimulation. Upon training the classifier **1210** on sufficiently large and varied datasets, such delay may be minimized further improving the device performance in prevent sexual dysfunction events. In some cases, sensing may comprise determining a global positioning system (GPS) location of the individual that, in combination with the parameter associated with the response from the individual, is used to treat the episode of sexual dysfunction.

Aspects of the disclosure provided herein may comprise a method of data processing. In some cases, the method of data processing may comprise: (i) receiving a measurement of a parameter previously measured by a sensor, which parameter is predictive of an episode of sexual dysfunction in an individual; (ii) analyzing the parameter; and (iii) synthesizing an electrical stimulation signal for the individual, such that when the electrical stimulation signal is provided by a stimulator electrode to the individual, the electrical stimulation signal, together with an effort from the individual that is intended to prevent an episode of sexual dysfunction, treats the episode of sexual dysfunction. In some instances, the parameter may be associated with a response from the individual intended to overcome sexual dysfunction. In some instances, the electrical stimulation signal is synthesized or modified as to supplement the individual's effort with an electrical stimulation pattern that will, together with the effort from the individual, be sufficient to treat the episode of sexual dysfunction. In some cases, the response from the individual may be insufficient on its own to prevent the episode of sexual dysfunction and the electrical stimulation signal is such that, when applied, it adds enough, together with the response, to treat the episode of sexual dysfunction.

In some instances, the parameter may comprise a signal from a sensor that is configured to sense a contraction of a muscle of the individual related to an erection of the erectile tissue. In some cases, the electrical stimulation signal may comprise an electrical stimulation of the pudendal nerve. In some instances, the electrical stimulation signal may be synthesized to comprise a base electrical stimulation component and a measurement parameter specific component. In some instances, an intensity or duration of the electrical stimulation that is provided by the electrical stimulation signal may vary according to the value of the parameter that is received. In some instances, the parameter may comprise an EMG signal. In some cases, the EMG signal may determine that a contraction of at least one pelvic muscle has occurred. In some cases, a strength of the EMG signal may be proportional to the strength of the contraction of at least one pelvic muscle. In some instances, the electrical stimulation signal may comprise a first and second signals for the stimulation of a first pudendal nerve and a second pudendal nerve respectively. In some cases, the method may further comprise recording the signal previously measured by the sensor. In some cases, the method may further comprise synthesizing an adjustment signal to adjust the sensor in response to the recorded signal.

### Ambulatory Assessments After Implantation

In some embodiments, various ambulatory assessments may be taken to determine the effectiveness of the implantation procedure. In some embodiments, the implantable pulse generator IPG permits telemetric downloading of data used for determining the adapted stimulation (inputs, outputs, event classification, prior settings). In some embodiments, the participant may be in an awake ambulatory setting and a series of resting and provoked electrophysiological data may be recorded. In some embodiments, at treatment initiation (24-48 hours post-implant) sensory and motor responses may be determined from the different sensors on the implanted leads. In some embodiments, based upon the responses, the electrodes with the most adequate response may be selected to initiate treatment.

In some embodiments, the patients may be subjected to different physiological events to program the implantable pulse generator. In an example, the results of this programming can be used in determining the adapted stimulation. In some embodiments, these events may comprise coughing, Valsalva maneuvers, picking up a 5kg weight, or any combination thereof. In some embodiments, intracorporal pressures may be measured. In some embodiments, 1 hour continuous 'resting' recording of inputs and outputs (downloaded by telemetry) may be taken. In some embodiments, recording during controlled participant provoked events, such as coughing, Valsalva, lifting 5 Kg weight, may be obtained. In some embodiments, recording during pelvic muscle EMG to correlate inputs from lead vs. surface EMG may be obtained. In some embodiments, patient tolerances of basal stimulation ramping, and actuation parameters may be obtained.

In some cases, a change in penile blood flow may be measured and/or quantified after implantation of the devices and/or systems, described elsewhere herein, to determine a decrease in sexual dysfunction after implantation compared to sexual dysfunction measured prior to implantation. Changes in penile blood flow can be measured with doppler ultrasound, where the doppler ultrasound provides a parameter of penile tumescence. In some cases, a rigidity of an erect penis of an individual may be measured and/or quantified after implantation of the devices and/or systems, described elsewhere herein, to determine a decrease in sexual dysfunction after implantation compared to sexual dysfunction measured prior to implantation. In some cases, a decrease in sexual dysfunction may be determined, observed, and/or quantified by measuring penile circumference and rigidity. In some cases, penile rigidity is measured by providing a force (e.g., radial pressure) to the erect penile tissue and measuring a response of the penile tissue to the applied force. In some cases penile circumference is measured by a flexible measuring tape .

### Definitions

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining", "measuring", "evaluating", "assessing," "assaying," and "analyzing" are often used interchangeably herein to refer to forms of measurement and include determining if an element is present or not (for example, detection). These terms can include quantitative, qualitative, or quantitative and qualitative determinations. Assessing is alternatively relative or absolute. "Detecting the presence of" includes determining the amount of something present, as well as determining whether it is present or absent.

The terms "subject," "individual," or "patient" are often used interchangeably herein. A "subject" can be a biological entity containing expressed genetic materials. The biological entity can be an animal. The subject can be a mammal. The mammal can be a human. The subject may be diagnosed or suspected of being at high risk for a disease or a condition. The condition can be erectile dysfunction.

The term *"in vivo"* is used to describe an event that takes place in a subject's body.

The term *"ex vivo"* is used to describe an event that takes place outside of a subject's body. An *"ex vivo"* assay is not performed on a subject. Rather, it is performed upon a sample separate from a subject. An example of an *"ex vivo"* assay performed on a sample is an "in vitro" assay.

As used herein, the term 'about' a number refers to that number plus or minus 10% of that number. The term 'about' a range refers to that range minus 10% of its lowest value and plus 10% of its greatest value.

As used herein, the terms "treatment" or "treating" are used in reference to an intervention regimen for obtaining beneficial or desired results in the recipient. Beneficial or desired results include but are not limited to a therapeutic benefit and/or a prophylactic benefit. A therapeutic benefit may refer to prevention or amelioration of symptoms or of an underlying disorder being treated. Also, a therapeutic benefit can be achieved with prevention or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or a condition, delaying or eliminating the onset of symptoms of a disease or a condition, slowing, halting, or reversing the progression of a disease or a condition, or any combination thereof. For prophylactic benefit, a subject at risk of developing a particular disease or a condition, or to a subject reporting one or more of the physiological symptoms of a disease or a condition may undergo treatment.

As used herein, the term "muscle" refers to a "myocyte", "muscle cell", "muscle fiber", "muscle tissue", or any combination thereof.

As used herein, the terms "base electrical stimulation" and "basal electrical stimulation" are used interchangeably.

As used herein, the term "sensor" comprises a detection apparatus that is attached to an implanted lead, including an "electrode", "sensor electrode", "sensory electrode", or any combination thereof. In some embodiments, the sensor, as used herein, detects one or more parameters associated with pain in the individual.

As used herein, the term "electrode lead" or "lead" comprises an implantable lead of an electrode that has at least one of a sensing or stimulating component, including a "stimulator", "stimulator electrode," "sensor," "sensor electrode," or any combination thereof. In some embodiments, the electrode, as used herein, provides an electrical signal to the target site in the individual.

As used herein, the terms "signal" and "bio-signal" are used interchangeably.

The section headings used herein are for organizational purposes only and are not to be construed as limiting the subject matter described.

The section headings used herein are for organizational purposes only and are not to be construed as limiting the subject matter described.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EXAMPLES

### Example 1: A system lacking a closed-loop configuration

**FIG. 1** shows an exemplary embodiment of an open-loop bioelectronic system comprising an implantable pulse generator. The system delivers a predefined stimulation protocol and does not receive input from the subject, resulting in inadequate treatment of sexual dysfunction.

### Example 2: EMG Measurement Reproducibility

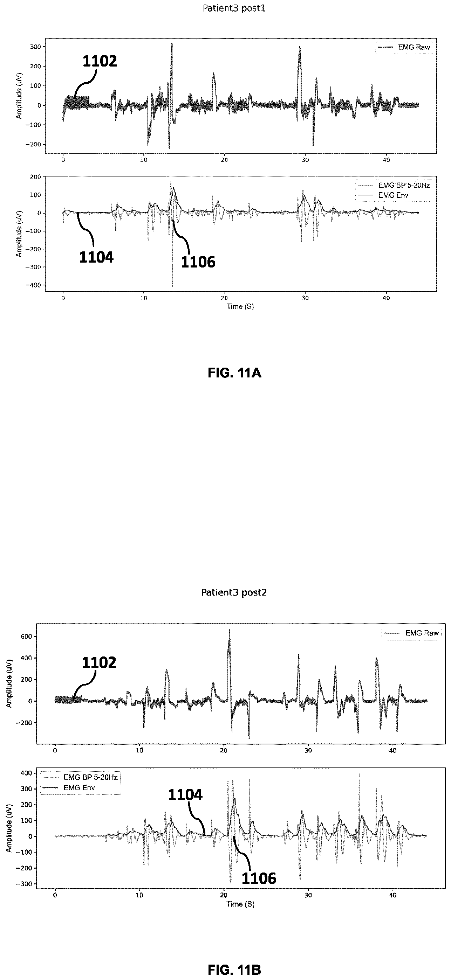

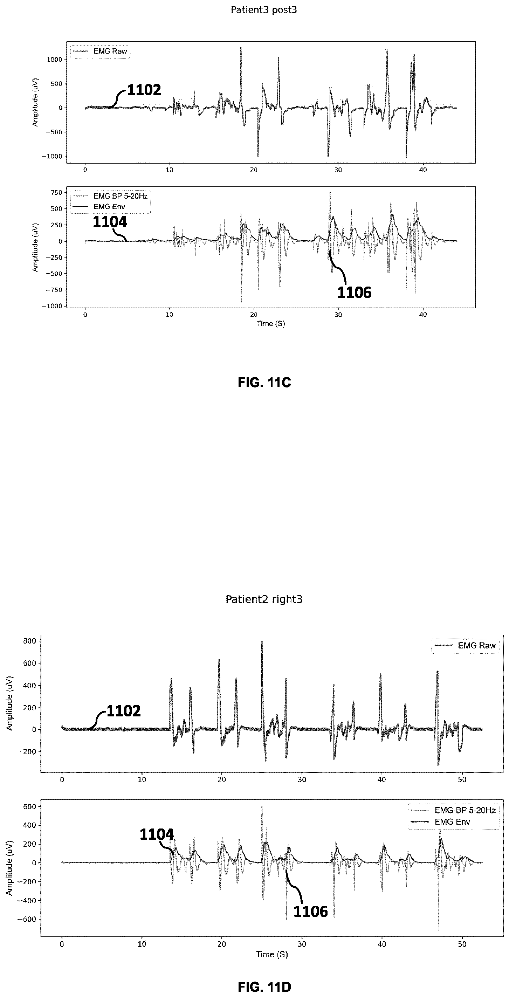

Using the systems, methods, and devices, described herein, patient EMG signals were measured and processed as patients performed muscle contractions, coughing, and Valsalva maneuvers, as can be seen in **FIGS. 11A-11E****.** For each patient, raw EMG data **1102** was recorded and amplified, as described elsewhere herein. Subsequently, the raw EMG data was filtered **1106,** rectified, and smoothed **1104.**

From the data shown in FIGS. **11A-11C****,** where a single patient iteratively performed the same sequence of muscle contractions three times, a distinct group of three signals may be observed in the temporal EMG data. Such a finding supports the reproducibility of the sensors of the devices and systems described herein and the potential ability of a trained classifier to distinguish EMG signals temporally. Similarly, data for a different patient shown in **FIGS. 11D-11E** shows similar results as the patient in **FIGS. 11A-11C****.**

In an example, a first electrical stimulation of 35 Hz +/- 5 Hz, 0.2ms and 4.3+/-1.0V is applied to the pudendal nerve unilaterally and a second electrical stimulation of 35 Hz +/- 5 Hz, 0.2ms and 4.3+/-1.0V is applied to the cavernous nerve unilaterally which is configured to increase an arousal state and intracorporal pressure.

### Example 3: Complete Erectile Dysfunction (ED)

Provided herein is an example of a complete ED, where there is no penile change when the individual is aroused. The patient may actuate initiation of stimulation using the devices, systems, and methods provided herein when aroused. In some cases, the stimulation location may be one or more of pudendal nerve, cavernous nerve, or a combination thereof. The patient may activate the stimulation by the device manually, activate by a tapping sequence on an actuating portion of the device, or through sensing pelvic floor EMG (e.g., a sequence of voluntary contractions of the pelvic floor). In some cases, the tapping sequence is an unusual sequence such as a double tap to reduce risk of accidently initiating stimulation.

The patient may sustain the erection state by sequence of taps change stimulation paradigm. In some cases, the change in paradigm in patients with single nerve stimulation results in going from single nerve to two nerve stimulation. In some cases, in patients already with combined stimulation, the change in paradigm results in changing the parameters of one or both stimulation locations, or changing the predominance in stimulation.

The patient may cease the erection. In some cases, the patient may manually switch stimulation with a remote. In some cases, the patient may actuate by specific sequence of taps. In some cases, the device may automatically cease the stimulation by sensing specific pattern of pelvic floor EMG patterns as orgasm/ejaculation is accompanied by rhythmic pelvic floor contractions.

### Example 4: Partial Erectile Dysfunction (ED)

Provided herein is an example of a partial ED, where there is tumescence but insufficient rigidity when the individual is aroused. The patient may actuate initiation of stimulation using the devices, systems, and methods provided herein when aroused. In some cases, the stimulation location may be one or more of pudendal nerve, cavernous nerve, or a combination thereof. The patient may activate the stimulation by the device manually, activate by a tapping sequence on an actuating portion of the device, or through sensing pelvic floor EMG (e.g., a sequence of voluntary contractions of the pelvic floor). In some cases, the tapping sequence is an unusual sequence such as a double tap to reduce risk of accidently initiating stimulation.

The patient may sustain the erection state by sequence of taps change stimulation paradigm. In some cases, the change in paradigm in patients with single nerve stimulation results in going from single nerve to two nerve stimulation. In some cases, in patients already with combined stimulation, the change in paradigm results in changing the parameters of one or both stimulation locations, or changing the predominance in stimulation.
The patient may cease the erection. In some cases, the patient may manually switch stimulation with a remote. In some cases, the patient may actuate by specific sequence of taps. In some cases, the device may automatically cease the stimulation by sensing specific pattern of pelvic floor EMG patterns as orgasm/ejaculation is accompanied by rhythmic pelvic floor contractions.

### Example 5: Networked Nerve Treatment of Clinically Significant Erectile Dysfunction

The systems, methods, and/or devices, described elsewhere herein, may be used to treat an individual's clinically significant erectile dysfunction. To treat the clinically significant erectile dysfunction, a first electrode one or more electrode leads (e.g., one or more stimulation or sensing electrodes), described elsewhere herein, is implanted on the individual's cavernous nerve and a second electrode of the one or more electrodes is implanted on the pudendal nerve. A pulse generator is implanted in the individual and electrically coupled to the one or more electrode leads. The implanted device is then configured by medical care personnel (e.g., an attending physician, nurse, or operating theater room medical support staff) based at least on the individual's clinically significant erectile dysfunction.

After a period of time subsequent to implantation of the one or more electrodes and stimulator, the individual's erectile dysfunction is analyzed with one or more sensors, tests, and/or questionnaires, as described elsewhere herein. For example, an increase to the individual's EHS, SHIM, and/or IIEF score and/or result, as described elsewhere herein, from a time prior to receiving the implant to a time after receiving the implanted device and treatment provided by the device is recorded as a clinically significant improvement in the individual's ED. The individual's erectile dysfunction is also compared pre and post implantation with a tumescence sensor (e.g., a cuff placed around the penis) comprising a pressure sensor, temperature sensor, blood flow sensor, stretch sensor, or any combination thereof. In some cases, the tumescence sensor detects one or more parameters associated with a change in penile length, girth, and/or rigidity.

### Example 6: Radical Prostate Surgery Proactive Sexual Dysfunction Treatment

The systems, methods, and/or devices, described elsewhere herein, are used to treat an individual's sexual dysfunction that may arise from surgical intervention in the individual's pelvic cavity. For example, individuals undergoing radical (e.g., robotic) prostate surgery for prostate cancer receive one or more implanted electrodes during the prostate surgery to treat potential future sexual dysfunction (e.g., erectile dysfunction) as a result of the prostate surgery. The one or more implanted electrodes are implanted unilaterally or bilaterally over the neurovascular bundles on the individual's pelvic wall, near the urethra, after the individual's prostate is removed and before ureterovesical anastomosis. The one or more electrodes are routed through the individual's pelvic side wall to the individual's buttock region, where the one or more electrodes may be connected to a stimulator at a point in time after implanting the one or more electrodes based at least on clinical need. The position of the neurovascular bundles where the one or more electrodes are implanted may be determined intra-operatively by electrophysiological mapping in the urethra-prostatic junction. In some cases, a surgeon's decision to leave the one or more electrodes in situ may be determined by observations of tumescence response, as described elsewhere herein, during electrophysiological mapping. In some instances, the target nerves that the one or more electrodes are implanted at or adjacent to are nerves that are intentionally or unintentionally divided during surgery.

After a period of time subsequent to the prostatectomy and one or more electrode lead and optional stimulator implantation, the subject may present with erectile dysfunction. "A device may then be implanted and configured" The implanted device may then be configured by medical care personnel (e.g., an attending physician, nurse, or operating theater room medical support staff) based at least on the individual's erectile dysfunction symptoms, to treat the individual's erectile dysfunction.

After a period of time subsequent to implantation of the one or more electrodes and stimulator, the individual's erectile dysfunction is analyzed with one or more sensors, tests, and/or questionnaires, as described elsewhere herein. For example, an increase to the individual's EHS, SHIM, and/or IIEF score and/or result, as described elsewhere herein, from a time prior to receiving the implant to a time after receiving the implanted device and treatment provided by the device is recorded as a clinically significant improvement in the individual's ED. The individual's erectile dysfunction is also compared pre and post implantation with a tumescence sensor (e.g., a cuff placed around the penis) comprising a pressure sensor, temperature sensor, blood flow sensor, stretch sensor, or any combination thereof. In some cases, the tumescence sensor detects one or more parameters associated with a change in penile length, girth, and/or rigidity.

### Example 7: Radical Rectal Cancer Surgery Proactive Sexual Dysfunction Treatment

The systems, methods, and/or devices, described elsewhere herein, are used to treat an individual's sexual dysfunction that may arise from surgical intervention in the individual's pelvic cavity. For example, individuals undergoing radical rectal cancer surgery, e.g., abdominoperineal excision of the rectum and/or pelvic exenteration, receive one or more implanted electrodes during the rectal cancer surgery to treat potential future sexual dysfunction (e.g., erectile dysfunction) as a result of the rectal cancer surgery. The one or more implanted electrodes are implanted at or adjacent to surgically exposed neurovascular structures in the individual's pelvic side wall or presacral region. The neurovascular structures may comprise pudendal nerve(s), sacral nerve(s), and/or pelvic autonomic nerve(s). The one or more electrodes are routed through the individual's pelvic side wall or through sacral foramina to the individual's buttock region, where the one or more electrodes are connected to may be connected to a stimulator at a point in time after implanting the one or more electrodes based at least on clinical need. In some instances, the target nerves that the one or more electrodes are implanted at or adjacent to are nerves that are intentionally or unintentionally divided during surgery.

After a period of time subsequent to the rectal cancer surgery and one or more electrode lead and optional stimulator implantation, the subject may present with erectile dysfunction. The implanted device may then be configured by medical care personnel (e.g., an attending physician, nurse, or operating theater room medical support staff) based at least on the individual's erectile dysfunction symptoms, to treat the individual's erectile dysfunction.

After a period of time subsequent to implantation of the one or more electrodes and stimulator, the individual's erectile dysfunction is analyzed with one or more sensors, tests, and/or questionnaires, as described elsewhere herein. For example, an increase to the individual's EHS, SHIM, and/or IIEF score and/or result, as described elsewhere herein, from a time prior to receiving the implant to a time after receiving the implanted device and treatment provided by the device is recorded as a clinically significant improvement in the individual's ED. The individual's erectile dysfunction is also compared pre and post implantation with a tumescence sensor (e.g., a cuff placed around the penis) comprising a pressure sensor, temperature sensor, blood flow sensor, stretch sensor, or any combination thereof. In some cases, the tumescence sensor detects one or more parameters associated with a change in penile length, girth, and/or rigidity. In some cases, the implantation of the one or more electrodes and stimulator during rectal cancer surgery may treat emergent bladder symptoms.

## Claims

1. A system for treating an episode of sexual dysfunction of an individual, the system comprising:
(a) a sensor (120) implanted in a pelvic region of the individual configured to sense a parameter associated with the episode of sexual dysfunction of the individual;
(b) a stimulator electrode (122) implanted in a pelvic region of the individual configured to provide an adapted electrical stimulation;
(c) a processor operably coupled to the stimulator electrode and the sensor; and
(d) a non-transitory computer readable storage medium comprising a software configured to cause the processor to:
(i) receive from the sensor a measurement of the parameter associated with the episode of sexual dysfunction;
(ii) analyze the measurement to determine at least one of an intensity, a frequency, or a duration of the adapted electrical stimulation; and
(iii) in response to receiving the measurement or a manual tap on an actuating portion of the system, cause the stimulator electrode (122) to provide the adapted electrical stimulation, wherein the adapted electrical stimulation provided is sufficient to treat the episode of sexual dysfunction.

2. The system of Claim 1, wherein the stimulator electrode is configured to be implanted to interface with a pudendal nerve.

3. The system of Claim 2, further comprising a second stimulator electrode configured to be implanted to interface with a cavernosous nerve.

4. The system of any one of the preceding claims, wherein the sensor is a tumescence sensor configured to detect one or more parameters associated with a change in penile length, girth, and/or rigidity.

5. The system of Claim 4, wherein the tumescence sensor comprises a pressure sensor, a temperature sensor, a blood flow sensor, a stretch sensor, or a combination thereof.

6. The system of any one of the preceding claims, wherein the adapted electrical stimulation is configured to vary over arousal or erectile states.

7. The system of any one of the preceding claims, wherein the adapted electrical stimulation is sufficient to result in tissue engorgement, wherein said tissue comprises penile or clitoral tissue.

8. The system of any one of the preceding claims, wherein the adapted electrical stimulation works synergistically with a medication to treat sexual dysfunction.

9. The system of any one of the preceding claims, wherein the parameter has diurnal variation.

10. The system of any one of the preceding claims, wherein the parameter comprises a temporal or circadian variation.

11. The system of any one of the preceding claims, further comprising an implantable pulse generator comprising the actuating portion.

12. The system of any one of the preceding claims, wherein the software is configured to cause the processor to: analyze the measurement to determine the adapted stimulation for starting a sexual activity, changing a stimulation paradigm during the sexual activity, and terminating the sexual activity.

13. The system of any one of the preceding claims, wherein the adapted electrical stimulation is configured to vary over time.

14. The system of any one of the preceding claims, wherein the parameter comprises a first parameter and a second later in time than the first parameter, and wherein the adapted stimulation is sustained, modified, or cancelled based on the second parameter.

15. A method of data processing, the method comprising:
(a) receiving a measurement of a parameter associated with an episode of sexual dysfunction of an individual measured by an implanted sensor in a pelvic region of the individual;
(b) analyzing the parameter; and
(c) in response to receiving the measurement or a manual tap on an actuating portion of the system, generating an electrical stimulation pattern configured to treat the episode sexual dysfunction of the individual when the stimulation pattern is performed by an implanted electrode in the pelvic region of the individual, wherein at least one of an intensity, a frequency, or a duration of the stimulation pattern varies according to the parameter that is measured.

16. The method of claim 15, further comprising using software configured to generate the electrical stimulation pattern based on the parameter, wherein the software comprises a machine learning model, and wherein the machine learning model is configured to classify the measurement received by the implanted sensor and generate the stimulation pattern.

## Patentansprüche

1. System zur Behandlung einer Episode sexueller Dysfunktion bei einer Person, das System umfassend:
(a) einen im Beckenbereich des Individuums implantierten Sensor (120), der so konfiguriert ist, dass er einen Parameter erfasst, der mit der Episode sexueller Dysfunktion des Individuums verbunden ist;
(b) eine Stimulationselektrode (122), die in einer Beckenregion des Individuums implantiert und so konfiguriert ist, dass sie eine angepasste elektrische Stimulation bereitstellt;
(c) einen Prozessor, der wirkend mit der Stimulationselektrode und dem Sensor gekoppelt ist; und
(d) ein nichtflüchtiges computerlesbares Speichermedium, umfassend eine Software,
die so konfiguriert ist, dass sie den Prozessor zu Folgendem veranlasst:
(i) Empfangen von dem Sensor einer Messung des Parameters, der mit der Episode der sexuellen Dysfunktion verbunden ist;
(ii) Analysieren der Messung, um mindestens eines aus einer Intensität, Frequenz, oder einer Dauer der angepassten elektrischen Stimulation zu bestimmen; und
(iii) als Reaktion auf das Empfangen der Messung oder auf ein manuelles Antippen eines Betätigungsabschnitts des Systems, Veranlassen, dass die Stimulationselektrode (122) die angepasste elektrische Stimulation bereitstellt, wobei die bereitgestellte angepasste elektrische Stimulation ausreichend ist, um die Episode sexueller Dysfunktion zu behandeln.

2. System nach Anspruch 1, wobei die Stimulationselektrode so konfiguriert ist, dass sie implantiert wird, um mit dem Nervus pudendus zu interagieren.

3. System nach Anspruch 2, ferner umfassend eine zweite Stimulationselektrode, die so konfiguriert ist, dass sie implantiert wird, um mit dem kavernösen Nerv zu interagieren.

4. System nach einem der vorstehenden Ansprüche, wobei der Sensor ein Tumeszenz-Sensor ist, der so konfiguriert ist, dass er einen oder mehrere Parameter erfasst, der/die mit einer Änderung der Penislänge, des Umfangs und/oder der Steifigkeit verbunden ist/sind.

5. System nach Anspruch 4, wobei der Tumeszenz-Sensor einen Drucksensor, einen Temperatursensor, einen Blutflusssensor, einen Dehnungssensor oder eine Kombination davon erfasst.

6. System nach einem der vorstehenden Ansprüche, wobei die angepasste elektrische Stimulation so konfiguriert ist, dass sie sich in Abhängigkeit von Erregungs- oder Erektionszuständen variiert.

7. System nach einem der vorstehenden Ansprüche, wobei die angepasste elektrische Stimulation ausreicht, um eine Gewebeschwellung zu veranlassen, wobei das Gewebe Penis- oder Klitorisgewebe umfasst.

8. System nach einem der vorstehenden Ansprüche, wobei die angepasste elektrische Stimulation synergistisch mit einem Medikament zur Behandlung sexueller Dysfunktion wirkt.

9. System nach einem der vorstehenden Ansprüche, wobei der Parameter eine tägliche Variation zeigt.

10. System nach einem der vorstehenden Ansprüche, wobei der Parameter eine zeitliche oder zirkadiane Variation umfasst.

11. System nach einem der vorstehenden Ansprüche, ferner umfassend einen implantierbaren Pulsgenerator, der den Betätigungsabschnitt umfasst.

12. System nach einem der vorstehenden Ansprüche, wobei die Software so konfiguriert ist, dass sie den Prozessor zu Folgendem veranlasst: Analysieren der Messung, um die angepasste Stimulation für den Beginn einer sexuellen Aktivität, zum Ändern eines Stimulationsparadigmas während der sexuellen Aktivität und zum Beenden der sexuellen Aktivität zu bestimmen.

13. System nach einem der vorstehenden Ansprüche, wobei die angepasste elektrische Stimulation so konfiguriert ist, dass sie über die Zeit variiert.

14. System nach einem der vorstehenden Ansprüche, wobei der Parameter einen ersten Parameter und einen zweiten, zeitlich nach dem ersten liegenden Parameter umfasst und wobei die angepasste Stimulation basierend auf dem zweiten Parameter aufrechterhalten, modifiziert oder abgebrochen wird.

15. Verfahren zur Datenverarbeitung, das Verfahren umfassend:
(a) Empfangen einer Messung eines Parameters, der mit einer Episode sexueller Dysfunktion eines Individuums verbunden ist und mit einem in einer Beckenregion des Individuums implantierten Sensor gemessen wird;
(b) Analysieren des Parameters; und
c) als Reaktion auf das Empfangen der Messung oder auf ein manuelles Antippen eines Betätigungsabschnitts des Systems, Erzeugen eines elektrischen Stimulationsmusters, das so konfiguriert ist, dass es die Episode sexueller Dysfunktion des Individuums behandelt, wenn das Stimulationsmuster durch eine im Beckenbereich des Individuums implantierte Elektrode ausgeführt wird, wobei sich mindestens eines aus Intensität, Frequenz oder Dauer des Stimulationsmusters gemäß dem gemessenen Parameter verändert.

16. Verfahren nach Anspruch 15, ferner umfassend das Verwenden von Software, die so konfiguriert ist, dass sie das elektrische Stimulationsmuster basierend auf dem Parameter erzeugt, wobei die Software ein Machine-Learning-Modell umfasst und wobei das Machine-Learning-Modell konfiguriert ist, die von dem implantierten Sensor empfangene Messung zu klassifizieren und das Stimulationsmuster zu erzeugen.

## Revendications

1. Système destiné à traiter un épisode de trouble de la fonction sexuelle d'un individu, le système comprenant :
(a) un capteur (120) implanté dans une région pelvienne de l'individu configuré pour détecter un paramètre associé à l'épisode de trouble de la fonction sexuelle de l'individu ;
(b) une électrode stimulatrice (122) implantée dans une région pelvienne de l'individu configurée pour fournir une stimulation électrique adaptée ;
(c) un processeur couplé fonctionnellement à l'électrode stimulatrice et au capteur ; et
(d) un support de stockage non transitoire lisible par ordinateur comprenant un logiciel configuré pour amener le processeur à :
(i) recevoir à partir du capteur une mesure du paramètre associé à l'épisode de trouble de la fonction sexuelle ;
(ii) analyser la mesure pour déterminer au moins l'une parmi une intensité, une fréquence, ou une durée de la stimulation électrique adaptée ; et
(iii) en réponse à la réception de la mesure ou à un tapotement manuel sur une portion d'actionnement du système, amener l'électrode stimulatrice (122) à fournir la stimulation électrique adaptée, dans lequel la stimulation électrique adaptée fournie est suffisante pour traiter l'épisode de trouble de la fonction sexuelle.

2. Système selon la revendication 1, dans lequel l'électrode stimulatrice est configurée pour être implantée afin de s'interfacer avec un nerf honteux.

3. Système selon la revendication 2, comprenant en outre une deuxième électrode stimulatrice configurée pour être implantée afin de s'interfacer avec un nerf caverneux.

4. Système selon l'une quelconque des revendications précédentes, dans lequel le capteur est un capteur de tumescence configuré pour détecter un ou plusieurs paramètres associés à un changement de longueur, de volume, et/ou de rigidité du pénis.

5. Système selon la revendication 4, dans lequel le capteur de tumescence comprend un capteur de pression, un capteur de température, un capteur de flux sanguin, un capteur d'étirement, ou une combinaison de ceux-ci.

6. Système selon l'une quelconque des revendications précédentes, dans lequel la stimulation électrique adaptée est configurée pour varier sur les états d'excitation et d'érection.

7. Système selon l'une quelconque des revendications précédentes, dans lequel la stimulation électrique adaptée est suffisante pour aboutir à un engorgement de tissu, dans lequel ledit tissu comprend le tissu pénien ou clitoridien.

8. Système selon l'une quelconque des revendications précédentes, dans lequel la stimulation électrique adaptée fonctionne en synergie avec un traitement médicamenteux pour traiter le trouble de la fonction sexuelle.

9. Système selon l'une quelconque des revendications précédentes, dans lequel le paramètre présente une variation diurne.

10. Système selon l'une quelconque des revendications précédentes, dans lequel le paramètre comprend une variation temporelle ou circadienne.

11. Système selon l'une quelconque des revendications précédentes, comprenant en outre un générateur d'impulsions implantable comprenant la portion d'actionnement.

12. Système selon l'une quelconque des revendications précédentes, dans lequel le logiciel est configuré pour amener le processeur à : analyser la mesure afin de déterminer la stimulation électrique adaptée destinée à débuter une activité sexuelle, changer un paradigme de stimulation pendant l'activité sexuelle, et mettre fin à l'activité sexuelle.

13. Système selon l'une quelconque des revendications précédentes, dans lequel la stimulation électrique adaptée est configurée pour varier dans le temps.

14. Système selon l'une quelconque des revendications précédentes, dans lequel le paramètre comprend un premier paramètre et un deuxième plus tard dans le temps que le premier paramètre, et dans lequel la stimulation électrique adaptée est prolongée, modifiée, ou annulée sur la base du deuxième paramètre.

15. Procédé de traitement de données, le procédé comprenant :
(a) la réception d'une mesure d'un paramètre associé à un épisode de trouble de la fonction sexuelle d'un individu mesurée par un capteur implanté dans une région pelvienne de l'individu ;
(b) l'analyse du paramètre ; et
(c) en réponse à la réception de la mesure ou à un tapotement manuel sur une portion d'actionnement du système, la génération d'un schéma de stimulation électrique configuré pour traiter l'épisode de trouble de la fonction sexuelle de l'individu lorsque le schéma de stimulation est réalisé par une électrode implantée dans la région pelvienne de l'individu, dans lequel au moins l'une parmi une intensité, une fréquence, ou une durée du schéma de stimulation varie selon le paramètre qui est mesuré.

16. Procédé selon la revendication 15, comprenant en outre l'utilisation d'un logiciel configuré pour générer le schéma de stimulation électrique sur la base du paramètre, dans lequel le logiciel comprend un modèle d'apprentissage automatique, et dans lequel le modèle d'apprentissage automatique est configuré pour classifier la mesure reçue par le capteur implanté et générer le schéma de stimulation.
